# EUROPEAN PATENT SPECIFICATION

(11) **EP 2 205 071 B1**
(45) Date of publication and mention of the grant of the patent: **22.07.2015**
(21) Application number: 08837617.3
(22) Date of filing: 10.10.2008
(51) Int. Cl.: A01N 37/18, A61K 31/00, A61K 39/395, A61K 38/18, A61P 27/06

(54) **LINGO-1 ANTAGONISTS AND TRKB AGONISTS FOR USE IN THE TREATMENT OF GLAUCOMA**
LINGO-1-ANTAGONISTEN UND TRKB-AGONISTEN ZUR VERWENDUNG ZUR BEHANDLUNG VON GLAUKOM
ANTAGONISTES LINGO-1 ET AGONISTES TRKB POR LEUR UTILISATION DANS LE TRAITEMENT DU GLAUCOME

(30) Priority: 11.10.2007 US 979338 P
(43) Date of publication of application: 14.07.2010
(73) Proprietor: Biogen MA Inc., Cambridge, MA 02142 (US)
(72) Inventor: MI, Sha, Belmont MA 02478 (US)
(74) Representative: Adams, Harvey Vaughan John
(86) International application number: PCT/US2008/011633
(87) International publication number: WO 2009/048605

(56) References cited:
- EP-A1- 0 958 831
- WO-A2-2010/005570
- US-A1- 2003 162 734
- US-A1- 2007 060 526
- US-A1- 2007 186 296
- FU Q-L ET AL: "LINGO-1 exerts neuroprotection in a rat glaucoma model", INVESTIGATIVE OPHTHALMOLOGY & VISUAL SCIENCE, ASSOCIATION FOR RESEARCH IN VISION AND OPHTHALMOLOGY, US, vol. 46, no. suppl, 1 May 2005 (2005-05-01), pages E-157, XP009125532, ISSN: 0146-0404
- MI SHA ET AL: "LINGO-1 antagonist promotes spinal cord remyelination and axonal integrity in MOG-induced experimental autoimmune encephalomyelitis", NATURE MEDICINE, NATURE PUBLISHING GROUP, NEW YORK, NY, US, vol. 13, no. 10, 30 September 2007 (2007-09-30), pages 1228-1233, XP002525677, ISSN: 1078-8956, DOI: 10.1038/NM1664 [retrieved on 2007-09-30]
- FU QING-LING ET AL: "Blocking LINGO-1 function promotes retinal ganglion cell survival following ocular hypertension and optic nerve transection.", INVESTIGATIVE OPHTHALMOLOGY & VISUAL SCIENCE MAR 2008 LNKD- PUBMED:18326721, vol. 49, no. 3, March 2008 (2008-03), pages 975-985, XP009158058, ISSN: 0146-0404
- FU Q L ET AL: "Combined effect of brain-derived neurotrophic factor and LINGO-1 fusion protein on long-term survival of retinal ganglion cells in chronic glaucoma", NEUROSCIENCE, NEW YORK, NY, US, vol. 162, no. 2, 18 August 2009 (2009-08-18), pages 375-382, XP026238241, ISSN: 0306-4522, DOI: 10.1016/J.NEUROSCIENCE.2009.04.075 [retrieved on 2009-05-05]
- FU QING-LING ET AL: "LINGO-1 negatively regulates TrkB phosphorylation after ocular hypertension.", THE EUROPEAN JOURNAL OF NEUROSCIENCE MAR 2010 LNKD- PUBMED:20377621, vol. 31, no. 6, March 2010 (2010-03), pages 1091-1097, XP002672788, ISSN: 1460-9568
- LEE XINHUA ET AL: "NGF REGULATES THE EXPRESSION OF AXONAL LINGO-1 TO INHIBIT OLIGODENDROCYTE DIFFERENTIATION AND MYELINATION", JOURNAL OF NEUROSCIENCE, NEW YORK, NY, US, vol. 27, no. 1, 3 January 2007 (2007-01-03), pages 220-225, XP002559457, ISSN: 0270-6474, DOI: 10.1523/JNEUROSCI.4175-06.2007
- FU Q L ET AL: "Combination Brain-Derived Neurotrophic Factor and LINGO-1 Fusion Protein Promote Long-Term Survival to Retinal Ganglion Cells after Ocular Hypertension", NEUROSCIENCE RESEARCH, ELSEVIER, SHANNON, IR, vol. 65, 1 January 2009 (2009-01-01), page S171, XP026753231, ISSN: 0168-0102, DOI: 10.1016/J.NEURES.2009.09.896 [retrieved on 2009-01-01]
- MARTIN KEITH R G ET AL: "Gene therapy with brain-derived neurotrophic factor as a protection: retinal ganglion cells in a rat glaucoma model.", INVESTIGATIVE OPHTHALMOLOGY & VISUAL SCIENCE OCT 2003 LNKD- PUBMED:14507880, vol. 44, no. 10, October 2003 (2003-10), pages 4357-4365, XP009158081, ISSN: 0146-0404
- CHENG ET AL.: 'TrkB Gene Transfer Protects Retinal Ganglion Cells from Axotomy-Induced Death In Vivo.' JOURNAL OF NEUROSCIENCE vol. 22, no. 10, 15 May 2002, pages 3977 - 3986, XP008133696
- HARTMANN ET AL.: 'Truncated TrkB receptor-induced outgrowth of dendritic filopodia involves the p75 neurotrophin receptor.' JOURNAL OF CELL SCIENCE vol. 117, 2004, pages 5803 - 5814, XP008133697

### SEQUENCE LISTING

<110> Biogen Idec MA Inc.
<120> Methods For Treating Pressure Induced Optic Neuropathy, Preventing Neuronal Degeneration And Promoting Neuronal Cell Survival Via Administration Of Lingo-1 Antagonists And Trkb Agonists
<130> P34726EP-PCT
<140> EP08837617.3
   <141> 2008-10-10
<150> US 60/979,338
   <151> 2007-10-11
<160> 32
<170> Patent In version 3.3
<210> 1
   <211> 1845
   <212> DNA
   <213> Homo sapiens
<400> 1
   atgctggcgg ggggcgtgag gagcatgccc agccccctcc tggcctgctg gcagcccatc      60
   ctcctgctgg tgctgggctc agtgctgtca ggctcggcca cgggctgccc gccccgctgc     120
   gagtgctccg cccaggaccg cgctgtgctg tgccaccgca agcgctttgt ggcagtcccc     180
   gagggcatcc ccaccgagac gcgcctgctg gacctaggca agaaccgcat caaaacgctc     240
   aaccaggacg agttcgccag cttcccgcac ctggaggagc tggagctcaa cgagaacatc     300
   gtgagcgccg tggagcccgg cgccttcaac aacctcttca acctccggac gctgggtctc     360
   cgcagcaacc gcctgaagct catcccgcta ggcgtcttca ctggcctcag caacctgacc     420
   aagctggaca tcagcgagaa caagattgtt atcctgctgg actacatgtt tcaggacctg     480
   tacaacctca agtcactgga ggttggcgac aatgacctcg tctacatctc tcaccgcgcc     540
   ttcagcggcc tcaacagcct ggagcagctg acgctggaga aatgcaacct gacctccatc     600
   cccaccgagg cgctgtccca cctgcacggc ctcatcgtcc tgaggctccg gcacctcaac     660
   atcaatgcca tccgggacta ctccttcaag aggctctacc gactcaaggt cttggagatc     720
   tcccactggc cctacttgga caccatgaca cccaactgcc tctacggcct caacctgacg     780
   tccctgtcca tcacacactg caatctgacc gctgtgccct acctggccgt ccgccaccta     840
   gtctatctcc gcttcctcaa cctctcctac aaccccatca gcaccattga gggctccatg     900
   ttgcatgagc tgctccggct gcaggagatc cagctggtgg gcgggcagct ggccgtggtg     960
   gagccctatg ccttccgcgg cctcaactac ctgcgcgtgc tcaatgtctc tggcaaccag    1020
   ctgaccacac tggaggaatc agtcttccac tcggtgggca acctggagac actcatcctg    1080
   gactccaacc cgctggcctg cgactgtcgg ctcctgtggg tgttccggcg ccgctggcgg    1140
   ctcaacttca accggcagca gcccacgtgc gccacgcccg agtttgtcca gggcaaggag    1200
   ttcaaggact tccctgatgt gctactgccc aactacttca cctgccgccg cgcccgcatc    1260
   cgggaccgca aggcccagca ggtgtttgtg gacgagggcc acacggtgca gtttgtgtgc    1320
   cgggccgatg gcgacccgcc gcccgccatc ctctggctct caccccgaaa gcacctggtc    1380
   tcagccaaga gcaatgggcg gctcacagtc ttccctgatg gcacgctgga ggtgcgctac    1440
   gcccaggtac aggacaacgg cacgtacctg tgcatcgcgg ccaacgcggg cggcaacgac    1500
   tccatgcccg cccacctgca tgtgcgcagc tactcgcccg actggcccca tcagcccaac    1560
   aagaccttcg ctttcatctc caaccagccg ggcgagggag aggccaacag cacccgcgcc    1620
   actgtgcctt tccccttcga catcaagacc ctcatcatcg ccaccaccat gggcttcatc    1680
   tctttcctgg gcgtcgtcct cttctgcctg gtgctgctgt ttctctggag ccggggcaag    1740
   ggcaacacaa agcacaacat cgagatcgag tatgtgcccc gaaagtcgga cgcaggcatc    1800
   agctccgccg acgcgccccg caagttcaac atgaagatga tatga                    1845
<210> 2
   <211> 614
   <212> PRT
   <213> Homo sapiens
<400> 2
   Met Leu Ala Gly Gly Val Arg Ser Met Pro Ser Pro Leu Leu Ala Cys
   1               5                   10                  15
   Trp Gln Pro Ile Leu Leu Leu Val Leu Gly Ser Val Leu Ser Gly Ser
               20                  25                  30
   Ala Thr Gly Cys Pro Pro Arg Cys Glu Cys Ser Ala Gln Asp Arg Ala
           35                  40                  45
   Val Leu Cys His Arg Lys Arg Phe Val Ala Val Pro Glu Gly Ile Pro
       50                  55                  60
   Thr Glu Thr Arg Leu Leu Asp Leu Gly Lys Asn Arg Ile Lys Thr Leu
   65 70 75 80
   Asn Gln Asp Glu Phe Ala Ser Phe Pro His Leu Glu Glu Leu Glu Leu
   85 90 95
   Asn Glu Asn Ile Val Ser Ala Val Glu Pro Gly Ala Phe Asn Asn Leu
   100 105 110
   Phe Asn Leu Arg Thr Leu Gly Leu Arg Ser Asn Arg Leu Lys Leu Ile
   115 120 125
   Pro Leu Gly Val Phe Thr Gly Leu Ser Asn Leu Thr Lys Leu Asp Ile
   130 135 140
   Ser Glu Asn Lys Ile Val Ile Leu Leu Asp Tyr Met Phe Gln Asp Leu
   145 150 155 160
   Tyr Asn Leu Lys Ser Leu Glu Val Gly Asp Asn Asp Leu Val Tyr Ile
   165 170 175
   Ser His Arg Ala Phe Ser Gly Leu Asn Ser Leu Glu Gln Leu Thr Leu
   180 185 190
   Glu Lys Cys Asn Leu Thr Ser Ile Pro Thr Glu Ala Leu Ser His Leu
   195 200 205
   His Gly Leu Ile Val Leu Arg Leu Arg His Leu Asn Ile Asn Ala Ile
   210 215 220
   Arg Asp Tyr Ser Phe Lys Arg Leu Tyr Arg Leu Lys Val Leu Glu Ile
   225 230 235 240
   Ser His Trp Pro Tyr Leu Asp Thr Met Thr Pro Asn Cys Leu Tyr Gly
   245 250 255
   Leu Asn Leu Thr Ser Leu Ser Ile Thr His Cys Asn Leu Thr Ala Val
   260 265 270
   Pro Tyr Leu Ala Val Arg His Leu Val Tyr Leu Arg Phe Leu Asn Leu
   275 280 285
   Ser Tyr Asn Pro Ile Ser Thr Ile Glu Gly Ser Met Leu His Glu Leu
   290 295 300
   Leu Arg Leu Gln Glu Ile Gln Leu Val Gly Gly Gln Leu Ala Val Val
   305 310 315 320
   Glu Pro Tyr Ala Phe Arg Gly Leu Asn Tyr Leu Arg Val Leu Asn Val
   325 330 335
   Ser Gly Asn Gln Leu Thr Thr Leu Glu Glu Ser Val Phe His Ser Val
   340 345 350
   Gly Asn Leu Glu Thr Leu Ile Leu Asp Ser Asn Pro Leu Ala Cys Asp
   355 360 365
   Cys Arg Leu Leu Trp Val Phe Arg Arg Arg Trp Arg Leu Asn Phe Asn
   370 375 380
   Arg Gln Gln Pro Thr Cys Ala Thr Pro Glu Phe Val Gln Gly Lys Glu
   385 390 395 400
   Phe Lys Asp Phe Pro Asp Val Leu Leu Pro Asn Tyr Phe Thr Cys Arg
   405 410 415
   Arg Ala Arg Ile Arg Asp Arg Lys Ala Gln Gln Val Phe Val Asp Glu
   420 425 430
   Gly His Thr Val Gln Phe Val Cys Arg Ala Asp Gly Asp Pro Pro Pro
   435 440 445
   Ala Ile Leu Trp Leu Ser Pro Arg Lys His Leu Val Ser Ala Lys Ser
   450 455 460
   Asn Gly Arg Leu Thr Val Phe Pro Asp Gly Thr Leu Glu Val Arg Tyr
   465 470 475 480
   Ala Gln Val Gln Asp Asn Gly Thr Tyr Leu Cys Ile Ala Ala Asn Ala
   485 490 495
   Gly Gly Asn Asp Ser Met Pro Ala His Leu His Val Arg Ser Tyr Ser
   500 505 510
   Pro Asp Trp Pro His Gln Pro Asn Lys Thr Phe Ala Phe Ile Ser Asn
   515 520 525
   Gln Pro Gly Glu Gly Glu Ala Asn Ser Thr Arg Ala Thr Val Pro Phe
       530                 535                 540
   Pro Phe Asp Ile Lys Thr Leu Ile Ile Ala Thr Thr Met Gly Phe Ile
   545                 550                 555                 560
   Ser Phe Leu Gly Val Val Leu Phe Cys Leu Val Leu Leu Phe Leu Trp
                   565                 570                 575
   Ser Arg Gly Lys Gly Asn Thr Lys His Asn Ile Glu Ile Glu Tyr Val
               580                 585                 590
   Pro Arg Lys Ser Asp Ala Gly Ile Ser Ser Ala Asp Ala Pro Arg Lys
           595                 600                 605
   Phe Asn Met Lys Met Ile
       610
<210> 3
   <211> 5608
   <212> DNA
   <213> Homo sapiens
<400> 3
   aagacggatt ctcagacaag gcttgcaaat gccccgcagc catcatttaa ctgcacccgc      60
   agaatagtta cggtttgtca cccgaccctc ccggatcgcc taatttgtcc ctagtgagac     120
   cccgaggctc tgcccgcgcc tggcttcttc gtagctggat gcatatcgtg ctccgggcag     180
   cgcgggcgca gggcacgcgt tcgcgcacac cctagcacac atgaacacgc gcaagagctg     240
   aaccaagcac ggtttccatt tcaaaaaggg agacagcctc taccgcgatt gtagaagaga     300
   ctgtggtgtg aattagggac cgggaggcgt cgaacggagg aacggttcat cttagagact     360
   aattttctgg agtttctgcc cctgctctgc gtcagccctc acgtcacttc gccagcagta     420
   gcagaggcgg cggcggcggc tcccggaatt gggttggagc aggagcctcg ctggctgctt     480
   cgctcgcgct ctacgcgctc agtccccggc ggtagcagga gcctggaccc aggcgccgcc     540
   ggcgggcgtg aggcgccgga gcccggcctc gaggtgcata ccggaccccc attcgcatct     600
   aacaaggaat ctgcgcccca gagagtcccg ggagcgccgc cggtcggtgc ccggcgcgcc     660
   gggccatgca gcgacggccg ccgcggagct ccgagcagcg gtagcgcccc cctgtaaagc     720
   ggttcgctat gccggggcca ctgtgaaccc tgccgcctgc cggaacactc ttcgctccgg     780
   accagctcag cctctgataa gctggactcg gcacgcccgc aacaagcacc gaggagttaa 840
   gagagccgca agcgcaggga aggcctcccc gcacgggtgg gggaaagcgg ccggtgcagc 900
   gcggggacag gcactcgggc tggcactggc tgctagggat gtcgtcctgg ataaggtggc 960
   atggacccgc catggcgcgg ctctggggct tctgctggct ggttgtgggc ttctggaggg 1020
   ccgctttcgc ctgtcccacg tcctgcaaat gcagtgcctc tcggatctgg tgcagcgacc 1080
   cttctcctgg catcgtggca tttccgagat tggagcctaa cagtgtagat cctgagaaca 1140
   tcaccgaaat tttcatcgca aaccagaaaa ggttagaaat catcaacgaa gatgatgttg 1200
   aagcttatgt gggactgaga aatctgacaa ttgtggattc tggattaaaa tttgtggctc 1260
   ataaagcatt tctgaaaaac agcaacctgc agcacatcaa ttttacccga aacaaactga 1320
   cgagtttgtc taggaaacat ttccgtcacc ttgacttgtc tgaactgatc ctggtgggca 1380
   atccatttac atgctcctgt gacattatgt ggatcaagac tctccaagag gctaaatcca 1440
   gtccagacac tcaggatttg tactgcctga atgaaagcag caagaatatt cccctggcaa 1500
   acctgcagat acccaattgt ggtttgccat ctgcaaatct ggccgcacct aacctcactg 1560
   tggaggaagg aaagtctatc acattatcct gtagtgtggc aggtgatccg gttcctaata 1620
   tgtattggga tgttggtaac ctggtttcca aacatatgaa tgaaacaagc cacacacagg 1680
   gctccttaag gataactaac atttcatccg atgacagtgg gaagcagatc tcttgtgtgg 1740
   cggaaaatct tgtaggagaa gatcaagatt ctgtcaacct cactgtgcat tttgcaccaa 1800
   ctatcacatt tctcgaatct ccaacctcag accaccactg gtgcattcca ttcactgtga 1860
   aaggcaaccc caaaccagcg cttcagtggt tctataacgg ggcaatattg aatgagtcca 1920
   aatacatctg tactaaaata catgttacca atcacacgga gtaccacggc tgcctccagc 1980
   tggataatcc cactcacatg aacaatgggg actacactct aatagccaag aatgagtatg 2040
   ggaaggatga gaaacagatt tctgctcact tcatgggctg gcctggaatt gacgatggtg 2100
   caaacccaaa ttatcctgat gtaatttatg aagattatgg aactgcagcg aatgacatcg 2160
   gggacaccac gaacagaagt aatgaaatcc cttccacaga cgtcactgat aaaaccggtc 2220
   gggaacatct ctcggtctat gctgtggtgg tgattgcgtc tgtggtggga ttttgccttt 2280
   tggtaatgct gtttctgctt aagttggcaa gacactccaa gtttggcatg aaagatttct 2340
   catggtttgg atttgggaaa gtaaaatcaa gacaaggtgt tggcccagcc tccgttatca 2400
   gcaatgatga tgactctgcc agcccactcc atcacatctc caatgggagt aacactccat 2460
   cttcttcgga aggtggccca gatgctgtca ttattggaat gaccaagatc cctgtcattg 2520
   aaaatcccca gtactttggc atcaccaaca gtcagctcaa gccagacaca tttgttcagc 2580
   acatcaagcg acataacatt gttctgaaaa gggagctagg cgaaggagcc tttggaaaag 2640
   tgttcctagc tgaatgctat aacctctgtc ctgagcagga caagatcttg gtggcagtga 2700
   agaccctgaa ggatgccagt gacaatgcac gcaaggactt ccaccgtgag gccgagctcc 2760
   tgaccaacct ccagcatgag cacatcgtca agttctatgg cgtctgcgtg gagggcgacc 2820
   ccctcatcat ggtctttgag tacatgaagc atggggacct caacaagttc ctcagggcac 2880
   acggccctga tgccgtgctg atggctgagg gcaacccgcc cacggaactg acgcagtcgc 2940
   agatgctgca tatagcccag cagatcgccg cgggcatggt ctacctggcg tcccagcact 3000
   tcgtgcaccg cgatttggcc accaggaact gcctggtcgg ggagaacttg ctggtgaaaa 3060
   tcggggactt tgggatgtcc cgggacgtgt acagcactga ctactacagg gtcggtggcc 3120
   acacaatgct gcccattcgc tggatgcctc cagagagcat catgtacagg aaattcacga 3180
   cggaaagcga cgtctggagc ctgggggtcg tgttgtggga gattttcacc tatggcaaac 3240
   agccctggta ccagctgtca aacaatgagg tgatagagtg tatcactcag ggccgagtcc 3300
   tgcagcgacc ccgcacgtgc ccccaggagg tgtatgagct gatgctgggg tgctggcagc 3360
   gagagcccca catgaggaag aacatcaagg gcatccatac cctccttcag aacttggcca 3420
   aggcatctcc ggtctacctg gacattctag gctagggccc ttttccccag accgatcctt 3480
   cccaacgtac tcctcagacg ggctgagagg atgaacatct tttaactgcc gctggaggcc 3540
   accaagctgc tctccttcac tctgacagta ttaacatcaa agactccgag aagctctcga 3600
   gggaagcagt gtgtacttct tcatccatag acacagtatt gacttctttt tggcattatc 3660
   tctttctctc tttccatctc ccttggttgt tcctttttct ttttttaaat tttctttttc 3720
   tttttttttt cgtcttccct gcttcacgat tcttaccctt tcttttgaat caatctggct 3780
   tctgcattac tattaactct gcatagacaa aggccttaac aaacgtaatt tgttatatca 3840
   gcagacactc cagtttgccc accacaacta acaatgcctt gttgtattcc tgcctttgat 3900
   gtggatgaaa aaaagggaaa acaaatattt cacttaaact ttgtcacttc tgctgtacag 3960
   atatcgagag tttctatgga ttcacttcta tttatttatt attattactg ttcttattgt 4020
   ttttggatgg cttaagcctg tgtataaaaa agaaaacttg tgttcaatct gtgaagcctt 4080
   tatctatggg agattaaaac cagagagaaa gaagatttat tatgaaccgc aatatgggag 4140
   gaacaaagac aaccactggg atcagctggt gtcagtccct acttaggaaa tactcagcaa 4200
   ctgttagctg ggaagaatgt attcggcacc ttcccctgag gacctttctg aggagtaaaa 4260
   agactactgg cctctgtgcc atggatgatt cttttcccat caccagaaat gatagcgtgc 4320
   agtagagagc aaagatggct tccgtgagac acaagatggc gcatagtgtg ctcggacaca 4380
   gttttgtctt cgtaggttgt gatgatagca ctggtttgtt tctcaagcgc tatccacaga 4440
   acctttgtca acttcagttg aaaagaggtg gattcatgtc cagagctcat ttcggggtca 4500
   ggtgggaaag ccaagaactt ggaaaagata agacaagcta taaattcgga ggcaagtttc 4560
   ttttacaatg aacttttcag atctcacttc cctccgaccc ctaacttcca tgcccacccg 4620
   tccttttaac tgtgcaagca aaattgtgca tggtcttcgt cgattaatac cttgtgtgca 4680
   gacactactg ctccagacgt cgtttccctg ataggtagag cagatccata aaaaggtatg 4740
   acttatacaa ttaggggaag ctaatggagt ttattagctg agtatcaatg tctctgcgtt 4800
   gtacggtggt gatgggtttt aatgaatatg gaccctgaag cctggaaatc ctcatccacg 4860
   tcgaacccac aggactgtgg gaagggcaga atcaatccct aagggaaagg aaacctcacc 4920
   ctgagggcat cacatgcact catgttcagt gtacacaggt caagtccctt gctctgggct 4980
   ctagttggga gagtggtttc attccaagtg tactccattg tcagtatgct gtttttgttt 5040
   ccttcactcc attcaaaaag tcaaaataca aaatttggca cagcatgcca acgggaggct 5100
   gtgcccagac caagcactgg aagtgtgctt ctaggcatag tcattggttt tgcaaaaaga 5160
   gggctcaaat ttaaatagaa atttacagct atttgaatgg tcagatatac caagaaagaa 5220
   aaatatttct gttcctcaag aaaacttgct accctctgtg aggggaattt tgctaaactt 5280
   gacatcttta taacatgagc cagattgaaa gggagtgatt ttcattcatc ttaggtcatg 5340
   ttatttcata tttgtttctg aaggtgcgat agctctgttt taggttttgc ttgcgcctgt 5400
   taattactgg aacaccttat ttttcattaa aggctttgaa agccaattct caaaaattca 5460
   aaagtgcaaa ttaacagaac aaaaggaaat ccagtagcaa ctgcagtcaa gcgagggagt 5520
   tgacaagata aaccttacgt ccattcaagt tatatgctgg cctatgagag atgagagttg 5580
   ggtcgtttgt tctctttgtt gatgattt 5608
<210> 4
   <211> 838
   <212> PRT
   <213> Homo sapiens
<400> 4
<210> 5
   <211> 4757
   <212> DNA
   <213> Rattus sp.
<400> 5
   atctgtgtgc gagtgcgtgt gcgtgcgtgt gtgtgtgtgt gtgtgtgtgt gtgtgtgtgt 60
   gtgtgagcgt gtgtgttttt ggatttcata ctaattttct ggagtttctg cccctgctct 120
   gcgtcagccc tcacgtcact tcgccagcag tagcagaggc ggcggcggcc gccggttaga 180
   gcccagtcgc tgcttcagct gctgttgctg cttctgcggc gctctgctcc ctgcgctggc 240
   tacgggaggc cgggggagcc gcgccgacag tcctctgtgg ccagggccgg cactgtcctg 300
   ctaccgcagt tgctccccag ccctgaggtg cgcaccgata tcgatatccg tgccggttta 360
   gcggttctgc gacccaaaga gtccagggag agccaccgag tggcgcctgg cgtataggac    420
   catgcagccg ccttgtggct tggagcagcg gcccgtgatg ttccagccac tgtgaaccat    480
   ttggtcagcg ccaacctgct cagccccagc accgacaggc tcagcctctg gtacgctcct    540
   ctcggcggga ggccatcagc accaagcagc aagagggctc agggaaggcc tcccccctcc   600
   ggcgggggac gcctggctca gcgtagggac acgcactctg actgactggc actggcagct    660
   cgggatgtcg ccctggccga ggtggcatgg acccgccatg gcgcggctct ggggcttatg    720
   cttgctggtc ttgggcttct ggagggcttc tcttgcctgc cccatgtcct gcaaatgcag    780
   caccactagg atttggtgta ccgagccttc tcctggcatc gtggcatttc cgaggttgga    840
   acctaacagc attgacccag agaacatcac cgaaattctc attgcaaacc agaaaaggtt    900
   agaaatcatc aatgaagatg atgtcgaagc ttacgtgggg ctgaaaaacc ttacaattgt    960
   ggattccggc ttaaagtttg tggcttacaa ggcgtttctg aagaacggca acctgcggca   1020
   catcaatttc actcgaaaca agctgacgag tttgtccagg agacatttcc gccaccttga   1080
   cttgtctgac ctgatcctga cgggtaatcc gttcacgtgt tcctgtgaca tcatgtggct   1140
   caagactctc caggagacga aatccagccc cgacactcag gatttgtatt gcctcaatga   1200
   gagcagcaag aatacccctc tggcgaacct gcagattccc aattgtggtc tgccgtctgc   1260
   acgtctggcc gctcctaacc tcacggtgga ggaagggaag tctgtgacca tttcctgcag   1320
   cgtcgggggt gacccgctcc ccaccttgta ctgggacgtt gggaatttgg tttccaaaca   1380
   catgaatgaa acaagccaca cacagggctc cttaaggata acaaacattt catcggatga   1440
   cagtgggaaa caaatctctt gtgtggcaga aaacctcgtc ggagaagatc aagactctgt   1500
   gaacctcact gtgcattttg caccaaccat cacatttctc gaatctccaa cctcagacca   1560
   ccactggtgc atcccattca ctgtgagagg caaccccaag ccagcacttc agtggttcta   1620
   caacggagcc atactgaatg aatccaagta catctgtacc aaaatacacg tcaccaatca   1680
   cacggagtac cacggctgcc tccagctgga taaccccact catatgaata atggagacta   1740
   caccctaatg gccaagaatg aatatgggaa ggacgagaga cagatttctg ctcacttcat   1800
   gggccggcct ggagttgact atgagacaaa cccaaattac cctgaagtcc tctatgaaga   1860
   ctggaccacg ccaactgaca tcggggatac tacaaacaaa agtaatgaga tcccctccac   1920
   ggatgttgct gaccaaacca atcgggagca tctctcggtc tatgctgtgg tggtgattgc   1980
   ctctgtggta ggattctgcc tgctggtgat gctgcttctg ctcaagttgg cgagacattc   2040
   caagtttggc atgaaaggcc cagcttccgt catcagcaac gacgatgact ctgccagccc 2100
   tctccaccac atctccaacg ggagcaacac tccgtcttct tcggagggcg ggcccgatgc 2160
   tgtcatcatt gggatgacca agatccctgt cattgaaaac ccccagtact tcggtatcac 2220
   caacagccag ctcaagccgg acacatttgt tcagcacatc aagagacaca acatcgttct 2280
   gaagagggag cttggagaag gagcctttgg gaaagttttc ctagcggagt gctataacct 2340
   ctgccccgag caggataaga tcctggtggc cgtgaagacg ctgaaggacg ccagcgacaa 2400
   tgctcgcaag gactttcatc gcgaagccga gctgctgacc aacctccagc acgagcacat 2460
   tgtcaagttc tacggtgtct gtgtggaggg cgacccactc atcatggtct ttgagtacat 2520
   gaagcacggg gacctcaaca agttccttag ggcacacggg ccagatgcag tgctgatggc 2580
   agagggtaac ccgcccaccg agctgacgca gtcgcagatg ctgcacatcg ctcagcaaat 2640
   cgcagcaggc atggtctacc tggcatccca acacttcgtg caccgagacc tggccacccg 2700
   gaactgcttg gtaggagaga acctgctggt gaaaattggg gacttcggga tgtcccggga 2760
   tgtatacagc accgactact accgggttgg tggccacaca atgttgccca tccgatggat 2820
   gcctccagag agcatcatgt acaggaaatt caccaccgag agtgacgtct ggagcctggg 2880
   agttgtgttg tgggagatct tcacctacgg caagcagccc tggtatcagc tatcaaacaa 2940
   cgaggtgata gaatgcatca cccagggcag agtccttcag cggcctcgca cgtgtcccca 3000
   ggaggtgtac gagctgatgc tgggatgctg gcagcgggaa ccacacacaa ggaagaacat 3060
   caagaacatc cacacactcc ttcagaactt ggcgaaggcg tcgcccgtct acctggacat 3120
   cctaggctag actccctctt ctcccagacg gcccttccca aggcacccct cagacctctt 3180
   aactgccgct gatgtcacca ccttgctgtc cttcgctctg acagtgttaa caagacaagg 3240
   agcggctctc cggggtgagg cagtgcgcac ttccccatcc acagacagta tcgactcgct 3300
   tctggctttg tcgctttctc tccctttggt ttgtttcttt cttttgccca ttctccattt 3360
   atttatttat ttatttattt atttatttat ttatttattt atttatctat ctatctatct 3420
   atttatttat ttatttattg gtcttcactg cttcatggtc ctcggcctct ctccttgacc 3480
   gatctggctt ctgtactcct attcactgta catagacaaa ggccttaaca aacctgattt 3540
   gttatatcag cagacactcc agtttgccca ccacaactaa caatgccttg ttgtattcct 3600
   gcctttgatg tggatgaaaa aaagggaaaa aaaataatca aacatctgac ttaaaccgtc 3660
   acttccgatg tacagacacg ggcgtttctt atggattcac ttctatctat ctatttattt 3720
   atttatctat ttatttattt ctcttctttg ttgttttccg gtggttttag cctgtgtatg 3780
   agaagggaaa gtcatgtaca gtctgggaaa actttatctg tgggaaatgg aaaccagaag    3840
   gggaaagaag ctttaccata aagcacagca ggagtgagac acagaaaagc cattggatca    3900
   gccagagtcc gtcctgcata ggaaaaccca gcagccatca ggctggagga tcatgttcgg    3960
   cactgacccc cgaggacctt tctgaggagg acacagaatg ttaaactctg catcatggac    4020
   acagtttccg atcacagata ctggccttca atggaaaaaa aaaaaaaaaa aacccagata    4080
   gttcttgtga gacctggaca gcacgtccaa catccagaca ttgtggtcgg gcacagtgac    4140
   agagttgatg catttctcac gggttattct acagagcttt tgtcaagtcc aatggaagga    4200
   ggtagattct tgttcagata tgatttcggg aaaaaccgag tccttgacaa agacaggaga    4260
   caccctcagt tgggaggcaa gtttctctta ccttggactt tctcacacag caattctcac    4320
   ccccaccccc tccactctca cctgtcttgt aactgtgcaa acaaaagtgt gcatggtctt    4380
   tgtcagttga tacctttgtg cacctctgtg cagaaactgc tgtctgtccc ggctgtggta    4440
   cccgatcagt ggggtagatc cacgaaaggt ctcattttag gccgctttgg gaaggtaacc    4500
   agatcggtag ctggaagcac tctccagtag gtggcgaagg gtgagtgggt ctgctgaagc    4560
   ctgcatatct tcacccacct caaacccacc gggctgcaca ggggacaggc acaggccacc    4620
   cctgagggac agggaagctc tcttgggata ccacctgagt ttacattcag tgtgctcagg    4680
   tcaagtctct cgctcggggc tctgtttcgg ggagaatggt ttcattccaa cgcactcatt    4740
   atcaggattc tgttttc                                                   4757
<210> 6
   <211> 821
   <212> PRT
   <213> Rattus sp.
<400> 6
   Met Ser Pro Trp Pro Arg Trp His Gly Pro Ala Met Ala Arg Leu Trp
   1               5                   10                  15
   Gly Leu Cys Leu Leu Val Leu Gly Phe Trp Arg Ala Ser Leu Ala Cys
               20                  25                  30
   Pro Met Ser Cys Lys Cys Ser Thr Thr Arg Ile Trp Cys Thr Glu Pro
           35                  40                  45
   Ser Pro Gly Ile Val Ala Phe Pro Arg Leu Glu Pro Asn Ser Ile Asp
       50                  55                  60
   Pro Glu Asn Ile Thr Glu Ile Leu Ile Ala Asn Gln Lys Arg Leu Glu
   65 70 75 80
   Ile Ile Asn Glu Asp Asp Val Glu Ala Tyr Val Gly Leu Lys Asn Leu
   85 90 95
   Thr Ile Val Asp Ser Gly Leu Lys Phe Val Ala Tyr Lys Ala Phe Leu
   100 105 110
   Lys Asn Gly Asn Leu Arg His Ile Asn Phe Thr Arg Asn Lys Leu Thr
   115 120 125
   Ser Leu Ser Arg Arg His Phe Arg His Leu Asp Leu Ser Asp Leu Ile
   130 135 140
   Leu Thr Gly Asn Pro Phe Thr Cys Ser Cys Asp Ile Met Trp Leu Lys
   145 150 155 160
   Thr Leu Gln Glu Thr Lys Ser Ser Pro Asp Thr Gln Asp Leu Tyr Cys
   165 170 175
   Leu Asn Glu Ser Ser Lys Asn Thr Pro Leu Ala Asn Leu Gln Ile Pro
   180 185 190
   Asn Cys Gly Leu Pro Ser Ala Arg Leu Ala Ala Pro Asn Leu Thr Val
   195 200 205
   Glu Glu Gly Lys Ser Val Thr Ile Ser Cys Ser Val Gly Gly Asp Pro
   210 215 220
   Leu Pro Thr Leu Tyr Trp Asp Val Gly Asn Leu Val Ser Lys His Met
   225 230 235 240
   Asn Glu Thr Ser His Thr Gln Gly Ser Leu Arg Ile Thr Asn Ile Ser
   245 250 255
   Ser Asp Asp Ser Gly Lys Gln Ile Ser Cys Val Ala Glu Asn Leu Val
   260 265 270
   Gly Glu Asp Gln Asp Ser Val Asn Leu Thr Val His Phe Ala Pro Thr
   275 280 285
   Ile Thr Phe Leu Glu Ser Pro Thr Ser Asp His His Trp Cys Ile Pro
       290                 295                 300
   Phe Thr Val Arg Gly Asn Pro Lys Pro Ala Leu Gln Trp Phe Tyr Asn
   305                 310                 315                 320
   Gly Ala Ile Leu Asn Glu Ser Lys Tyr Ile Cys Thr Lys Ile His Val
                   325                 330                 335
   Thr Asn His Thr Glu Tyr His Gly Cys Leu Gln Leu Asp Asn Pro Thr
               340                 345                 350
   His Met Asn Asn Gly Asp Tyr Thr Leu Met Ala Lys Asn Glu Tyr Gly
           355                 360                 365
   Lys Asp Glu Arg Gln Ile Ser Ala His Phe Met Gly Arg Pro Gly Val
       370                 375                 380
   Asp Tyr Glu Thr Asn Pro Asn Tyr Pro Glu Val Leu Tyr Glu Asp Trp
   385                 390                 395                 400
   Thr Thr Pro Thr Asp Ile Gly Asp Thr Thr Asn Lys Ser Asn Glu Ile
                   405                 410                 415
   Pro Ser Thr Asp Val Ala Asp Gln Thr Asn Arg Glu His Leu Ser Val
               420                 425                 430
   Tyr Ala Val Val Val Ile Ala Ser Val Val Gly Phe Cys Leu Leu Val
           435                 440                 445
   Met Leu Leu Leu Leu Lys Leu Ala Arg His Ser Lys Phe Gly Met Lys
       450                 455                 460
   Gly Pro Ala Ser Val Ile Ser Asn Asp Asp Asp Ser Ala Ser Pro Leu
   465                 470                 475                 480
   His His Ile Ser Asn Gly Ser Asn Thr Pro Ser Ser Ser Glu Gly Gly
                   485                 490                 495
   Pro Asp Ala Val Ile Ile Gly Met Thr Lys Ile Pro Val Ile Glu Asn
               500                 505                 510
   Pro Gln Tyr Phe Gly Ile Thr Asn Ser Gln Leu Lys Pro Asp Thr Phe
   515 520 525
   Val Gln His Ile Lys Arg His Asn Ile Val Leu Lys Arg Glu Leu Gly
   530 535 540
   Glu Gly Ala Phe Gly Lys Val Phe Leu Ala Glu Cys Tyr Asn Leu Cys
   545 550 555 560
   Pro Glu Gln Asp Lys Ile Leu Val Ala Val Lys Thr Leu Lys Asp Ala
   565 570 575
   Ser Asp Asn Ala Arg Lys Asp Phe His Arg Glu Ala Glu Leu Leu Thr
   580 585 590
   Asn Leu Gln His Glu His Ile Val Lys Phe Tyr Gly Val Cys Val Glu
   595 600 605
   Gly Asp Pro Leu Ile Met Val Phe Glu Tyr Met Lys His Gly Asp Leu
   610 615 620
   Asn Lys Phe Leu Arg Ala His Gly Pro Asp Ala Val Leu Met Ala Glu
   625 630 635 640
   Gly Asn Pro Pro Thr Glu Leu Thr Gln Ser Gln Met Leu His Ile Ala
   645 650 655
   Gln Gln Ile Ala Ala Gly Met Val Tyr Leu Ala Ser Gln His Phe Val
   660 665 670
   His Arg Asp Leu Ala Thr Arg Asn Cys Leu Val Gly Glu Asn Leu Leu
   675 680 685
   Val Lys Ile Gly Asp Phe Gly Met Ser Arg Asp Val Tyr Ser Thr Asp
   690 695 700
   Tyr Tyr Arg Val Gly Gly His Thr Met Leu Pro Ile Arg Trp Met Pro
   705 710 715 720
   Pro Glu Ser Ile Met Tyr Arg Lys Phe Thr Thr Glu Ser Asp Val Trp
   725 730 735
   Ser Leu Gly Val Val Leu Trp Glu Ile Phe Thr Tyr Gly Lys Gln Pro
   740 745 750
   Trp Tyr Gln Leu Ser Asn Asn Glu Val Ile Glu Cys Ile Thr Gln Gly
   755 760 765
   Arg Val Leu Gln Arg Pro Arg Thr Cys Pro Gln Glu Val Tyr Glu Leu
   770 775 780
   Met Leu Gly Cys Trp Gln Arg Glu Pro His Thr Arg Lys Asn Ile Lys
   785 790 795 800
   Asn Ile His Thr Leu Leu Gln Asn Leu Ala Lys Ala Ser Pro Val Tyr
   805 810 815
   Leu Asp Ile Leu Gly
   820
<210> 7
   <211> 6
   <212> PRT
   <213> Homo sapiens
<400> 7
   Met Gln Val Ser Lys Arg
   1 5
<210> 8
   <211> 116
   <212> PRT
   <213> Artificial sequence
<220>
   <223> VH Sequence of the 1A7 antibody
<400> 8
   Gln Val Gln Leu Val Gln Ser Gly Pro Glu Leu Lys Lys Pro Gly Glu
   1 5 10 15
   Thr Val Lys Ile Ser Cys Lys Ala Ser Gly Tyr Thr Phe Thr Asn Tyr
   20 25 30
   Gly Met Asn Trp Val Lys Gln Ala Pro Gly Lys Gly Leu Lys Trp Met
   35 40 45
   Gly Trp Ile Asn Thr Asp Thr Gly Glu Pro Thr Tyr Thr Glu Asp Phe
   50 55 60
   Gln Gly Arg Phe Ala Phe Ser Leu Glu Thr Ser Ala Ser Thr Val Tyr
   65 70 75 80
   Leu Gln Phe Asn Asn Leu Lys Asn Glu Asp Thr Ala Thr Tyr Phe Cys
   85 90 95
   Ala Arg Glu Gly Val His Phe Asp Tyr Trp Gly Gln Gly Thr Thr Val
   100 105 110
   Thr Val Ser Ser
   115
<210> 9
   <211> 106
   <212> PRT
   <213> Artificial sequence
<220>
   <223> VL Sequence of the IA7 antibody
<400> 9
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Tyr Met
   20 25 30
   His Trp Tyr Gln Gln Lys Ser Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Ser Ser Asn Pro Phe Thr
   85 90 95
   Phe Gly Ser Gly Thr Lys Leu Glu Ile Lys
   100 105
<210> 10
   <211> 5
   <212> PRT
   <213> Artificial sequence
<220>
   <223> VH CDR1 sequence of the 1A7 antibody
<400> 10
   Asn Tyr Gly Met Asn
   1               5
<210> 11
   <211> 17
   <212> PRT
   <213> Artificial sequence
   <220>
   <223> VH CDR2 sequence of the 1A7 antibody
<400> 11
   Trp Ile Asn Thr Asp Thr Gly Glu Pro Thr Tyr Thr Glu Asp Phe Gln
   1               5                   10                  15
   Gly
<210> 12
   <211> 7
   <212> PRT
   <213> Artificial sequence
<220>
   <223> VH CDR3 sequence of the 1A7 antibody
<400> 12
   Glu Gly Val His Phe Asp Tyr
   1               5
<210> 13
   <211> 10
   <212> PRT
   <213> Artificial sequence
<220>
   <223> VL CDR1 sequence of the 1A7 antibody
<400> 13
   Ser Ala Ser Ser Ser Val Ser Tyr Met His
   1               5                   10
<210> 14
   <211> 7
   <212> PRT
   <213> Artificial sequence
<220>
   <223> VL CDR2 sequence of the 1A7 antibody
<400> 14
   Asp Thr Ser Lys Leu Ala Ser
   1 5
<210> 15
   <211> 9
   <212> PRT
   <213> Artificial sequence
<220>
   <223> VL CDR3 sequence of the 1A7 antibody
<400> 15
   Gln Gln Trp Ser Ser Asn Pro Phe Thr
   1 5
<210> 16
   <211> 120
   <212> PRT
   <213> Artificial sequence
<220>
   <223> VH Sequence of the 3B5 antibody
<400> 16
   Gln Val Gln Leu Gln Gln Pro Gly Ala Glu Leu Val Arg Pro Gly Thr
   1 5 10 15
   Ser Val Lys Leu Ser Cys Arg Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Val Ile Asp Pro Ser Asp Ser Tyr Thr Asn Tyr Asn Gln Lys Phe
   50 55 60
   Arg Gly Lys Ala Thr Leu Thr Val Asp Thr Ser Ser Ser Thr Ala Tyr
   65 70 75 80
   Met Gln Leu Ser Ser Leu Thr Ser Glu Asp Ser Ala Val Tyr Tyr Cys
   85 90 95
   Ala Arg Pro Tyr Tyr Gly Ser His Trp Phe Phe Asp Val Trp Gly Thr
   100 105 110
   Gly Thr Thr Val Thr Val Ser Ser
   115 120
<210> 17
   <211> 106
   <212> PRT
   <213> Artificial sequence
<220>
   <223> VL Sequence of the 3B5 antibody
<400> 17
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Arg Val Ser Tyr Val
   20 25 30
   His Trp Tyr Gln Gln Lys Ser Gly Thr Ser Pro Lys Arg Trp Leu Tyr
   35 40 45
   Asp Thr Ser Asn Leu Ala Ser Gly Val Pro Ala Arg Phe Gly Gly Asn
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Ser Thr Asn Pro Pro Thr
   85 90 95
   Phe Gly Gly Gly Thr Lys Leu Glu Ile Lys
   100 105
<210> 18
   <211> 5
   <212> PRT
   <213> Artificial sequence
<220>
   <223> VH CDR1 sequence of the 3B5 antibody
<400> 18
   Ser Tyr Trp Met His
   1 5
<210> 19
   <211> 17
   <212> PRT
   <213> Unknown
<220>
   <223> VH CDR2 sequence of the 3B5 antibody
<400> 19
   Val Ile Asp Pro Ser Asp Ser Tyr Thr Asn Tyr Asn Gln Lys Phe Arg
   1 5 10 15
   Gly
<210> 20
   <211> 10
   <212> PRT
   <213> Artificial sequence
<220>
   <223> VH CDR3 sequence of the 3B5 antibody
<400> 20
   Pro Tyr Tyr Gly Ser His Trp Phe Phe Asp
   1 5 10
<210> 21
   <211> 10
   <212> PRT
   <213> Artificial sequence
<220>
   <223> VL CDR1 sequence of the 3B5 antibody
<400> 21
   Ser Ala Ser Ser Arg Val Ser Tyr Val His
   1 5 10
<210> 22
   <211> 7
   <212> PRT
   <213> Artificial sequence
<220>
   <223> VL CDR2 sequence of the 3B5 antibody
<400> 22
   Asp Thr Ser Asn Leu Ala Ser
   1 5
<210> 23
   <211> 9
   <212> PRT
   <213> Artificial sequence
<220>
   <223> VL CDR3 sequence of the 3B5 antibody
<400> 23
   Gln Gln Trp Ser Thr Asn Pro Pro Thr
   1 5
<210> 24
   <211> 119
   <212> PRT
   <213> Artificial sequence
<220>
   <223> VH Sequence of the LI33 antibody
<400> 24
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ile Tyr
   20 25 30
   Pro Met Phe Trp Val Arg Gln Ala Pro Gly Lys Gly Leu Glu Trp Val
   35 40 45
   Ser Trp Ile Gly Pro Ser Gly Gly Ile Thr Lys Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Thr Tyr Tyr Cys
   85 90 95
   Ala Arg Glu Gly His Asn Asp Trp Tyr Phe Asp Leu Trp Gly Arg Gly
   100 105 110
   Thr Leu Val Thr Val Ser Ser
   115
<210> 25
   <211> 107
   <212> PRT
   <213> Artificial sequence
<220>
   <223> VL Sequence of the LI33 antibody
<400> 25
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asp Lys Trp Pro Leu
                   85                  90                  95
   Thr Phe Gly Gly Gly Thr Lys Val Glu Ile Lys
               100                 105
<210> 26
   <211> 5
   <212> PRT
   <213> Artificial sequence
<220>
   <223> VH CDR1 sequence of the LI33 antibody
<400> 26
   Ile Tyr Pro Met Phe
   1               5
<210> 27
   <211> 17
   <212> PRT
   <213> Artificial sequence
<220>
   <223> VH CDR2 sequence of the LI33 antibody
<400> 27
   Trp Ile Gly Pro Ser Gly Gly Ile Thr Lys Tyr Ala Asp Ser Val Lys
   1 5 10 15
   Gly
<210> 28
   <211> 10
   <212> PRT
   <213> Artificial sequence
<220>
   <223> VH CDR3 sequence of the LI33 antibody
<400> 28
   Glu Gly His Asn Asp Trp Tyr Phe Asp Leu
   1 5 10
<210> 29
   <211> 11
   <212> PRT
   <213> Artificial sequence
<220>
   <223> VL CDR1 sequence of the LI33 antibody
<400> 29
   Arg Ala Ser Gln Ser Val Ser Ser Tyr Leu Ala
   1 5 10
<210> 30
   <211> 7
   <212> PRT
   <213> Artificial sequence
<220>
   <223> VL CDR2 sequence of the LI33 antibody
<400> 30
   Asp Ala Ser Asn Arg Ala Thr
   1 5
<210> 31
   <211> 9
   <212> PRT
   <213> Artificial sequence
<220>
   <223> VL CDR3 sequence of the LI33 antibody
<400> 31
   Gln Gln Tyr Asp Lys Trp Pro Leu Thr
   1 5
<210> 32
   <211> 55
   <212> RNA
   <213> Artificial sequence
<220>
   <223> shRNA sequence of LINGO-1 antagonist polynucleotide
<400> 32
   ugaucgucau ccugcuagac uucaagagag ucuagcagga ugacgaucuu uuuuc 55

## Description

### BACKGROUND OF THE INVENTION

### Field of the Invention

This invention relates to neurology, neurobiology, molecular biology and pharmacology. More particularly, this invention relates to LINGO-1 antagonists and TrkB agonists for use in treating pressure induced optic neuropathies, i.e. glaucoma.

### Background Art

Optical neuropathies are a group of eye diseases encompassing various clinical presentations and etiologies. Glaucoma is an exemplary optical neuropathy which includes pathological changes in the optic nerve, visible on the optic disk, and corresponding visual field loss, resulting in blindness if untreated. Glaucoma is associated with increased intraocular pressure, but other factors are involved.

Current therapies for glaucoma are directed at decreasing intraocular pressure. Medical therapy includes topical ophthalmic drops or oral medications that reduce the production or increase the outflow of intraocular fluid. However, these drug therapies for glaucoma are sometimes associated with significant side effects, such as headache, blurred vision, allergic reactions, death from cardiopulmonary complications and potential interactions with other drugs. Surgical therapies also are used, but they also have numerous disadvantages and modest success rates.

Accordingly, there remains a need for additional treatment methods for pressure induced optical neuropathies, including glaucoma and other conditions characterized by degeneration or death of retinal ganglion cells (RGCs).

### BRIEF SUMMARY OF THE INVENTION

The present invention is based on the discovery that LINGO-1 interacts with and inhibits TrkB in neurons. After ocular hypertension, the TrkB ligand brain-derived neurotrophic factor (BDNF) is upregnated BDNF promotes TrkB phosphorylation and activation of a cell survival signaling pathway. However, LINGO-1 is also upregulated after ocular hypertension. The experiments described here use LINGO-1 antagonists to show that LINGO-1 inhibits the phosphorylation and activation of TrkB, thereby inhibiting the cell survival signaling pathway. The experiments described here also show that LINGO-1 promotes activity of the JNK signaling pathway, which is associated with cell death. Antagonists of LINGO-1 and agonists of TrkB, therefore promote cell survival.

Based on the disclosure that is contained herein, the present invention provides a LINGO-1 antagonist for use in the treatment of glaucoma, wherein the treatment comprises administration of the LINGO-1 antagonist in combination with a TrkB agonist; wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody or fragment thereof;
(iv) a TrkB agonist polynucleotide; and
(v) a TrkB agonist aptamer.

In a related aspect, the invention provides a TrkB agonist for use in the treatment of glaucoma, wherein the treatment comprises administration of the TrkB agonist in combination with a LINGO-1 antagonist; wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and BAG, optionally fused to a non-TrkB-agonist heterologous polypeptide;
(iii) a TrkB agonist antibody or fragment thereof;
(iv) a TrkB agonist polynucleotide; and
(v) a TrkB agonist aptamer.

In a related aspect, the present invention provides a LINGO-1 antagonist and TrkB agonist for use in the treatment of glaucoma, wherein the treatment comprises administration of the TrkB agonist and the LINGO-1 antagonist simultaneously, separately or sequentially; wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody or fragment thereof;
(iv) a TrkB agonist polynucleotide; and
(v) a TrkB agonist aptamer.

The present invention and embodiments thereof are set out in the appended claims.

The TrkB agonist may be any molecule which increases the ability of TrkB to promote neuronal survival. In certain embodiments, the TrkB agonist is selected from the group consisting of a TrkB agonist compound, a TrkB agonist polypeptide, a TrkB agonist antibody or fragment thereof, a TrkB agonist polynucleotide, a TrkB aptamer, or a combination of two or more TrkB agonists.

In certain embodiments, the TrkB agonist is a TrkB agonist compound. TrkB agonist compounds for use according to the invention include, but are not limited to, L-783,281 adenosine and CGS 21680.

In certain embodiments, the TrkB agonist is a TrkB agonist polypeptide. Certain TrkB agonist polypeptides for use according to the invention include BDNF, NT-3 and NT-4/5. In certain embodiments, the TrkB agonist polypeptide comprises SEQ ID NO:4. In some embodiments, the TrkB agonist is a fusion polypeptide or conjugate comprising a non-TrkB-agonist heterologous polypeptide or polymer. In some embodiments, the non TrkB-agonist polypeptide or polymer is selected from the group consisting of polyethylene glycol, a 1-acyl-glycerol derivative, an antibody Ig peptide, a serum albumin peptide, a targeting peptide, a reporter peptide, and a purification-facilitating peptide. In some embodiments, the antibody Ig peptide is a hinge and Fc peptide.

In alternative embodiments, the TrkB agonist is an antibody or fragment thereof which binds to a TrkB polypeptide. TrkB agonist antibodies for use in the methods of the present invention include, but are not limited to, 6E2, 7F5, 11E, 16E11, 17D11, 19E12, 29D7.

In other embodiments, the TrkB agonist is TrkB agonist polynucleotide, such as a peptide or protein encoding polynucleotide, or an apatamer.

In additional embodiments, the TrkB agonist is a TrkB aptamer. A TrkB aptamer is a small polynucleotide which binds TrkB and promotes the ability of TrkB to increase neuronal survival.

The LINGO-1 antagonist may be any molecule which interferes with the ability of LINGO-1 to negatively regulate neuronal survival and/or to bind to TrkB and/or to inhibit or decrease TrkB phosphorylation. In certain embodiments, the LINGO-1 antagonist is selected from the group consisting of a LINGO-1 antagonist polypeptide, a LINGO-1 antagonist antibody or fragment thereof, a LINGO-1 antagonist polynucleotide (e.g. RNA interference), a LINGO-1 aptamer, or a combination of two or more LINGO-1 antagonists.

In certain embodiments, the LINGO-1 antagonist polypeptide is a soluble LINGO-1 polypeptide. Certain soluble LINGO-1 polypeptides for use according to the invention include but are not limited to, soluble LINGO-1 polypeptides which comprise or lack one or more of the following domains: (i) a LINGO-1 Leucine-Rich Repeat (LRR) domain, (ii) a LINGO-1 basic region C-terminal to the LRR domain, and (iii) a LINGO-1 immunoglobulin (Ig) domain. In some embodiments, the soluble LINGO-1 polypeptide lacks one or more of a LINGO-1 Ig domain, a LINGO-1 LRR domain, a transmembrane domain, and a cytoplasmic domain. Additional LINGO-1 soluble polypeptides for use according to the invention include polypeptides which lack a transmembrane domain and a cytoplasmic domain. In some embodiments, the soluble LINGO-1 polypeptide comprises a LINGO-1 LRR domain and lacks a LINGO-1 Ig domain, a LINGO-1 basic region, a transmembrane domain, and a cytoplasmic domain. In some embodiments, the soluble LINGO-1 polypeptide comprises amino acid residues 34-532 of SEQ ID NO: 2 or 36-532 of SEQ ID NO:2.

In some embodiments, the LINGO-1 antagonist is a fusion polypeptide comprising a non-LINGO-1-antagonist heterologous polypeptide. In some embodiments, the non-LINGO-1-antagonist polypeptide is selected from the group consisting of an antibody Ig polypeptide, a serum albumin polypeptide, a targeting polypeptide, a reporter polypeptide, and a purification-facilitating polypeptide. In some embodiments, the antibody Ig polypeptide is a hinge and Fc polypeptide.

In alternative embodiment, the LINGO-1 antagonist is an antibody or fragment thereof which binds to a LINGO-1 polypeptide comprising one or more of the following LINGO-1 domains: (i) a LINGO-1 Leucine-Rich Repeat (LRR) domain, (ii) a LINGO-1 basic region C-terminal to the LRR domain, and (iii) a LINGO-1 immunoglobulin (Ig) domain. Additionally, the LINGO-1 antagonist antibody or fragment thereof specifically binds to an epitope within a polypeptide comprising a LINGO-1 polypeptide fragment as described herein. In additional embodiments, the LINGO-1 antagonist antibody or fragment there of is selected from the group consisting of 201', 3A3, 3A6, 3B5, 1A7, 1D5, 1G7, 2B10, 2C11, 2F3, 3P1B1.1F9, 3P1D10.2C3, 3P1E11.3B7, 3P2C6.3G10.2H7, 3P2C9.2G4, 3P4A6.1D9, 3P4A1.2B9, 3P4C2.2D2, 3P4C5.1D8, 3P4C8.2G9, 6P4F4.1Ds, 6P4F4.1F9, 7P1D5.1G9, 1B6.4, 2C7.2, 2D6.1, 2F7.3, 2H3.2, 3C11.1, 3E3.1, 3H11.2, 3G8.1, 2B8.1, 3B5.230-C12 (Li01), 38-D01 (Li02), 35-E04 (Li03), 36-C09 (Li04), 30-A11 (Li05), 34-F02 (Li06), 29-E07 (Li07), 34-G04 (Li08), 36-A12 (Li09), 28-D02 (Li10), 30-B01 (Li11), 34-B03 (Li12), Li13, Li32, Li33, Li34, 3383 (L1a.1), 3495(L1a.2), 3563 (L1a.3), 3564 (L1a.4), 3565 (L1a.5), 3566 (L1a.6), 3567 (L1a.7), 3568 (L1a.8), 3569 (L1a.9), 3570 (L1a.10), 3571 (L1a.11), 3582 (L1a.12), 1968 (L1a.13), 3011, 3012, 3013, 3418, 3422, 3562, D05, D07, D08, D10 and D11. The LINGO-1 antagonist can also be an antigen-binging fragment of any one of these antibodies or a combination of two or more antibodies or fragments thereof.

In other embodiments, the LINGO-1 antagonist is a LINGO-1 "antagonist polynucleotide such as an antisense polynucleotide, an aptamer, a ribozyme, a small interfering RNA (siRNA), or a small-hairpin RNA (shRNA).

In additional embodiments, the LINGO-1 antagonist is a LINGO-1 aptamer. A LINGO-1 aptamer is a small polypeptide or a polynucleotide which binds LINGO-1 and interferes with LINGO-1 and TrkB interaction and/or promotes or increases TrkB phosphorylation.

In some instances the TrkB agonist and/or LINGO-1 antagonist is administered by a method comprising (a) introducing into CNS neurons a polynucleotide which encodes the TrkB agonist and/or the LINGO-1 antagonist through operable association with an expression control sequence; and (b) allowing expression of said TrkB agonist and/or LINGO-1 antagonist. In some instances the CNS neurons are in a mammal and said introducing comprises (a) administering to said mammal a polynucleotide which encodes a TrkB agonist and/or a LINGO-1 antagonist through operable association with an expression control sequence. In some instances, the cultured host cell is derived from the mammal to be treated. In certain instances, the polynucleotide is introduced into the host cell or CNS neuron via transfection, electroporation, viral transduction or direct microinjection.

In certain embodiments the TrkB agonist and/or the LINGO-1 antagonist is a poynucleotide that can be administered to a mammal, at or near the site of the disease, disorder or injury. In some instances, the polynucleotide is administered as an expression vector. In certain instances, the vector is a viral vector which is selected from the group consisting of an adenoviral vector, an alphavirus vector, an enterovirus vector, a pestivirus vector, a lentiviral vector, a baculoviral vector, a herpesvirus vector (*e.g.* an Epstein Barr viral vector, or a herpes simplex viral vector) a papovaviral vector, a poxvirus vector (*e.g*. a vaccinia viral vector) and a parvovirus. In some instances, the vector is administered by a route selected from the group consisting of topical administration, intraocular administration, and parenteral administration (*e.g.* intravenous, intraarterial, intramuscular, intracardiac, subcutaneous, intradermal, intrathecal, intraperitoneal).

### BRIEF DESCRIPTION OF THE DRAWINGS/FIGURES

Figures 1A and B - Schematic summarizing the 2-week (FIG 1A) and 4-week (FIG 1B) experimental ocular hypertension models used in the examples.
Figure 2 - Schematic showing sections used to quantitate retinal ganglion cells (RGCs) in flat-mounted retinas. RGCs were quantified under an eyepiece grid of 200 x 200 µm² along the median line of each quadrant, starting from the optic disc to the border at 500 µm intervals.
Figure 3 - Images of aqueous veins prior to (top) and immediately after (bottom) laser photocoagulation.
Figures 4A and B - LINGO-1 expression in normal and injured rat retina sections.
Figure 5 - Western blot of LINGO-1 in normal and injured rat retina and quantitation of the same.
Figure 6 - Measurements of intraocular pressure in left (normal) and right (injured) eyes treated with PBS, control protein, anti-LINGO-1 antibody (1A7) or soluble LINGO-1 (LINGO-1-Fc).
Figures 7A and B - Quantitation of the number (FIG 7A) and density (FIG 7B) of surviving RGCs two weeks and four weeks after injury and treated with PBS, control protein, 1A7 or LINGO-1-Fc.
Figures 8A and B - Images of microstructures in RGCs (FIG 8A) and cells of the inner plexiform layer (FIG 8B) in normal animals and injured animals treated with PBS, LINGO-1-Fc or 1A7.
Figure 9 - Quantitation of surviving RGCs grown in vitro and exposed to control protein, LINGO-1-Fc, BDNF and control protein, or BDNF and LINGO-1-Fc.
Figure 10 - Western blot of BDNF in normal retina, in injured retina with no treatment and in injured retina treated with PBS, LINGO-1-Fc or 1A7 and quantitation of the same.
Figures 11A and B - Quantitation of the number (FIG 11A) and density (FIG 11B) of RGCs in injured eyes treated with PBS, anti-BDNF antibody, 1A7, 1A7 and anti-BDNF antibody, LINGO-1-Fc or LINGO-1-Fc and anti-BDNF antibody.
Figures 12A and B - Western blots of TrkB and LINGO-1 in anti-LINGO-1 immunoprecipitates from 293 cells co-expressing TrkB and LINGO-1 (FIG 12A). Western blots of phospho-TrkB, total TrkB and LINGO-1 in anti-TrkB immunoprecipitates from cells with and without LINGO-1 expression and with and without BDNF stimulation and quantitation of the same (FIG 12B).
Figures 13A and B - Western blot of LINGO-1 in anti-TrkB, anti-LINGO-1 and anti-control protein immunoprecipitates from normal and injured retinal lysates (FIG 13A). LINGO-1 and phospho-TrkB immunostaining in retinal sections (FIG 13B).
Figures 14A and B - Western blot of phospho-TrkB and total TrkB in normal eyes and injured eyes treated with PBS, LINGO-1-Fc or 1A7 and quantitation of the same (FIG 14A). Western blot of phospho-TrkB and total TrkB in normal eyes and injured eyes treated with BDNF, BDNF and LINGO-1-Fc or BDNF and 1A7 (FIG 14B).
Figure 15 - Western blot of phospho-Akt and total Akt in normal and injured eyes with or without LINGO-1-Fc treatment and quantitation of the same.
Figure 16 - pAkt immunostaining in retinal sections.
Figures 17A and B - Quantitation of the number (FIG. 17A) and density (FIG. 17B) of RGCs in injured eyes treated with PBS, LY294002 (an inhibitor of the PI3K/Akt pathway), LINGO-1-Fc, or LY294002 and LINGO-1-Fc.
Figure 18 - Measurement of intraocular pressure in left (normal) and right (injured) eyes in animals treated with LINGO-1-Fc and LY294002 or LY294002 alone.
Figure 19 - Western blot of phospho-JNK-2, phospho-JNK-1, total JNK-2 and total JNK-1 in normal and injured eyes treated with PBS, LINGO-1-Fc and 1A7 and quantitation of the same.

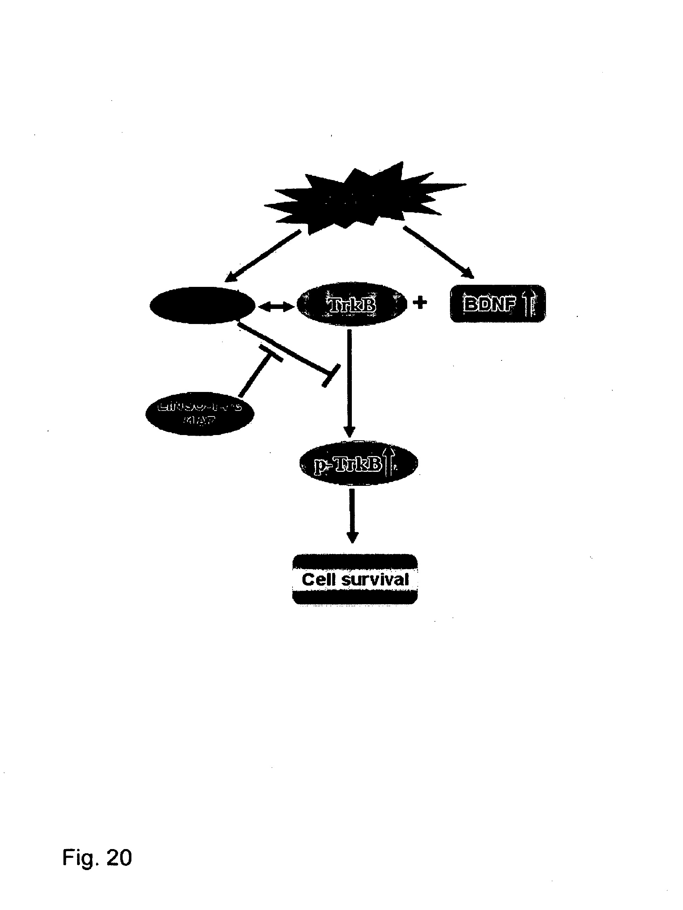

Figure 20 - A schematic showing the proposed molecular mechanism. Elevations in intraocular pressure result in increased levels of both LINGO-1 and BDNF. BDNF promotes phosphorylation of TrkB and activation of a cell survival signaling pathway, but the LINGO-1 inhibits this activity. LINGO-1 antagonists, such as a 1A7 or LINGO-1-Fc, promote cell survival by interfering with the ability of LINGO-1 to prevent TrkB phosphorylation in response to BDNF signaling.
Figure 21 - Western blot of GTP-RhoA and total RhoA in normal and injured eyes treated with PBS or LINGO-1-Fc and quantitation of the same.

### DETAILED DESCRIPTION OF THE INVENTION

### Definitions

Unless defined otherwise, all technical and scientific terms used herein have the same meaning as commonly understood by one of ordinary skill in the art to which this invention belongs. In case of conflict, the present application including the definitions will control. Unless otherwise required by context, singular terms shall include pluralities and plural terms shall include the singular.

Although methods and materials similar or equivalent to those described herein can be used in practice or testing of the present invention, suitable methods and materials are described below. The materials, methods and examples are illustrative only and are not intended to be limiting. Other features and advantages of the invention will be apparent from the detailed description and from the claims.

In order to further define this invention, the following terms and definitions are provided.

It is to be noted that the term "a" or "an" entity, refers to one or more of that entity; for example, "an immunoglobulin molecule," is understood to represent one or more immunoglobulin molecules. As such, the terms "a" (or "an"), "one or more," and "at least one" can be used interchangeably herein.

Throughout this specification and claims, the word "comprise," or variations such as "comprises" or "comprising," indicate the inclusion of any recited integer or group of integers but not the exclusion of any other integer or group of integers.. The term "comprising" is inclusive or open-ended and does not exclude additional, unrecited elements or method steps. The phrase "consisting essentially of" indicates the inclusion of the specified materials or steps as well as those which do not materially affect the basic and novel characteristics of the claimed invention. As used herein, the term "consisting" refers only to indicated material or method steps.

As used herein, a "therapeutically effective amount" refers to an amount effective, at dosages and for periods of time necessary, to achieve a desired therapeutic result. A therapeutic result may be, e.g., lessening of symptoms, prolonged survival, improved mobility, and the like. A therapeutic result need not be a "cure".

As used herein, the term "treatment" or "treating" refers to the administration of an agent to an animal in order to ameliorate or lessen the symptoms of a disease. Additionally, the terms "treatment" or "treating" refers to the administration of an agent to an animal to prevent the progression of a disease.

As used herein, a "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

As used herein, a "polynucleotide" can contain a nucleic acid sequence of a full length cDNA sequence and may include untranslated 5' and 3' sequences, the coding region or fragments or and variants of the nucleic acid sequence. The polynucleotide can be composed of any polyribonucleotide or polydeoxyribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotides can be composed of triple-stranded regions comprising RNA or DNA or both RNA and DNA. Polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus; "polynucleotide" embraces chemically, enzymatically, or metabolically modified forms.

In the present invention, a "polypeptide" can be composed of amino acids joined to each other by peptide bonds or modified peptide bonds, i.e., peptide isosteres, and may contain amino acids other than the 20 gene-encoded amino acids (e.g. non-naturally occurring amino acids). As used to describe the present invention, the terms "peptide" and "polypeptide" may be used interchangeably. The polypeptides for use according to the invention may be modified by either natural processes, such as posttranslational processing, or by chemical modification techniques which are well known in the art. Such modifications are well described in basic texts and in more detailed monographs, as well as in a voluminous research literature. Modifications can occur anywhere in the polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formulation, gamma carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, Proteins - Structure And Molecular Properties, 2nd Ed., T.E. Creighton, W.H. Freeman and Company, New York (1993); Posttranslational Covalent Modification of Proteins, B.C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

The terms "fragment," "variant," "derivative" and "analog" when referring to a LINGO-1 antagonist or a TrkB agonist for use according to the present invention include any antagonist or agonist molecules which retain at least some ability to inhibits LINGO-1 activity or tq promote TrkB activity. LINGO-1 antagonists and TrkB agonists as described herein may include fragment, variant, or derivative molecules therein without limitation, so long as the LINGO-1 antagonist or TrkB agonist still serves its function. LINGO-1 antagonist or TrkB agonist polypeptides for use according to the invention may include LINGO-1 or TrkB-agonist proteolytic fragments, deletion fragments and in particular, fragments which more easily reach the site of action when delivered to an animal. Polypeptide fragments further include any portion of the polypeptide which comprises an antigenic or immunogenic epitope of the native polypeptide, including linear as well as three-dimensional epitopes. LINGO-1 or TrkB-agonist polypeptides for use according to the invention may comprise variant LINGO-1 or TrkB-agonist regions, including fragments as described above, and also polypeptides with altered amino acid sequences due to amino acid substitutions, deletions, or insertions. Variants may occur naturally, such as an allelic variant. By an "allelic variant" is intended alternate forms of a gene occupying a given locus on a chromosome of an organism. Genes II, Lewin, B., ed., John Wiley & Sons, New York (1985). Non-naturally occurring variants may be produced using art-known mutagenesis techniques. LINGO-1 or TrkB-agonist polypeptides may comprise conservative or non-conservative amino acid substitutions, deletions or additions. LINGO-1 antagonists or TrkB agonists for use according to the present invention may also include derivative molecules. For example, LINGO-1 or TrkB agonists for use according to the present invention may include LINGO-1 or TrkB-agonist regions which have been altered so as to exhibit additional features not found on the native polypeptide. Examples include fusion proteins and protein conjugates.

In the present invention, a "polypeptide fragment" refers to a short amino acid sequence of a LINGO-1 or TrkB-agonist polypeptide. Protein fragments may be "freestanding," or comprised within a larger polypeptide of which the fragment forms a part or region Representative examples of polypeptide fragments for use according to the invention include, for example, fragments comprising about 5 amino acids, about 10 amino acids, about 15 amino acids, about 20 amino acids, about 30 amino acids, about 40 amino acids, about 50 amino acids, about 60 amino acids, about 70 amino acids, about 80 amino acids, about 90 amino acids, and about 100 amino acids or more in length.

In certain embodiments, LINGO-1 antagonists or TrkB agonists for use in the methods disclosed herein are "antibody" or "immunoglobulin" molecules, or immunospecific fragments thereof, *e.g.,* naturally occurring antibody or immunoglobulin molecules or engineered antibody molecules or fragments that bind antigen in a manner similar to antibody molecules. The terms "antibody" and "immunoglobulin" are used interchangeably herein. Additionally, immunoglobulin molecules used in the invention are also described as "immunospecific" or "antigen-specific" or "antigen-binding" molecules and are used interchangeably to refer to antibody molecules and fragments thereof. An antibody or immunoglobulin comprises at least the variable domain of a heavy chain, and normally comprises at least the variable domains of a heavy chain and a light chain. Basic immunoglobulin structures in vertebrate systems are relatively well understood. *See, e.g.,* Harlow et al., Antibodies: A Laboratory Manual, (Cold Spring Harbor Laboratory Press, 2nd ed. 1988).

As will be discussed in more detail below, the term "immunoglobulin" comprises five broad classes of polypeptides that can be distinguished biochemically. All five classes are clearly within the scope of the present invention, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Both the light and heavy chains are divided into regions of structural and functional homology. The terms "constant" and "variable" are used functionally. In this regard, it will be appreciated that the variable domains of both the light (V_{L}) and heavy (V_{H}) chain portions determine antigen recognition and specificity. Conversely, the constant domains of the light chain (C_{L}) and the heavy chain (C_{H}1, C_{H}2 or C_{H}3) confer important biological properties such as secretion, transplacental mobility, Fc receptor binding, complement binding, and the like. By convention the numbering of the constant region domains increases as they become more distal from the antigen binding site or amino-terminus of the antibody. The N-terminal portion is a variable region and at the C-terminal portion is a constant region; the C_{H}3 and C_{L} domains actually comprise the carboxy-terminus of the heavy and light chain, respectively.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. Those skilled in the art will appreciate that heavy chains are classified as gamma, mu, alpha, delta, or epsilon, (γ, µ, α, δ, ε) with some subclasses among them (*e.g.,* γ1-γ4). It is the nature of this chain that determines the "class" of the antibody as IgG, IgM, IgA IgG, or IgE, respectively. The immunoglobulin subclasses (isotypes) *e.g.,* IgG₁, IgG₂, IgG₃, IgG₄, IgA₁, etc. are well characterized and are known to confer functional specialization. Modified versions of each of these classes and isotypes are readily discernable to the skilled artisan in view of the instant disclosure and, accordingly, are within the scope of the instant invention.

As indicated above, the variable region allows the antibody to selectively recognize and specifically bind epitopes on antigens. That is, the V_{L} domain and V_{H} domain of an antibody combine to form the variable region that defines a three dimensional antigen binding site. This quaternary antibody structure forms the antigen binding site present at the end of each arm of the Y. More specifically, the antigen binding site is defined by three complementary determining regions (CDRs) on each of the V_{H} and V_{L} chains. In some instances, *e.g*., certain immunoglobulin molecules derived from camelid species or engineered based on camelid immunoglobulins, a complete immunoglobulin molecule may consist of heavy chains only, with no light chains. See, *e.g.,* Hamers-Casterman et al., Nature 363:446-448 (1993).

In naturally occurring antibodies, the six "complementarity determining regions" or "CDRs" present in each antigen binding domain are short, non-contiguous sequences of amino acids that are specifically positioned to form the antigen binding domain as the antibody assumes its three dimensional configuration in an aqueous environment. The remainder of the amino acids in the antigen binding domains, referred to as "framework" regions, show less inter-molecular variability. The framework regions largely adopt a β-sheet conformation and the CDRs form loops which connect, and in some cases form part of, the β-sheet structure. Thus, framework regions act to form a scaffold that provides for positioning the CDRs in correct orientation by inter-chain, non-covalent interactions. The antigen binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined (see, "Sequences of Proteins of Immunological Interest," Kabat, E., et al., U.S. Department of Health and Human Services, (1983); and Chothia and Lesk, J. Mol. Biol., 196:901-917 (1987)).

In camelid species, however, the heavy chain variable region, referred to as V_{H}H, forms the entire CDR. The main differences between camelid V_{H}H variable regions and those derived from conventional antibodies (V_{H}) include (a) more hydrophobic amino acids in the light chain contact surface of V_{H} as compared to the corresponding region in V_{H}H, (b) a longer CDR3 in V_{H}H, and (c) the frequent occurrence of a disulfide bond between CDR1 and CDR3 in V_{R}H.

In one embodiment, an antigen binding molecule for use in the invention comprises at least one heavy or light chain CDR of an antibody molecule. In another embodiment, an antigen binding molecule for use in the invention comprises at least two CDRs from one or more antibody molecules. In another embodiment, an antigen binding molecule for use in the invention comprises at least three CDRs from one or more antibody molecules. In another embodiment, an antigen binding molecule for use in the invention comprises at least four CDRs from one or more antibody molecules. In another embodiment, an antigen binding molecule for use in the invention comprises at least five CDRs from one or more antibody molecules. In another embodiment, an antigen binding molecule for use in the invention comprises at least six CDRs from one or more antibody molecules. Exemplary antibody molecules comprising at least one CDR that can be included in the subject antigen binding molecules are known in the art and exemplary molecules are described herein.

Antibodies or immunospecific fragments thereof for use in the invention include, but are not limited to, polyclonal, monoclonal, multispecific, human, humanized, primatized, or chimeric antibodies, single chain antibodies, epitope-binding fragments, *e.g.,* Fab, Fab' and F(ab')2, Fd, Fvs, single-chain Fvs (scFv), single-chain antibodies, disulfide-linked Fvs (sdFv), fragments comprising either a VL or VH domain, fragments produced by a Fab expression library, and anti-idiotypic (anti-Id) antibodies (including, *e.g.,* anti-Id antibodies to binding molecules disclosed herein). ScFv molecules are known in the art and are described, *e.g*., in US patent 5,892,019. Immunoglobulin or antibody molecules for use according to the invention can be of any type (*e.g.,* IgG, IgE, IgM, IgD, IgA, and IgY), class (*e.g*., IgG₁, IgG₂, IgG₃, IgG₄, IgA₁ and IgA₂) or subclass of immunoglobulin molecule.

Antibody fragments, including single-chain antibodies, may comprise the variable region(s) alone or in combination with the entirety or a portion of the following: hinge region, C_{H}1, C_{H}2, and C_{H}3 domains of the heavy chain, or C_{L} of the light chain. Also included in the invention are antigen-binding fragments also comprising any combination of variable region(s) with a hinge region, C_{H}1, C_{H}2, C_{H}3, or C_{L} domain. Antibodies or immunospecific fragments thereof for use in the methods disclosed herein may be from any animal origin including birds and mammals. The antibodies can be human, murine, donkey, rabbit, goat, guinea pig, camel, llama, horse, or chicken antibodies. In another embodiment, the variable region may be condricthoid in origin (*e.g.,* from sharks). As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described *infra* and, for example, in U.S. Pat. No. 5,939,598 by Kucherlapati e*t al*. Such antibodies also include variants that contain one or more amino acid substitutions.

As used herein, the term "heavy chain portion" includes amino acid sequences derived from an immunoglobulin heavy chain. A polypeptide comprising a heavy chain portion comprises at least one of: a C_{H}1 domain, a hinge (*e.g.,* upper, middle, and/or lower hinge region) domain, a C_{H}2 domain, a C_{H}3 domain, or a variant or fragment thereof. For example, a heavy chain portion may comprise a polypeptide chain comprising a C_{H}1 domain; a polypeptide chain comprising a C_{H}1 domain, at least a portion of a hinge domain, and a C_{H}2 domain; a polypeptide chain comprising a C_{H}1 domain and a C_{H}3 domain; a polypeptide chain comprising a C_{H}1 domain, at least a portion of a hinge domain, and a C_{H}3 domain, or a polypeptide chain comprising a C_{H}1 domain, at least a portion of a hinge domain, a C_{H}2 domain, and a C_{H}3 domain. The heavy chain portion may also include a polypeptide comprising a polypeptide chain comprising a C_{H}3 domain. Further, a binding polypeptide for use in the invention may lack at least a portion of a C_{H}2 domain (*e.g.,* all or part of a C_{H}2 domain). As set forth above, it will be understood by one of ordinary skill in the art that these domains (*e.g.,* the heavy chain portions) may be modified such that they vary in amino acid sequence from the naturally occurring immunoglobulin molecule.

In certain LINGO-1 antagonist or TrkB agonist antibodies or immunospecific fragments thereof for use in the methods disclosed herein, the heavy chain portions of one polypeptide chain of a multimer are identical to those on a second polypeptide chain of the multimer. Alternatively, heavy chain portion-containing monomers for use in the invention are not identical. For example, each monomer may comprise a different target binding site, forming, for example, a bispecific antibody.

The heavy chain portions of a binding polypeptide for use in the methods disclosed herein may be derived from different immunoglobulin molecules. For example, a heavy chain portion of a polypeptide may comprise a C_{H}1 domain derived from an IgG₁ molecule and a hinge region derived from an IgG₃ molecule. In another example, a heavy chain portion can comprise a hinge region derived, in part, from an IgG₁ molecule and, in part, from an IgG₃ molecule. In another example, a heavy chain portion can comprise a chimeric hinge derived, in part, from an IgG₁ molecule and, in part, from an IgG₄ molecule.

As used herein, the term "light chain portion" includes amino acid sequences derived from an immunoglobulin light chain. The light chain portion can comprise at least one of a V_{L} or C_{L} domain.

An isolated nucleic acid molecule encoding a non-natural variant of a polypeptide derived from an immunoglobulin (*e.g.,* an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. Conservative amino acid substitutions can be made at one or more non-essential amino acid residues.

Antibodies or immunospecific fragments thereof for use in the methods disclosed herein may also be described or specified in terms of their binding affinity to a polypeptide for use according to the invention. In some embodiments, binding affinities are those with a dissociation constant or Kd less than 5 x 10⁻² M, 10⁻² M, 5 x 10⁻³ M, 10⁻³ M, 5 x 10⁻⁴ M, 10⁻⁴ M, 5 x 10⁻⁵ M, 10⁻⁵ M, 5 x 10⁻⁶ M, 10⁻⁶ M, 5 x 10⁻⁷ M, 10⁻⁷ M, 5 x 10⁻⁸ M, 10⁻⁸ M, 5 x 10⁻⁹ M, 10⁻⁹ M, 5 x 10⁻¹⁰ M, 10⁻¹⁰ M, 5 x 10⁻¹¹ M, 10⁻¹¹ M, 5 x 10⁻¹² M, 10⁻¹² M, 5 x 10⁻¹³ M, 10⁻¹³ M, 5 x 10⁻¹⁴ M, 10⁻¹⁴ M, 5 x 10⁻¹⁵ M, or 10⁻¹⁵ M.

Antibodies or immunospecific fragments thereof for use in the methods disclosed herein act as antagonists of LINGO-1 or agonists of TrkB as described herein. For example, an antibody for use in the present invention may function as an antagonist, blocking or inhibiting the suppressive activity of the LINGO-1 polypeptide or as an agonist promoting the activity of TrkB.

As used herein, the term "chimeric antibody" will be held to mean any antibody wherein the immunoreactive region or site is obtained or derived from a first species and the constant region (which may be intact, partial or modified in accordance with the instant invention) is obtained from a second species. In certain embodiments the target binding region or site will be from a non-human source (*e.g.* mouse or primate) and the constant region is human.

As used herein, the term "engineered antibody" refers to an antibody in which the variable domain in either the heavy or light chain or both is altered by at least partial replacement of one or more CDRs from an antibody of known specificity and, if necessary, by partial framework region replacement and sequence changing. Although the CDRs may be derived from an antibody of the same class or even subclass as the antibody from which the framework regions are derived, it is envisaged that the CDRs will be derived from an antibody of different class or from an antibody from a different species. An engineered antibody in which one or more "donor" CDRs from a non-human antibody of known specificity is grafted into a human heavy or light chain framework region is referred to herein as a "humanized antibody." It may not be necessary to replace all of the CDRs with the complete CDRs from the donor variable region to transfer the antigen binding capacity of one variable domain to another. Rather, it may only be necessary to transfer those residues that are necessary to maintain the activity of the target binding site. Given the explanations set forth in, *e.g.,* U. S. Pat. Nos. 5,585,089, 5,693,761, 5,693,762, and 6,180,370, it will be well within the competence of those skilled in the art, either by carrying out routine experimentation or by trial and error testing to obtain a functional engineered or humanized antibody.

As used herein, the terms "linked," "fused" or "fusion" are used interchangeably. These terms refer to the joining together of two more elements or components, by whatever means including chemical conjugation or recombinant means. An "in-frame fusion" refers to the joining of two or more open reading frames (ORFs) to form a continuous longer ORF, in a manner that maintains the correct reading frame of the original ORFs. Thus, the resulting recombinant fusion protein is a single protein containing two ore more segments that correspond to polypeptides encoded by the original ORFs (which segments are not normally so joined in nature.) Although the reading frame is thus made continuous throughout the fused segments, the segments may be physically or spatially separated by, for example, in-frame linker sequence.

In the context of polypeptides, a "linear sequence" or a "sequence" is an order of amino acids in a polypeptide in an amino to carboxyl terminal direction in which residues that neighbor each other in the sequence are contiguous in the primary structure of the polypeptide.

The term "expression" as used herein refers to a process by which a DNA sequence is used for the production a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product and the translation of such mRNA into polypeptide(s). If the final desired product is biochemical, expression includes the creation of that biochemical and any precursors.

By "subject" or "individual" or "animal" or "patient" or "mammal," is meant any subject, particularly a mammalian subject, for whom diagnosis, prognosis, or therapy is desired. Mammalian subjects include, but are not limited to, humans, domestic animals, farm animals, zoo animals, sport animals, pet animals such as dogs, cats, guinea pigs, hamsters, rabbits, rats, mice; primates such as apes, monkeys, orangutans, and chimpanzees; canids such as foxes and wolves; felids such as lions, and tigers; equids such as horses, donkeys, and zebras; food animals such as cows, pigs, and sheep; ungulates such as deer and giraffes; bears; and so on. In certain embodiments, the mammal is a human subject.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

### LINGO-1 (Sp35/LRRN6)

Naturally occurring human LINGO-1 is a glycosylated nervous-system-specific protein consisting of 614 amino acids (SEQ ID NO: 2). The human LINGO-1 polypeptide contains an LRR domain consisting of 14 leucine-rich repeats (including N-and C-terminal caps), an Ig domain, a transmembrane region, and a cytoplasmic domain. The cytoplasmic domain contains a canonical tyrosine phosphorylation site. In addition, the naturally occurring LINGO-1 protein contains a signal sequence, a short basic region between the LRRCT and Ig domain, and a transmembrane region between the Ig domain and the cytoplasmic domain. The human LINGO-1 gene contains alternative translation start codons, so that six additional amino acids (MQVSKR; SEQ ID NO:7) may or may not be present at the N-terminus of the LINGO-1 signal sequence. Table 1 lists the LINGO-1 domains and other regions, according to amino acid residue number, based on the sequence of SEQ ID NO:2. As one of skill in the art will appreciate, the beginning and ending residues of the domains listed below may vary depending upon the computer modeling program used or the method used for determining the domain. The LINGO-1 polypeptide is characterized in more detail in PCT Publication No. WO 2004/085648 and U.S. Published Application No. 2006/0009388 A1.

**Table 1**

| **Domain or Region** | **Beginning Residue** | **Ending Residue** |
|---|---|---|
| Signal Sequence | 1 | 33 or 35 |
| LRRNT | 34 or 36 | 64 |
| LRR | 66 | 89 |
| LRR | 90 | 113 |
| LRR | 114 | 137 |
| LRR | 138 | 161 |
| LRR | 162 | 185 |
| LRR | 186 | 209 |
| LRR | 210 | 233 |
| LRR | 234 | 257 |
| LRR | 258 | 281 |
| LRR | 282 | 305 |
| LRR | 306 | 329 |
| LRR | 330 | 353 |
| LRRCT | 363 | 414 or 416 |
| Basic | 415 or 417 | 424 |
| Ig | 419 | 493 |
| Connecting sequence | 494 | 551 |
| Transmembrane | 552 | 576 |
| Cytoplasmic | 577 | 614 |

Tissue distribution and developmental expression of LINGO-1 have been studied in humans and rats. LINGO-1 biology has been studied in an experimental animal (rat) model. Expression of rat LINGO-1 is localized to nervous-system neurons and brain oligodendrocytes, as determined by northern blot and immuno-histochemical staining. Rat LINGO-1 mRNA expression level is regulated developmentally, peaking shortly after birth, *i.e.,* ca. postnatal day one. In a rat spinal cord transection injury model, LINGO-1 is up-regulated at the injury site, as determined by RT-PCR In addition, LINGO-1 has been shown to interact with Nogo66 Receptor (Nogo receptor). See, *e.g.,* International Patent Application No. PCT/US2004/00832, PCT Publication No. WO2004/08564.

LINGO-1 is an additional component of the Nogo Receptor-1-p75-Taj neurotrophin receptor complex. See Mi et al., Nat Neurosci. 7:221-228 (2004).

Unlike Nogo receptor 1, LINGO-1 gene expression is increased when adult nerve cells in the spinal cord are exposed to traumatic injuries, suggesting that LINGO-1 has an important biological role for CNS neurological function. Id.

The nucleotide sequence for the full-length human LINGO-1 molecule is as follows:

The polypeptide sequence for the full-length human LINGO-1 polypeptide is as follows:

### TrkB (NTRK2)

The neurotrophins are a small family of highly homologous growth factors responsible for differentiation, survival and function of neurons. In mammals, the known neurotrophins are nerve growth factor (NGF), brain-derived neurotrophic factor (BDNF), neurotrophin-3 (NT-3), neurotrophin-4 (NT-4), also known as NT-4/5 or NT-5, neurotrophin-6 (NT-6) and neurotrophin-7 (NT-7) (Barbacid, J. of Neurobiol. 25:1386-1403 (1994) and Nilsson et al. FEBS 424:285-290 (1998)). Neurotrophins bind two receptor types, the p75 neurotrophin receptor (p75NTR) and the three members (in mammals) of the Trk receptor family of tyrosine kinases (TrkA, TrkB and TrkC). Binding of a neurotrophin to a Trk receptor extracellular domain initiates a signal transduction pathway. The binding of the neurotrophin leads to dimerization and autophosphorylation of the receptor. Autophosphorylation of TrkB leads to activation of signaling pathways including mitogen-activated protein kinase (MAPK), phospholipase C-γ (PLC-γ) and phosphatidylinositol-3 kinase (PI3-K) (Yamada, J. Pharmacol. Sci. 91:267-270 (2003)).

The following nucleotide sequence was reported as the mRNA for human TrkB receptor and is accession number NM_006180 in Genbank.

The following polypeptide sequence was reported as the human TrkB polypeptide sequence (encoded by nucleotides 939-3455 of SEQ ID NO:3) and has the accession number NP_006171 in Genbank.

The following nucleotide sequence was reported as the mRNA for rat TrkB receptor and is accession number NM_012731 in Genbank.

The following polypeptide sequence was reported as the rat TrkB polypeptide sequence (encoded by nucleotides 665-3130 of SEQ ID NO:5) and has the accession number NP_036863 in Genbank.

Table 2 lists the TrkB domains and other regions, according to amino acid residue number, based on SEQ ID NO:4. As one of skill in the art will appreciate, the beginning and ending residues of the domains listed below may vary depending upon the computer modeling program used or the method used for determining the domain.

**Table 2**

| **Domain or Region** | **Beginning Residue** | **Ending Residue** |
|---|---|---|
| Signal Peptide | 1 | 31 |
| Cysteine-rich 1 | 32 | 67 |
| LRR | 68 | 139 |
| Cysteine-rich 2 | 140 | 195 |
| Ig-Like 1 | 214 | 270 |
| Ig-Like 2 | 301 | 365 |
| Transmembrane | 434 | 454 |
| Tyrosine Kinase Catalytic Domain | 552 | 828 |

### Methods Using Antagonists of LINGO-1

The present disclosure provides methods for inhibiting LINGO-1 and TrkB interaction in a cell comprising contacting a cell co-expressing LINGO-1 and TrkB with a LINGO-1 antagonist. A LINGO-1 antagonist may be a LINGO-1 antagonist polypeptide, a LINGO-1 antibody, a LINGO-1 antagonist polynucleotide, a LINGO-1 aptamer or a combination of two or more LINGO-1 antagonists.

Additional instances of the disclosure include methods for promoting TrkB phosphorylation in a cell, relative to the level of TrkB phosphorylation in the absence of a LINGO-1 antagonist, comprising contacting a cell co-expressing LINGO-1 and TrkB with a LINGO-1 antagonist. Similarly, the disclosure also provide methods for promoting TrkB signal transduction comprising contacting cells co-expressing LINGO-1 and TrkB with a LINGO-1 antagonist. The disclosure also includes methods for promoting TrkB phosphorylation comprising contacting CNS neurons with a LINGO-1 antagonist.

Further instances of the disclosure include methods for inhibiting JNK phosphorylation in a cell comprising contacting a cell co-expressing LINGO-1 and JNK with a LINGO-1 antagonist as well as methods for inhibiting JNK pathway signal transduction comprising contacting a cell co-expressing LINGO-1 and JNK with a LINGO-1 antagonist. Inhibiting JNK phosphorylation, according to the present disclosure, includes inhibition of JNK-1 phosphorylation, inhibition of JNK-2 phosphorylation or inhibition of JNK-1 and JNK-2 phosphorylation. Inhibiting JNK phosphorylation, according to the present disclosure, can include decreasing the overall quantity of phosphorylated JNK protein, or decreasing the length of time for which JNK proteins remain phosphorylated. The disclosure also includes methods for inhibiting JNK phosphorylation comprising contacting CNS neurons with a LINGO-1 antagonist.

An additional instance of the disclosure provides a method for promoting survival of retinal ganglion cells in a mammal displaying signs or symptoms of a pressure induced ocular neuropathy comprising administering to a mammal in need of such treatment an effective amount of a LINGO-1 antagonist and a carrier. In one instance the pressure induced ocular neuropathy is glaucoma.

### Methods Using Antagonists of LINGO-1 and TrkB Agonists

In some methods of the present disclosure both a LINGO-1 antagonist and a TrkB agonist are used. For example, one instance of the disclosure, includes a method for promoting survival of a neuron at risk of dying comprising contacting the neuron with an effective amount of a combination of a LINGO-1 antagonist and a TrkB agonist. In instances in which both a LINGO-1 antagonist and a TrkB agonist are used the term "effective amount" refers to the amount of the combination of the LINGO-1 antagonist and the TrkB agonist that is sufficient to produce the desired result. In some instances the amount of a LINGO-1 antagonist required to produce the desired effect maybe greater when the LINGO-1 antagonist is used alone than when it is used in combination with a TrkB agonist. Similarly, in some instances, the amount of a TrkB agonist that is rqeuired to produce the desired effect may be greater when the TrkB agonist is used alone than when it is used in combination with a LINGO-1 antagonist.

Another instance of the disclosure provides methods for treating a disease or disorder associated with neuronal cell death comprising administering to an animal in need of such treatment an effective amount of a combination of a LINGO-1 antagonist and a TrkB agonist. In some particular instances the disease or disorder can be a pressure induced optical neuropathy. Further instances of the disclosure include methods for promoting regeneration or survival of CNS neurons in a mammal displaying signs or symptoms of a condition involving neuronal cell death comprising administering to the mammal an effective amount of a combination of a LINGO-1 antagonist and a TrkB agonist. In some instances of the disclosure the disease or disorder is ALS, Huntington's disease, Alzheimer's disease, Parkinson's disease, diabetic neuropathy, stroke or hearing loss. In another particular instance the CNS neurons are sensory neurons such as retinal ganglion cells or hairy cells.

In embodiments of the invention that involve a combination of a LINGO-1 antagonist and a TrkB agonist, the LINGO-1 antagonist and TrkB agonist can be administered in a single composition or can be administered separately. In addition, the LINGO-1 antagonist and the TrkB agonist can be administered simultaneously or sequentially.

### LINGO-1 Antagonist and TrkB Agonist Compounds

LINGO-1 antagonists in the present invention include any chemical or synthetic compound which inhibits or decreases the activity of LINGO-1 compared to the activity of LINGO-1 in the absence of the antagonist compound.

TrkB agonists in the present invention include any chemical or synthetic compound which promotes or increases the activity of TrkB or promotes or increases the phosphorylation of TrkB when compared to the state of TrkB in the absence of the agonist compound.

TrkB agonist compounds include, but are not limited to neurotrophic factor mimetics. TrkB agonist compounds also include, but are not limited to L-783,281 adenosine, CGS 21680, etc. as reviewed in Pollack et al. Curr. Drug Targ- CNS and Neurol. Disorders 1:59-80 (2002).

In some embodiments, the TrkB agonist compounds are selective for TrkB and activate TrkB to a greater extent than TrkA or TrkC. In some embodiments, the TrkB agonist compounds are specific for TrkB and do not activate TrkA or TrkC. In addition, the TrkB agonist compounds can also be small molecules that mimic critical regions of neurotrophins. For example, the small molecule can be a mimetic of a BDNF β-turn loop. Particular examples of small molecule mimetics that may be used according to the invention are disclosed in U.S. Published Application No. 2007/0060526 A1.

One of ordinary skill in the art would know how to screen and test for LINGO-1 antagonist and TrkB agonist compounds which would be useful in the present invention, for example by screening for compounds that modify neuronal survival using assays described elsewhere herein.

### Soluble LINGO-1 Antagonist and TrkB Agonist Polypeptides

### Soluble LINGO-1 polypeptides

LINGO-1 antagonists to be used in the present invention include those polypeptides which block, inhibit or interfere with the biological function of naturally occurring LINGO-1. Specifically, soluble LINGO-1 polypeptides for use according to the present invention include fragments, variants, or derivatives thereof of a soluble LINGO-1 polypeptide. Table 1 above describes the various domains of the LINGO-1 polypeptide. Soluble LINGO-1 polypeptides lack the transmembrane domain and typically lack the intracellular domain of the LINGO-1 polypeptide. For example, certain soluble LINGO-1 polypeptides lack amino acids 552-576 which comprise the transmembrane domain of LINGO-1 and/or amino acids 577-614 which comprise the intracellular domain of LINGO-1. Additionally, certain soluble LINGO-1 polypeptides comprise the LRR domains, Ig domain, basic region and/or the entire extracellular domain (corresponding to amino acids 34 to 532 of SEQ ID NO: 2) of the LINGO-1 polypeptide. As one of skill in the art would appreciate, the entire extracellular domain of LINGO-1 may comprise additional or fewer amino acids on either the C-terminal or N-terminal end of the extracellular domain polypeptide.

As such, soluble LINGO-1 polypeptides for use in the present invention include, but are not limited to, a LINGO-1 polypeptide comprising, consisting essentially of, or consisting of amino acids 41 to 525 of SEQ ID NO:2; 40 to 526 of SEQ ID NO:2; 39 to 527 of SEQ ID NO:2; 38 to 528 of SEQ ID NO:2; 37 to 529 of SEQ ID NO:2; 36 to 530 of SEQ ID NO:2; 35 to 531 of SEQ ID NO:2; 34 to 531 of SEQ ID NO:2; 46 to 520 of SEQ ID NO:2; 45 to 521 of SEQ ID NO:2; 44 to 522 of SEQ ID NO:2; 43 to 523 of SEQ ID NO:2; and 42 to 524 of SEQ ID NO:2 or fragments, variants, or derivatives of such polypeptides. LINGO-1 polypeptide antagonists may include any combination of domains as described in Table 1.

Additional soluble LINGO-1 polypeptides for use in the present invention include, but are not limited to, a LINGO-1 polypeptide comprising, consisting essentially of, or consisting of amino acids 1 to 33 of SEQ ID NO:2; 1 to 35 of SEQ ID NO:2; 34 to 64 of SEQ ID NO:2; 36 to 64 of SEQ ID NO:2; 66 to 89 of SEQ ID NO:2; 90 to 113 of SEQ ID NO:2; 114 to 137 of SEQ ID NO:2; 138 to 161 of SEQ ID NO:2; 162 to 185 of SEQ ID NO:2; 186 to 209 of SEQ ID NO:2; 210 to 233 of SEQ ID NO:2; 234 to 257 of SEQ ID NO:2; 258 to 281 of SEQ ID NO:2; 282 to 305 of SEQ ID NO:2; 306 to 329 of SEQ ID NO:2; 330 to 353 of SEQ ID NO:2; 363 to 416 of SEQ ID NO:2; 417 to 424 of SEQ ID NO:2; 419 to 493 of SEQ ID NO:2; and 494 to 551 of SEQ ID NO:2 or fragments, variants, or derivatives of such polypeptides.

Further soluble LINGO-1 polypeptides for use in the present invention include, but are not limited to, a LINGO-1 polypeptide comprising, consisting essentially of, or consisting of amino acids 1 to 33 of SEQ ID NO:2; 1 to 35 of SEQ ID NO:2; 1 to 64 of SEQ ID NO:2; 1 to 89 of SEQ ID NO:2; 1 to 113 of SEQ ID NO:2; 1 to 137 of SEQ ID NO:2; 1 to 161 of SEQ ID NO:2; 1 to 185 of SEQ ID NO:2; 1 to 209 of SEQ ID NO:2; 1 to 233 of SEQ ID NO:2; 1 to 257 of SEQ ID NO:2; 1 to 281 of SEQ ID NO:2; 1 to 305 of SEQ ID NO:2; 1 to 329 of SEQ ID NO:2; 1 to 353 of SEQ ID NO:2; 1 to 416 of SEQ ID NO:2; 1 to 424 of SEQ ID NO:2; 1 to 493 of SEQ ID NO:2; 1 to 551 of SEQ ID NO:2; 1 to 531 of SEQ ID NO:2 and 1 to 532 of SEQ ID NO:2 or fragments, variants, or derivatives of such polypeptides.

Still further soluble LINGO-1 polypeptides for use in the present invention include, but are not limited to, a LINGO-1 polypeptide comprising, consisting essentially of, or consisting of amino acids 34 to 64 of SEQ ID NO:2; 34 to 89 of SEQ ID NO:2; 34 to 113 of SEQ ID NO:2; 34 to 137 of SEQ ID NO:2; 34 to 161 of SEQ ID NO:2; 34 to 185 of SEQ ID NO:2; 34 to 209 of SEQ ID NO:2; 34 to 233 of SEQ ID NO:2; 34 to 257 of SEQ ID NO:2; 34 to 281 of SEQ ID NO:2; 34 to 305 of SEQ ID NO:2; 34 to 329 of SEQ ID NO:2; 34 to 353 of SEQ ID NO:2; 34 to 416 of SEQ ID NO:2; 34 to 424 of SEQ ID NO:2; 34 to 493 of SEQ ID NO:2; and 34 to 551 of SEQ ID NO:2 or fragments, variants, or derivatives of such polypeptides.

Additional soluble LINGO-1 polypeptides for use in the present invention include, but are not limited to, a LINGO-1 polypeptide comprising, consisting essentially of, or consisting of amino acids 34 to 530 of SEQ ID NO:2; 34 to 531 of SEQ ID NO:2; 34 to 532 of SEQ ID NO:2; 34 to 533 of SEQ ID NO:2; 34 to 534 of SEQ ID NO:2; 34 to 535 of SEQ ID NO:2; 34 to 536 of SEQ ID NO:2; 34 to 537 of SEQ ID NO:2; 34 to 538 of SEQ ID NO:2; 34 to 539 of SEQ ID NO:2; 30 to 532 of SEQ ID NO:2; 31 to 532 of SEQ ID NO:2; 32 to 532 of SEQ ID NO:2; 33 to 532 of SEQ ID NO:2; 34 to 532 of SEQ ID NO:2; 35 to 532 of SEQ ID NO:2; 36 to 532 of SEQ ID NO:2; 30 to 531 of SEQ ID NO:2; 31 to 531 of SEQ ID NO:2; 32 to 531 of SEQ ID NO:2; 33 to 531 of SEQ ID NO:2; 34 to 531 of SEQ ID NO:2; 35 to 531 of SEQ ID NO:2; and 36 to 531 of SEQ ID NO:2 or fragments, variants, or derivatives of such polypeptides.

Still further soluble LINGO-1 polypeptides for use in the presents invention include, but are not limited to, a LINGO-1 polypeptide comprising, consisting essentially of, or consisting of amino acids 36 to 64 of SEQ ID NO:2; 36 to 89 of SEQ ID NO:2; 36 to 113 of SEQ ID NO:2; 36 to 137 of SEQ ID NO:2; 36 to 161 of SEQ ID NO:2; 36 to 185 of SEQ ID NO:2; 36 to 209 of SEQ ID NO:2; 36 to 233 of SEQ ID NO:2; 36 to 257 of SEQ ID NO:2; 36 to 281 of SEQ ID NO:2; 36 to 305 of SEQ ID NO:2; 36 to 329 of SEQ ID NO:2; 36 to 353 of SEQ ID NO:2; 36 to 416 of SEQ ID NO:2; 36 to 424 of SEQ ID NO:2; 36 to 493 of SEQ ID NO:2; and 36 to 551 of SEQ ID NO:2 or fragments, variants, or derivatives of such polypeptides.

Additional soluble LINGO-1 polypeptides for use in the present invention include, but are not limited to, a LINGO-1 polypeptide comprising, consisting essentially of, or consisting of amino acids 36 to 530 of SEQ ID NO:2; 36 to 531 of SEQ ID NO:2; 36 to 532 of SEQ ID NO:2; 36 to 533 of SEQ ID NO:2; 36 to 534 of SEQ ID NO:2; 36 to 535 of SEQ ID NO:2; 36 to 536 of SEQ ID NO:2; 36 to 537 of SEQ ID NO:2; 36 to 538 of SEQ ID NO:2; and 36 to 539 of SEQ ID NO:2; or fragments, variants, or derivatives of such polypeptides.

Additional soluble LINGO-1 polypeptides, fragments, variants or derivatives thereof include polypeptides comprising the Ig domain of LINGO-1. For example, a LINGO-1 polypeptide comprising, consisting essentially of, or consisting of amino acids 417 to 493 of SEQ ID NO:2; 417 to 494 of SEQ ID NO:2; 417 to 495 of SEQ ID NO:2; 417 to 496 of SEQ ID NO:2; 417 to 497 of SEQ ID NO:2; 417 to 498 of SEQ ID NO:2; 417 to 499 of SEQ ID NO:2; 417 to 500 of SEQ ID NO:2; 417 to 492 of SEQ ID NO:2; 417 to 491 of SEQ ID NO:2; 412 to 493 of SEQ ID NO:2; 413 to 493 of SEQ ID NO:2; 414 to 493 of SEQ ID NO:2; 415 to 493 of SEQ ID NO:2; 416 to 493 of SEQ ID NO:2; 411 to 493 of SEQ ID NO:2; 410 to 493 of SEQ ID NO:2; 410 to 494 of SEQ ID NO:2; 411 to 494 of SEQ ID NO:2; 412 to 494 of SEQ ID NO:2; 413 to 494 of SEQ ID NO:2; 414 to 494 of SEQ ID NO:2; 415 to 494 of SEQ ID NO:2; 416 to 494 of SEQ ID NO:2; 417 to 494 of SEQ ID NO:2; and 418 to 494 of SEQ ID NO:2 or fragments, variants, or derivatives of such polypeptides.

Soluble LINGO-1 polypeptides for use in the present invention also include combinations of two or more soluble LINGO-1 polypeptides disclosed herein. The two or more soluble LINGO-1 polypeptides for use in the invention may be fused together to form a single polypeptide comprising multiple LINGO-1 soluble polypeptides disclosed herein or may be individual soluble LINGO-1 polypeptides comprising a composition for use in then present invention.

Various exemplary soluble LINGO-1 polypeptides and methods and materials for - obtaining these molecules for practicing the present invention are described below and/or may be found, *e.g*., in PCT Publication Nos. WO2004/085648, WO 2006/002437, WO 2007/0059793, WO 2007/008547, WO 2007/056161 and WO 2007/064882 and U.S. - Published Application No. 2006/017673.

### TrkB agonist polypeptides

TrkB agonist polypeptides for use in the present invention include TrkB ligands : *i.e*. neurotrophins such as BDNF, NT-3 and NT-4/5. Additionally, TrkB agonist polypeptides for use according to the invention also include chimeric neurotrophin molecules. In some embodiments, the TrkB ligand polypeptides, fragments, variants or derivatives thereof are dimerized or multimerized. TrkB agonist polypeptides for use according to the invention can act as homodimers or heterodimers. TrkB agonist polypeptides for use according to the invention also include pan-neurotrophins such as those disclosed in Ibanez et al. EMBO 12:12:2281-2293 (1993). In particular, TrkB agonist polypeptides include domains of TrkB ligands that bind to TrkB or variants of such polypeptides. In one particular embodiment of the invention, the TrkB agonist is Brain-Derived Neurotrophic Factor (BDNF), or a fragment, variant or derivative thereof.

The Trkb agonist polypeptide may be a hairpin motif of a TrkB ligand as disclosed in U.S. Patent No. 7,205,387. Additionally TrkB ligand polypeptides, fragments, variants or derivatives thereof may be fused to other protein or peptide sequences and/or may be attached to water soluble polymers including polyethylene glycol as disclosed in U.S. Patent No. 5,770,577 or to 1-acyl-glycerol derivatives as disclosed in U.S. Patent No. 6,800,607. Additionally or alternatively, the TrkB ligands, fragments or variants thereof can be altered in order to increase stability upon administration, for example, by using variants with lower isoelectric points as described in U.S. Patent No. 6,723,701. TrkB ligands, as well as fragments and variants thereof, can be arranged in tandem and cyclized, for example, to form. "mini-neurotrophins, for example B_{AG}, as described in Williams at al. JBC 280:5862-5869 (2004).

Additionally, TrkB agonists polypeptides for use in the present invention include, but are not limited to TrkB polypeptides, fragments, variants or derivates thereof. TrkB polypeptides, fragments, variants or derivatives thereof for use in the present invention also include a TrkB polypeptide comprising, consisting essentially of, or consisting of the full-length TrkB protein fused to an immunoglobulin domain, for example, a TrkB polypeptide comprising, consisting essentially of, or consisting of amino acids 1 to 828 of SEQ ID NO:4; 32 to 828 of SEQ ID NO:4 fused to an IgG domain. Additional TrkB polypeptides, fragments, variants or derivatives thereof described herein may also be fused to an immunoglobulin domain. In some embodiments, the TrkB polypeptides for use according to the present invention are dimerized or multimerized. Trkb polypeptides for use according to the invention also include TrkB fragments, variants, isoforms or derivates that do not interact with LINGO-1, have an increased tendency for dimerization or multimerization, have an increased affinity for endogenous or non-endogenous ligands and/or have increased kinase activity compared to the polypeptide of SEQ ID NO:4.

Additional TrkB agonist polypeptides for use in the present invention also include a combination of two or more TrkB agonist polypeptides disclosed herein. The two or more TrkB agonist polypeptides for use in the invention may be fused together to form a single polypeptide comprising multiple TrkB agonist polypeptides disclosed herein or may be individual TrkB agonist polypeptides comprising a composition for use in the present invention.

### Soluble LINGO-1 antagonist and/or trkB agonist polypeptides

Soluble LINGO-1 antagonist and TrkB agonist polypeptides for use in the present invention described herein may be cyclic. Cyclization of the soluble LINGO-1 antagonist or TrkB agonist polypeptides reduces the conformational freedom of linear peptides and results in a more structurally constrained molecule. Many methods of peptide cyclization are known in the art, for example, "backbone to backbone" cyclization by the formation of an amide bond between the N-terminal and the C-terminal amino acid residues of the peptide. The "backbone to backbone" cyclization method includes the formation of disulfide bridges between two ω-thio amino acid residues (*e.g*. cysteine, homocysteine). Certain soluble LINGO-1 antagonist or TrkB agonist peptides for use according to the present invention include modifications on the N- and C- terminus -of the peptide to form a cyclic LINGO-1 antagonist or TrkB agonist polypeptide. Such modifications include, but are not limited to, cysteine residues, acetylated cysteine residues, cysteine residues with a NH₂ moiety and biotin. Other methods of peptide cyclization are described in Li & Roller, Curr. Top. Med. Chem. 3:325-341 (2002).

Soluble LINGO-1 antagonist or TrkB agonist polypeptides described herein may have various alterations such as substitutions, insertions or deletions. For examples, substitutions include, but are not limited to the following substitutions: valine at position 6 of the LINGO-1 polypeptide of SEQ ID NO:2 to methionine; serine at position 294 of the LINGO-1 polypeptide of SEQ ID NO:2 to glycine; valine at position 348 of the LINGO-1 polypeptide of SEQ ID NO:2 to alanine; arginine at position 419 of the LINGO-1 polypeptide to histidine; arginine at position 456 to glutamic acid; and histidine at position 458 of SEQ ID NO:2 to valine.

Corresponding fragments of soluble LINGO-1 antagonist or TrkB agonist polypeptides at least 70%, 75%, 80%, 85%, 90%, or 95% identical to polypeptides of SEQ ID NO:2 or SEQ ID NO:4 described herein are also contemplated.

As known in the art, "sequence identity" between two polypeptides is determined by comparing the amino acid sequence of one polypeptide to the sequence of a second polypeptide. When discussed herein, whether any particular polypeptide is at least about 70%, 75%, 80%, 85%, 90% or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full length of the reference polypeptide sequence and that gaps in homology of up to 5% of the total number of amino acids in the reference sequence are allowed.

Soluble LINGO-1 antagonist or TrkB-agonist polypeptides for use in the present invention may include any combination of two or more soluble LINGO-1 antagonist or TrkB agonist polypeptides.

### Antibodies ar Antigen-binding Fragments Thereof

In one embodiment, LINGO-1 antagonists or TrkB agonists for use in the invention include antibodies or antigen-binding fragments, variants or derivates therof which are LINGO-1 antagonists or TrkB agonists. For example, binding of certain LINGO-1 or TrkB antibodies to LINGO-1 or TrkB, as expressed by CNS neurons, promotes neuronal cell survival.

In some embodiments, the LINGO-1 antibody is the antibody 1A7. The 1A7 antibody has been described in International Application WO2007/008547, filed July 7, 2006. Sequences of the 1A7 antibody are shown in the table below.

**Table 3- 1A7 Antibody Sequences**

| | Polypeptide Sequence | SEQ ID NO: |
|---|---|---|
| VH | | 8 |
| VL | | 9 |

The antibody for use according to the invention can also be an antibody that includes one or more than one CDR of the 1A7 antibody. The following are the sequences for the VH CDR1, CDR2 and CDR3 regions of the 1A7 antibody respectively: NYGMN (SEQ ID NO:10), WINTDTGEPTYTEDFQG (SEQ ID NO:11), and EGVHFDY (SEQ ID NO:12). The following are the sequences for the VL CDR1, CDR2 and CDR3 regions of the 1A7 antibody respectively: SASSSVSYMH (SEQ ID NO:13), DTSKLAS (SEQ ID NO:14), and QQWSSNPFT (SEQ ID NO:15).

In some embodiments, the LINGO-1 antibody is the monoclonal antibody 3B5.2 (also referred to as 3B5), which can be produced from the hybridoma 2.P3B5.2. The 2.P3B5.2 hybridoma was deposited with the American Type Culture Collection (ATCC) in Mannasssas, VA on December 27, 2006, and the 3B5 antibody has been described in U.S. Provisional Patent Application No. 60/879,324, filed on January 9, 2007. in the table below.

**Table 4- 3B5 Antibody Sequences**

| | Polypeptide Sequence | SEQ ID NO: |
|---|---|---|
| VH | | 16 |
| VL | | 17 |

The antibody for use according to the invention can also be an antibody that includes one or more than one CDR of the 3B5 antibody. The following are the sequences for the VH CDR1, CDR2 and CDR3 regions of the 3B5, antibody respectively: SYWMH (SEQ ID NO:18), VIDPSDSYTNYNQKFRG (SEQ ID NO:19), and PYYGSHWFFDV (SEQ ID NO:20). The following are the sequences for the VL CDR1, CDR2 and CDR3 regions of the 3B5 antibody respectively: SASSRVSYVH (SEQ ID NO:21), DTSNLAS (SEQ ID NO:22), and QQWSTNPPT (SEQ ID NO:23).

In some embodiments, the LINGO-1 antibody is the monoclonal antibody LI33. The LI33 antibody has been described in U:S. Provisional Patent Application No. 60/879,324, filed on January 9, 2007.

Sequences of the LI33 antibody are shown in the table below.

**Table 5- LI33 Antibody Sequences**

| | Polypeptide Sequence | SEQ ID NO: |
|---|---|---|
| VH | | 24 |
| VL | | 25 |

The antibody for use according to the invention can also be an antibody that includes one or more than one CDR of the LI33 antibody. The following are the sequences for the VH CDR1, CDR2 and CDR3 regions of the LI33 antibody respectively: IYPMF (SEQ ID NO:26), WIGPSGGITKYADSVKG (SEQ ID NO:27), and EGHNDWYFDL (SEQ ID NO:28). The following are the sequences for the VL CDR1, CDR2 and CDR3 regions of the LI33 antibody respectively: RASQSVSSYLA (SEQ ID NO:29), DASNRAT (SEQ ID NO:30 and QQYDKWPLT (SEQ ID NO:31).

In another embodiment the LINGO-1 antibody is the monoclonal antibody 7P1D5.1G9, which can be produced from the hydridoma 7.P1D5.1.G9. The 7.P1D5.1.G9 hybridomas was deposited with the American Type Culture Collection (ATCC) in Mannasssas, VA on December 27,2006, and the 7P1D5.1G9 antibody is described in

Certain LINGO-1 antagonist antibodies for use in the methods described herein specifically or preferentially bind to a particular LINGO-1 polypeptide fragment or domain. Such LINGO-1 polypeptide fragments include, but are not limited to, a LINGO-1 polypeptide comprising, consisting essentially of, or consisting of amino acids 34 to 532; 34 to 417, 34 to 425, 34 to 493, 66 to 532, 66 to 417 (LRR domain), 66 to 426, 66 to 493, 66 to 532, 417 to 532, 417 to 425 (the LINGO-1 basic region), 417 to 424 (the LINGO-1 basic region), 417 to 493, 417 to 532, 419 to 493 (the LINGO-1 Ig region), or 425 to 532 of SEQ ID NO:2, or a LINGO-1 variant polypeptide at least 70%, 75%, 80%, 85%, 90%, or 95% identical to amino acids 34 to 532; 34 to 417, 34 to 425, 34 to 493, 66 to 532,66 to 417, 66 to 426, 66 to 493, 66 to 532, 417 to 532, 417 to 425 (the LINGO-1 basic region), 417 to 493, 417 to 532, 419 to 493 (the LINGO-1 Ig region), or 425 to 532 of SEQ ID NO:2.

Additional LINGO-1 peptide fragments to which certain LINGO-1 specific antibodies, or antigen-binding fragments, variants, or derivatives thereof for use in the present invention bind include, but are not limited to, those fragments comprising, consisting essentially of, or consisting of one or more leucine-rich-repeats (LRR) of LINGO-1. Such fragments, include, for example, fragments comprising, consisting essentially of, or consisting of amino acids 66 to 89, 66 to 113, 66 to 137, 90 to 113, 114 to 137, 138 to 161, 162 to 185, 186 to 209, 210 to 233, 234 to 257, 258 to 281, 282 to 305, 306 to 329, or 330 to 353 of SEQ ID NO:2. Corresponding fragments of a variant LINGO-1 polypeptide at least 70%, 75%, 80%, 85%, 90%, or 95% identical to amino acids 66 to 89, 66 to 113, 90 to 113, 114 to 137, 138 to 161, 162 to 185, 186 to 209, 210 to 233, 234 to 257,258 to 281, 282 to 305, 306 to 329, or 330 to 353 of SEQ ID NO:2 are also contemplated.

Additional LINGO-1 peptide fragments to which certain antibodies, or antigen-binding fragments, variants, or derivatives thereof bind include, but are not limited to those fragments comprising, consisting essentially of, or consisting of one or more cysteine rich regions flanking the LRR of LINGO-1. Such fragments, include, for example, a fragment comprising, consisting essentially of, or consisting of amino acids 34 to 64 of SEQ ID NO:2 (the N-terminal LRR flanking region (LRRNT)), or a fragment comprising, consisting essentially of, or consisting of amino acids 363 to 416 of SEQ ID NO:2 (the C-terminal LRR flanking region (LRRCT)). Corresponding fragments of a variant LINGO-1 polypeptide at least 70%, 75%, 80%, 85%, 90%, or 95% identical to amino acids 34 to 64 and 363 to 416 of SEQ ID NO:2 are also contemplated.

In other embodiments, the LINGO-1 antagonists to be used in the methods described herein include an antibody, or antigen-binding fragment, variant, or derivative thereof which specifically or preferentially binds to at least one epitope of LINGO-1, where the epitope comprises, consists essentially of, or consists of at least about four to five amino acids of SEQ ID NO:2, at least seven, at least nine, or between at least about 15 to about 30 amino acids of SEQ ID NO:2. The amino acids of a given epitope of SEQ ID NO:2 as described may be, but need not be, contiguous or linear. In certain embodiments, at least one epitope of LINGO-1 comprises, consists essentially of, or consists of a non-linear epitope formed by the extracellular domain of LINGO-1 as expressed on the surface of a cell or as a soluble fragment, *e.g*., fused to an IgG Fc region. Thus, in certain embodiments the at least one epitope of LINGO-1 comprises, consists essentially of, or consists of at least 4, at least 5, at least 6, at least 7, at least 8, at least 9, at least 10, at least 15, at least 20, at least 25, between about 15 to about 30, or at least 10, 15, 20, 25, 30, 35, 40, 45, 50, 55, 60, 65, 70, 75, 80, 85, 90, 95, or 100 contiguous or non-contiguous amino acids of SEQ ID NO:2, where non-contiguous amino acids form an epitope through protein folding.

Certain TrkB agonist antibodies for use in the methods described herein specifically or preferentially bind to a particular TrkB polypeptide fragment or domain. For example, antibodies for use according to the invention can bind epitope(s) in the IgG-1 Or IgG-2 regions of TrkB and can bind sequences in the loop region of the IgG-1 and/or IgG-2 domains. Antibodies for use according to the invention also include those that increase TrkB autophosphorylation. Certain non-limiting examples of TrkB agonist antibodies also include, but are not limited to, monoclonal antibodies which block binding of ligands such as BDNF to the TrkB receptor, monoclonal antibodies which partially block binding of ligands such as BDNF to the TrkB receptor, monoclonal antibodies that do not block the binding of ligands such as BDNF to the TrkB receptor and monoclonal antibodies that promote or increase the binding of ligands such as BDNF to the TrkB receptor. TrkB agonist antibodies for use according to the invention also include monoclonal antibodies that do not affect the binding of ligands such as BDNF to the TrkB receptor. Certain non-limiting examples of TrkB agonist antibodies include 6E2, 7F5, 11E1, 16E11, 17D11, 19E12, 29D7, which are described in U.S. Published Patent Application No. 2007/0059304 and in Quin et al., Jour. of Neuroscience. 26:9394-9403 (2006), and antibodies or antigen-binding fragments which bind to the same epitopes as those antibodies. Exemplary TrkB agonist antibodies for use in the present invention also include isolated antibodies or antigen binding fragments thereof which specifically bind to the same TrkB epitopes as the TrkB agonist antibodies listed above.

Exemplary antibodies or fragments thereof for use in the present invention include, but are not limited to, isolated antibodies or antigen binding fragments thereof which specifically binds to the same LINGO-1 epitope as a reference monoclonal antibody selected from the group consisting of 201', 3A3, 3A6, 3B5, 1A7, 1D5, 1G7, 2B10, 2C11, 2F3, 3P1B1.1F9, 3P1D10.2C3, 3P1E11.3B7, 3P2C6.3G10.2H7, 3P2C9.2G4, 3P4A6.1D9, 3P4A1.2B9, 3P4C2.2D2, 3P4C5.1D8, 3P4C8.2G9, 6P4F4.1Ds, 6P4F4.1F9, 7P1D5.1G9, 1B6.4, 2C7.2, 2D6.1, 2F7.3, 2H3.2, 3C11.1, 3E3.1, 3H11.2, . 3G8.1, 2B8.1, 3B5.230-C12 (Li01), 38-D01 (Li02), 35-E04 (Li03), 36-C09 (Li04),30-A11 (Li05), 34-F02 (Li06), 29-E07 (Li07), 34-G04 (Li08), 36-A12 (Li09), 28-D02 (Li10), 30-B01 (Li11), 34-B03 (Li12), Li13, Li32, Li33, Li34, 3383 (L1a1), 3495(L1a.2), 3563 (L1a.3), 3564 (L1a.4), 3565 (L1a.5), 3566 (L1a.6), 3567 (L1a. 7),3568 (L1a.8), 3569 (L1a.9), 3570 (L1a.10), 3571 (L1a.11), 3582 (L1a.12), 1968 (L1a.13), 3011, 3012, 3013, 3418, 3422, 3562, D05, D07, D08, D10 and D11, as described in the International Application WO2007/008547 entitled "Sp35 Antibodies and Uses Thereof'' to Mi *et al,* filed July 7,2006.

In other embodiments, the LINGO-1 antagonists and TrkB agonists to be used in the present invention include LINGO-1 or TrkB antibodies, or antigen-binding fragments, variants, or derivatives thereof which specifically or preferentially bind to at least one epitope of LINGO-1 or TrkB, where the epitope comprises, consists essentially of, or consists of, in addition to one, two, three, four, five, six or more contiguous or non-contiguous amino acids of SEQ ID NO:2 or SEQ ID NO:4, respectively, as described above, and an additional moiety which modifies the protein, *e.g.*, a carbohydrate moiety may be included such that the LINGO-1 or TrkB antibody binds with higher affinity to modified target protein than it does to an unmodified version of the protein. Alternatively, the LINGO-1 or TrkB antibody does not bind the unmodified version of the target protein at all.

In certain embodiments, the LINGO-1 antagonist or TrkB agonists to be used in the present invention include an antibody, or antigen-binding fragment, variant, or derivative thereof that binds specifically to at least one epitope of LINGO-1 or TrkB or fragment or variant described above, *i.e.*, binds to such an epitope more readily than it would bind to an unrelated, or random epitope; binds preferentially to at least one epitope of LINGO-1 or TrkB or fragment or variant described above, *i.e.*, binds to such an epitope more readily than it would bind to a related, similar, homologous, or analogous epitope; competitively inhibits binding of a reference antibody which itself binds specifically or preferentially to a certain epitope of LINGO-1 or TrkB or fragment or variant described above; or binds to at least one epitope of LINGO-1 or TrkB or fragment or variant described above with an affinity characterized by a dissociation constant K_{D} of less than about 5 x 10⁻² M, about 10⁻² M, about 5 x 10⁻³ M, about 10⁻³ M, about 5 x 10⁻⁴ M, about 10⁻⁴ M, about 5 x 10⁻⁵ M, about 10⁻⁵ M, about 5 x 10⁻⁶ M, about 10⁻⁶ M, about 5 x 10⁻⁷ M, about 10⁻⁷ M, about 5 x 10⁻⁸ M, about 10⁻⁸ M, about 5 x 10⁻⁹ M, about 10⁻⁹ M, about 5 x 10⁻¹⁰ M, about 10⁻¹⁰ M, about 5 x 10⁻¹¹ M, about 10⁻¹¹ M, about 5 x 10⁻¹² M, about 10⁻¹² M, about 5 x 10⁻¹³ M, about 10⁻¹³ M, about 5 x 10⁻¹⁴ M, about 10⁻¹⁴ M, about 5 x 10⁻¹⁵ M, or about 10⁻¹⁵ M. In a particular aspect, the antibody or fragment thereof preferentially binds to a human LINGO-1 or TrkB polypeptide or fragment thereof, relative to a murine LINGO-1 or TrkB polypeptide or fragment thereof.

As used in the context of antibody binding dissociation constants, the term "about" allows for the degree of variation inherent in the methods utilized for measuring antibody affinity. For example, depending on the level of precision of the instrumentation used, standard error based on the number of samples measured, and rounding error, the term "about 10⁻² M" might include, for example, from 0.05 M to 0.005 M.

In specific embodiments, the LINGO-1 antagonist on TrkB agonists for use in the the present invention include an antibody, or antigen-binding fragment, variant, or derivative thereof that binds LINGO-1 or TrkB polypeptides or fragments or variants thereof with an off rate (k(off)) of less than or equal to 5 X 10⁻² sec⁻¹, 10⁻² sec⁻¹, 5 X 10⁻³ sec⁻¹ or 10⁻³ sec⁻¹. Alternatively, an antibody, or antigen-binding fragment, variant, or derivative thereof of the invention binds LINGO-1 or TrkB polypeptides or fragments or variants thereof with an off rate (k(off)) of less than or equal to 5 X 10⁻⁴ sec⁻¹ 10⁻⁴ sec⁻¹, 5 X 10⁻⁵ sec⁻¹, or 10⁻⁵ sec⁻¹ 5 X 10⁻⁶ sec⁻¹, 10⁻⁶ sec⁻¹, 5 X 10⁻⁷ sec⁻¹ or 10⁻⁷ sec⁻¹.

In other embodiments, the LINGO-1 antagonist or TrkB agonists for use in the present invention include an antibody, or antigen-binding fragment, variant, or derivative thereof that binds LINGO-1 or TrkB polypeptides or fragments or variants thereof with an on rate (k(on)) of greater than or equal to 10³ M⁻¹ sec⁻¹, 5 X 10³ M⁻¹ sec⁻¹, 10⁴ M⁻¹ sec⁻¹ or 5 X 10⁴ M⁻¹ sec⁻¹. Alternatively, an antibody, or antigen-binding fragment, variant, or derivative thereof binds LINGO-1 or TrkB polypeptides or fragments or variants thereof with an on rate (k(on)) greater than or equal to 10⁵ M⁻¹ sec⁻¹, 5 X 10⁵ M⁻¹ sec⁻¹, 10⁶ M⁻¹ sec⁻¹, or 5 X 10⁶ M-¹ sec or 10⁷ M⁻¹ sec⁻¹

In one embodiment, the LINGO-1 antagonist or TrkB agonist antibody for use in the invention is an antibody molecule or an immunospecific fragment thereof. Unless it is specifically noted, as used herein, a "fragment thereof' in reference to an antibody refers to an immunospecific fragment, *i*.*e*., an antigen-specific fragment. In one embodiment, an antibody for use in the invention is a bispecific binding molecule, binding polypeptide, or antibody, *e.g*., a bispecific antibody, minibody, domain deleted antibody, or fusion protein having binding specificity for more than one epitope, *e.g*., more than one antigen or more than one epitope on the same antigen. In one embodiment, a bispecific antibody has at least one binding domain specific for at least one epitope on LINGO-1 or TrkB. A bispecific antibody may be a tetravalent antibody that has two target binding domains specific for an epitope of LINGO-1 or TrkB and two target binding domains specific for a second target. Thus, a tetravalent bispecific antibody may be bivalent for each specificity.

Certain methods of the present disclosure comprise administration of a LINGO-1 antagonist or TrkB agonist antibody, or immunospecific fragment thereof, in which at least a fraction of one or more of the constant region domains has been deleted or otherwise altered so as to provide desired biochemical characteristics such as reduced effector functions, the ability to non-covalently dimerize, increased ability to localize to the desired cite, reduced serum half-life, or increased serum half-life when compared with a whole, unaltered antibody of approximately the same immunogenicity. For example, certain antibodies for use in the methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the C_{H}2 domain will be deleted.

In certain LINGO-1 antagonist or TrkB agonist antibodies or immunospecific fragments thereof for use in the methods described herein, the Fc portion may be mutated to decrease effector function using techniques known in the art. For example, the deletion or inactivation (through point mutations or other means) of a constant region domain may reduce Fc receptor binding of the circulating modified antibody thereby increasing localization at the desired cite of action. In other cases it may be that constant region modifications consistent with the instant invention moderate complement binding and thus reduce the serum half life and nonspecific association of a conjugated cytotoxin. Other desirable modifications to the constant region include mutations designed to alter glycosylation, for example to reduce glycosylation, in order to affect the efficacy, safety, antigen binding, Fc effector fuctions, stability and/or antibody product consistency. Further desirable modifications include modifications that alter antibody solubility, for example by modification of residues that are known or predicted to be involved in multimer formation. Yet other modifications of the constant region may be used to modify disulfide linkages or oligosaccharide moieties that allow for enhanced localization due to increased antigen specificity or antibody flexibility. The resulting physiological profile, bioavailability and other biochemical effects of the modifications, such as localization, biodistribution and serum half-life, may easily be measured and quantified using well-known immunological techniques without undue experimentation.

Modified forms of antibodies or immunospecific fragments thereof for use in the methods disclosed herein can be made from whole precursor or parent antibodies using techniques known in the art. Exemplary techniques are discussed in more detail herein.

In certain embodiments both the variable and constant regions of LINGO-1 antagonist or TrkB agonist antibodies or immunospecific fragments thereof for use in the methods disclosed herein are fully human. Fully human antibodies can be made using -techniques that are known in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in US patents: 6,150,584; 6,458,592; 6,420,140. Other techniques are known in the art. Fully human antibodies can likewise be produced by various display technologies, *e.g*., phage display or other viral display systems, as described in more detail elsewhere herein.

LINGO-1 antagonist or TrkB agonist antibodies or immunospecific fragments thereof for use in the methods disclosed herein can be made or manufactured using techniques that are known in the art. In certain embodiments, antibody molecules or fragments thereof are "recombinantly produced," *i.e.*, are produced using recombinant DNA technology. Exemplary techniques for making antibody molecules or fragments thereof are discussed in more detail elsewhere herein.

LINGO-1 antagonist and TrkB agonist antibodies or fragments thereof for use in the present invention may be generated by any suitable method known in the art.

Polyclonal antibodies can be produced by various procedures well known in the art. For example, a LINGO-1 or TrkB immunospecific fragment can be administered to various host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum.* Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, Cold Spring Harbor Laboratory Press, 2nd ed. (1988); Hammerling et al., in: Monoclonal Antibodies and T-Cell Hybridomas Elsevier, N.Y., 563-681 (1981).

The term "monoclonal antibody as used herein is not limited to antibodies, produced through hybridoma technology. The term "monoclonal antibody" refers to an antibody that is derived from a single clone, including any eukaryotic, prokaryotic, or phage clone, and not the method by which it is produced. Thus, the term "monoclonal antibody" is not limited to antibodies produced through hybridoma technology. Monoclonal antibodies can be prepared using a wide variety of techniques known in the art including the use of hybridoma and recombinant and phage display technology.

Using art recognized protocols, in one example, antibodies are raised in mammals by multiple subcutaneous or intraperitoneal injections of the relevant antigen (*e.g*., purified LINGO-1 or TrkB antigens or cells or cellular extracts comprising such antigens) and an adjuvant. This immunization typically elicits an immune response that comprises production of antigen-reactive antibodies from activated splenocytes or lymphocytes. While the resulting antibodies may be harvested from the serum of the animal to provide polyclonal preparations, it is often desirable to isolate individual lymphocytes from the spleen, lymph nodes or peripheral blood to provide homogenous preparations of monoclonal antibodies (mAbs). The lymphocytes can be obtained, for example, from the spleen.

In this well known process (Kohler et al., Nature 256:495 (1975)) the relatively short-lived, or mortal, lymphocytes from a mammal which has been injected with antigen are fused with an immortal tumor cell line (*e.g*. a myeloma cell line), thus producing hybrid cells or "hybridomas" which are both immortal and capable of producing the genetically coded antibody of the B cell. The resulting hybrids are segregated into single genetic strains by selection, dilution, and regrowth with each individual strain comprising specific genes for the formation of a single antibody. They produce antibodies which are homogeneous against a desired antigen and, in reference to their pure genetic parentage, are termed "monoclonal."

Typically, hybridoma cells thus prepared are seeded and grown in a suitable culture medium that can contains one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. The binding specificity of the monoclonal antibodies produced by hybridoma cells can be determined by *in vitro* assays such as immunoprecipitation, radioimmunoassay (RIA) or enzyme-linked immunoabsorbent assay (ELISA). After hybridoma cells are identified that produce antibodies of the desired specificity, affinity and/or activity, the clones may be subcloned by limiting dilution procedures and grown by standard methods (Goding, Monoclonal Antibodies: Principles and Practice, Academic Press, pp 59-103 (1986)). It will further be appreciated that the monoclonal antibodies secreted by the subclones may. be separated from culture medium, ascites fluid or serum by conventional purification procedures such as, for example, protein-A, hydroxylapatite chromatography, gel electrophoresis, dialysis or affinity chromatography.

Antibody fragments that recognize specific epitopes may be generated by known techniques. For example, Fab and F(ab')₂ fragments may be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the C_{H}1 domain of the heavy chain.

Those skilled in the art will also appreciate that DNA encoding antibodies or antibody fragments (*e.g.*, antigen binding sites) may also be derived from antibody phage libraries. In a particular, such phage can be utilized to display antigen-binding domains expressed from a repertoire or combinatorial antibody library (*e.g*., human or murine). Phage expressing an antigen binding domain that binds the antigen of interest can be selected or identified with antigen, *e.g*., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Exemplary methods are set forth, for example, in EP 368 684 B1; U.S. patent. 5,969,108, Hoogenboom, H.R. and Chames, Immunol. Today 21:371 (2000); Nagy et al. Nat. Med. 8:801 (2002); Huie et al., Proc. Natl. Acad. Sci. USA 98:2682 (2001); Lui et al., J. Mol. Biol. 315:1063 (2002).

Several publications (*e.g*., Marks et al., Bio/Technology 10:779-783 (1992)) have described the production of high affinity human antibodies by chain shuffling, as well as combinatorial infection and *in vivo* recombination as a strategy for constructing large phage libraries. In another embodiment, ribosomal display can be used to replace bacteriophage as the display platform (*see, e.g.,* Hanes et al., Nat. Biotechnol. 18:1287 (2000); Wilson et al., Proc. Natl. Acad. Sci. USA 98:3750 (2001); or Irving et al., J. Immunol. Methods 248:31 (2001)). In yet another embodiment, cell surface libraries can be screened for antibodies (Boder et al., Proc. Natl. Acad. Sci. USA 97:10701 (2000); Daugherty et al., J. Immunol. Methods 243:211 (2000)). Such procedures provide alternatives to traditional hybridoma techniques for the isolation and subsequent cloning of monoclonal antibodies.

In phage display methods, functional antibody domains are displayed on the surface of phage particles which carry the polynucleotide sequences encoding them. In particular, DNA sequences encoding V_{H} and V_{L} regions are amplified from animal cDNA libraries (*e.g*., human or murine cDNA libraries of lymphoid tissues) or synthetic cDNA libraries. In certain embodiments, the DNA encoding the V_{H} and V_{L} regions are joined together by an scFv linker by PCR and cloned into a phagemid vector (*e.g*., p CANTAB 6 or pComb 3 HSS). The vector is electroporated in *E*. *coli* and the *E. coli* is infected with helper phage. Phage used in these methods are typically filamentous phage including fd and M13 and the V_{H} or V_{L} regions are usually recombinantly fused to either the phage gene III or gene VIII. Phage expressing an antigen binding domain that binds to an antigen of interest (*i.e.*, a LINGO-1 or TrkB polypeptide or a fragment thereof) can be selected or identified with antigen, *e.g*., using labeled antigen or antigen bound or captured to a solid surface or bead.

Additional examples of phage display methods that can be used to make the antibodies include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187:9-18 (1997); Burton et al., Advances in Immunology 57:191-280 (1994); PCT Application No. PCT/GB91/01134; PCT publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

In another embodiment, DNA encoding desired monoclonal antibodies for use in the present invention may be readily isolated and sequenced using conventional procedures (*e.g*., by using oligonucleotide probes that are capable of binding specifically to genes encoding the heavy and light chains of murine antibodies). The isolated and subcloned hybridoma cells serve as a possible source of such DNA. Once isolated, the DNA may be placed into expression vectors, which are then transfected into prokaryotic or eukaryotic host cells such as *E. coli* cells, simian COS cells, Chinese Hamster Ovary (CHO) cells or myeloma cells that do not otherwise produce immunoglobulins. More particularly, the isolated DNA (which may be synthetic as described herein) may be used to clone constant and variable region sequences for the manufacture antibodies as described in Newman et al., U.S. Pat. No. 5,658,570, filed January 25, 1995. Essentially, this entails extraction of RNA from the selected cells, conversion to cDNA, and. amplification by PCR using Ig specific primers. Suitable primers for this purpose are also described in U.S. Pat. No. 5,658,570. As will be discussed in more detail below, transformed cells expressing the desired antibody may be grown up in relatively large quantities to provide clinical and commercial supplies of the immunoglobulin.

In a specific embodiment, the amino acid sequence of the heavy and/or light chain variable domains may be inspected to identify the sequences of the complementarity determining regions (CDRs) by methods that are well known in the art, *e.g*., by comparison to known amino acid sequences of other heavy and light chain variable regions to determine the regions of sequence hypervariability. Using routine recombinant DNA techniques, one or more of the CDRs may be inserted within framework regions, *e.g*., into human framework regions to humanize a non-human antibody. The framework regions may be naturally occurring or consensus framework regions, and may be human framework regions (see, *e.g.*, Chothia et al., J. Mol. Biol. 278:457-479 (1998) for a listing of human framework regions). The polynucleotide generated by the combination of the framework regions and CDRs may encode an antibody that specifically binds to at least one epitope of a desired polypeptide, *e.g*., LINGO-1. One or more amino acid substitutions may be made within the framework regions, and the amino acid substitutions may improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

In certain embodiments, a LINGO-1 antagonist or TrkB agonist antibody or immunospecific fragment thereof for use in the methods disclosed herein will not elicit a deleterious immune response in the animal to be treated, *e.g*., in a human. In one embodiment, LINGO-1 antagonist or TrkB agonist antibodies or immunospecific fragments thereof for use in the methods disclosed herein can be modified to reduce their immunogenicity using art-recognized techniques. For example, antibodies can be humanized, primatized, deimmunized, or chimeric antibodies can be made. These types of antibodies are derived from a non-human antibody, typically a murine or primate antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting at least a part of one or more of the non-human complementarity determining regions (CDRs) into a human framework and constant regions with or without retention of critical framework residues; or (c) transplanting the entire non-human variable domains, but "cloaking" them with a human-like section by replacement of surface residues. Such methods are disclosed in Morrison et al., Proc. Natl. Acad. Sci. 81:6851-6855 (1984); Morrison et al., Adv. Immunol. 44:65-92 (1988); Verhoeyen et al., Science 239:1534-1536 (1988); Padlan, Molec. Immun. 28:489-498 (1991); Padlan, Molec. Immun. 31:169-217 (1994), and U.S. Pat. Nos. 5,585,089, 5,693,761, 5,693,762, and 6,190,370.

De-immunization can also be used to decrease the immunogenicity of an antibody. As used herein, the term "de-immunization" includes alteration of an antibody to modify T cell epitopes (see, *e.g*., WO9852976A1, WO0034317A2). For example, V_{H} and V_{L} sequences from the starting antibody are analyzed and a human T cell epitope "map" from each V region showing the location of epitopes in relation to complementarity-determining regions (CDRs) and other key residues within the sequence. Individual T cell epitopes from the T cell epitope map are analyzed in order to identify alternative amino acid substitutions with a low risk of altering activity of the final antibody. A range of alternative V_{H} and V_{L} sequences are designed comprising combinations of amino acid substitutions and these sequences are subsequently incorporated into a range of binding polypeptides, *e.g*., LINGO-1 antagonist or TrkB agonist antibodies or immunospecific fragments thereof for use in the methods disclosed herein, which are then tested for function. Typically, between 12 and 24 variant antibodies are generated and tested. Complete heavy and light chain genes comprising modified V and human C regions are then cloned into expression vectors and the subsequent plasmids introduced into cell lines for the production of whole antibody. The antibodies are then compared in appropriate biochemical and biological assays, and the optimal variant is identified.

A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. *See, e.g*., Morrison, Science 229:1202 (1985); Oi et al., BioTechniques 4:214 (1986); Gillies et al., J. Immunol. Methods 125:191-202 (1989); U.S. Pat. Nos. 5,807,715; 4,816,567; and 4,816397. Humanized antibodies are antibody molecules from non-human species antibody that binds the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, and potentially improve, antigen binding. These framework substitutions are identified by methods well known in the art, *e.g*., by modeling of the interactions of the CDR and framework residues to identify framework residues important for antigen binding and sequence comparison to identify unusual framework residues at particular positions. (See, *e.g.*, Queen *et al*., U.S. Pat. No. 5,585,089; Riechmann et al., Nature 332:323 (1988)).

Antibodies can be humanized using a variety of techniques known in the art including, for example, GDR-grafting (EP 239,400; PCT publication WO 91/09967; U.S. Pat. Nos. 5,225,539; 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Pat. No. 5,565,332).

Yet another highly efficient means for generating recombinant antibodies is disclosed by Newman, Biotechnology 10: 1455-1460 (1992). Specifically, this technique results in the generation of primatized antibodies that contain monkey variable domains and human constant sequences.

Moreover, this technique is also described in commonly assigned U.S. Pat. Nos. 5,658,570, 5,693,780 and 5,756,096.

Completely human antibodies are particularly desirable for therapeutic treatment of human patients. Human antibodies can be made by a variety of methods known in the art including phage display methods described above using antibody libraries derived from human immunoglobulin sequences. See also, U.S. Pat. Nos. 4,444,887 and 4,716,111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, *e.g*., all or a portion of a desired target polypeptide. Monoclonal antibodies directed against the antigen can be obtained from the immunized, transgenic mice using conventional hybridoma technology. The human immunoglobulin transgenes harbored by the transgenic mice rearrange during B-cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar Int. Rev. Immunol. 13:65-93 (1995). For a detailed discussion of this technology for producing human antibodies and human monoclonal antibodies and protocols for producing such antibodies, see, *e.g*., PCT publications WO 98/24893; WO 96/34096; WO 96/33735; U.S. Pat. Nos. 5,413,923; 5,625,126; 5,633,425; 5,569,825; 5,661,016; 5,545,806; 5,814,318; and 5,939,598.

In addition, companies such as Abgenix, Inc. (Freemont, Calif.) and GenPharm (San Jose, Calif.) can be engaged to provide human antibodies directed against a selected antigen using technology similar to that described above.

Another means of generating human antibodies using SCID mice is disclosed in U.S. Pat. No. 5,811,524. It will be appreciated that the genetic material associated with these human antibodies may also be isolated and manipulated as described herein.

Completely human antibodies which recognize a selected epitope can be generated using a technique referred to as "guided selection." In this approach a selected non-human monoclonal antibody, *e.g*., a mouse antibody, is used to guide the selection of a completely human antibody recognizing the same epitope. (Jespers et al., Bio/Technology 12:899-903 (1988)). *See also*, U.S. Patent No. 5,565,332.

Alternatively, techniques described for the production of single chain antibodies (U.S. Pat. No. 4,694,778; Bird, Science 242:423-442 (1988); Huston et al., Proc. Natl. Acad. Sci. USA 85:5879-5883 (1988); and Ward et al., Nature 334:544-554 (1989)) can be adapted to produce single chain antibodies. Single chain antibodies are formed by linking the heavy and light chain fragments of the Fv region via an amino acid bridge, resulting in a single chain antibody. Techniques for the assembly of functional Fv fragments in E coli may also be used (Skecra et al., Science 242:1038-1041 (1988)). Examples of techniques which can be used to produce single-chain Fvs and antibodies include those described in U.S. Pat. Nos. 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, especially including *in vivo* use of antibodies in humans and *in vitro* detection assays, chimeric, humanized, or human antibodies can be used.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As used herein, a chimeric antibody is a molecule in which different portions are derived from different animal species, such as those having a variable region derived from a murine monoclonal antibody and a human immunoglobulin constant region, *e.g*., humanized antibodies.

LINGO-1 antagonist or TrkB agonist antibodies may also be human or substantially human antibodies generated in transgenic animals (*e.g*., mice) that are incapable of endogenous immunoglobulin production (see *e.g.,* U.S. Pat. Nos. 6,075,181, 5,939,598, 5,591,669 and 5,589,369. For example, it has been described that the homozygous deletion of the antibody heavy-chain joining region in chimeric and germ-line mutant mice results in complete inhibition of endogenous antibody production. Transfer of a human immunoglobulin gene array to such germ line mutant mice will result in the production of human antibodies upon antigen challenge.

In another embodiment, lymphocytes can be selected by micromanipulation and the variable genes isolated. For example, peripheral blood mononuclear cells can be isolated from an immunized mammal and cultured for about 7 days *in vitro.* The cultures can be screened for specific IgGs that meet the screening criteria. Cells from positive wells can be isolated. Individual Ig-producing B cells can be isolated by FACS or by identifying them in a complement-mediated hemolytic plaque assay. Ig-producing B cells can be micromanipulated into a tube and the V_{H} and V_{L} genes can be amplified using, *e.g*., RT-PCR. The V_{H} and V_{L} genes can be cloned into an antibody expression vector and transfected into cells (*e.g.*, eukaryotic or prokaryotic cells) for expression.

Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991).

Antibodies for use in the methods disclosed herein can be produced by any method known in the art for the synthesis of antibodies, in particular, by chemical synthesis or by recombinant expression techniques as described herein.

It will further be appreciated that the scope of this invention further encompasses all alleles, variants and mutations of antigen binding DNA sequences.

As is well known, RNA may be isolated from the original hybridoma cells or from other transformed cells by standard techniques, such as guanidinium isothiocyanate extraction and precipitation followed by centrifugation or chromatography. Where desirable, mRNA may be isolated from total RNA by standard techniques such as chromatography on oligo dT cellulose. Suitable techniques are familiar in the art.

In one embodiment, cDNAs that encode the light and the heavy chains of the antibody for use in the present invention may be made, either simultaneously or separately, using reverse transcriptase and DNA polymerase in accordance with well known methods. PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as mouse constant region probes.

DNA, typically plasmid DNA, may be isolated from the cells using techniques known in the art, restriction mapped and sequenced in accordance with standard, well known techniques set forth in detail, *e.g*., in the foregoing references relating to recombinant DNA techniques. Of course, the DNA may be synthetic according to the present invention at any point during the isolation process or subsequent analysis.

Recombinant expression of an antibody, or fragment, derivative or analog thereof, *e.g.*, a heavy or light chain of an antibody which is a LINGO-1 antagonist or TrkB agonist, requires construction of an expression vector containing a polynucleotide that encodes the antibody. Once a polynucleotide encoding an antibody molecule or a heavy or light chain of an antibody, or portion thereof (which may contain the heavy or light chain variable domain), for use according to the invention has been obtained, the vector for the production of of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule for use according to the invention, or a heavy or light chain thereof, or a heavy or light chain variable domain, operably linked to a promoter. Such vectors may include the nucleotide sequence encoding the constant region of the antibody molecule (see, *e.g*., PCT Publication WO 86/05807; PCT Publication WO 89/01036; and U.S. Pat. No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody for use in the methods described herein. Thus, the disclosure includes host cells containing a polynucleotide encoding an antibody for use according to the invention, or a heavy or light chain thereof, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express antibody molecules for use in the methods described elsewhere herein.

The host cell may be co-transfected with two expression vectors the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Once an antibody molecule for use according to the invention has been recombinantly (expressed may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Alternatively, another method for increasing the affinity of antibodies for use according to the invention is disclosed in US 2002 0123057 A1.

In one embodiment, a binding molecule or antigen binding molecule for use in the invention comprises a synthetic constant region wherein one or more domains are partially or entirely deleted ("domain-deleted antibodies"). In certain embodiments compatible modified antibodies will comprise domain deleted constructs or variants wherein the entire C_{H}2 domain has been removed (ΔC_{H}2 constructs). For other embodiments a short connecting peptide may be substituted for the deleted domain to provide flexibility and freedom of movement for the variable region. Those skilled in the art will appreciate that such constructs are particularly useful due to the regulatory properties of the C_{H}2 domain on the catabolic rate of the antibody.

In certain embodiments, modified antibodies for use in the methods disclosed herein are minibodies. Minibodies can be made using methods described in the art (*see*, *e.g*., US patent 5,837,821 or WO 94/09817A1).

In another embodiment, modified antibodies for use in the methods disclosed herein are C_{H}2 domain deleted antibodies which are known in the art. Domain deleted constructs can be derived using a vector (*e.g.*, from Biogen IDEC Incorporated) encoding an IgG₁ human constant domain (see, *e.g.*, WO 02/060955A2 and WO02/096948A2. This exemplary vector was engineered to delete the C_{H}2 domain and provide a synthetic vector expressing a domain deleted IgG₁ constant region.

In one embodiment, a LINGO-1 antagonist or TrkB agonist antibody or fragment thereof for use in the methods disclosed herein comprises an immunoglobulin heavy chain having deletion or substitution of a few or even a single amino acid as long as it permits association between the monomeric subunits. Similarly, it may be desirable to simply delete that part of one or more constant region domains that controls the effector function (*e.g*. complement binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be synthetic through the mutation or substitution of one or more amino acids that enhances the profile of the resulting construct. In this respect it may be possible to disrupt the activity provided by a conserved binding site (*e.g*. Fc binding) while substantially maintaining the configuration and immunogenic profile of the modified antibody. Yet other embodiments comprise the addition of one or more amino acids to the constant region to enhance desirable characteristics such as effector function or provide for more cytotoxin or carbohydrate attachment. In such embodiments it may be desirable to insert or replicate specific sequences derived from selected constant region domains.

The present invention also provides the use of antibodies that comprise, consist essentially of, or consist of, variants (including derivatives) of antibody molecules (*e.g*., the V_{H} regions and/or V_{L} regions) described herein, which antibodies or fragments thereof immunospecifically bind to a LINGO-1 or TrkB polypeptide. Standard techniques known to those of skill in the art can be used to introduce mutations in the nucleotide sequence encoding a binding molecule, including, but not limited to, site-directed mutagenesis and PCR-mediated mutagenesis which result in amino acid substitutions. The variants (including derivatives) can encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference V_{H} region, V_{H}CDR1, V_{H}CDR2, V_{H}CDR3, V_{L} region, V_{L}CDR1, V_{L}CDR2, or V_{L}CDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains (*e.g*., lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity.

For example, it is possible to introduce mutations only in framework regions or only in CDR regions of an antibody molecule. Introduced mutations may be silent or neutral missense mutations, *i.e*., have no, or little, effect on an antibody's ability to bind antigen. These types of mutations may be useful to optimize codon usage, or improve a hybridoma's antibody production. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen binding activity or alteration in binding activity (*e.g*., improvements in antigen binding activity or change in antibody specificity). Following mutagenesis, the encoded protein may routinely be expressed and the functional and/or biological activity of the encoded protein can be determined using techniques described herein or by routinely modifying techniques known in the art.

Additional Antagonists and Agonists and Combinations Thereof for Use in the Methods of the Invention

In addition to the agonists and antagonists described previously, additional TrkB agonists : include any polypeptide, antibody, compound or nucleotide which would promote, increase or enhance the activity of TrkB. Such agonist include polypeptides, antibodies, compounds or nucleotides which interfere with or promote the binding of a TrkB ligand such as Brain-Derived Neurotrophic Factor (BDNF), to the TrkB receptor and as such are also considered BDNF agonists. Such molecules include but are not limited to antibodies which disrupt the interact between a ligand and TrkB, peptidomimetic agonists of TrkB and ligand analogs such as those described in 5,770,577, 6,077,829, 6,723,701, and 6,800,607. -

The antagonists and agonists described herein for use in the present invention may be administered as compositions in various combinations. For example, various TrkB agonists may be used in combination with LINGO-1 antagonists. Compositions for use in the present invention may also include multiple TrkB agonists and/or LINGO-1 antagonists.

Additionally, compositions for use in the present invention may include other antagonists or agonists of proteins expressed in the CNS such as Nogo Receptor 1 (NgR1), LINGO-1 (LINGO-1), TAJ or Oligodendrocyte-myelin glycoprotein (OMgp). Antagonists of NgR1 are described in U.S. Publication Nos. 2002/0077295 and 2005/0271655 A1 and International Application Publication Nos. WO 01/51520, WO 03/031462, WO 2004/014311 and WO 2005/016955.

Antagonists of LINGO-1 (LINGO-1) may be found in U.S. Publication No. 2006/0009388 A1 and International Publication No. WO 2004/085648.

Examples of TAJ antagonists are described in U.S. Publication No. 2006/0058223 A1.

OMgp antagonists are described in U.S. Provisional Application Nos. 60/730,357 and 60/735,170.

Compositions for use in the methods of the present invention may also include any number and combination of TrkB, LINGO-1, NgR1, TAJ and OMgp antagonists.

### Aptamers

In another embodiment, the LINGO-1 antagonist or TrkB agonist for use in the methods of the present invention is an aptamer. An aptamer can be a nucleotide or a polypeptide which has a unique sequence, has the property of binding specifically to a desired target (*e.g*. a polypeptide), and is a specific ligand of a given target. Nucleotide aptamers for use according to the invention include double stranded DNA and single stranded RNA molecules that bind to LINGO-1 or TrkB.

Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in *e.g*. U.S. Pat. Nos. 5,475,096, 5,580,737, 5,567,588, 5,707,796, 5,763,177, 6, 011,577, and 6,699,843.

Another screening method to identify aptamers is described in U.S. Pat. No. 5,270,163. The SELEX process is based on the capacity of nucleic acids for forming a variety of two- and three-dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets.

The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, which can comprise a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield highly specific high affinity nucleic acid ligands to the target molecule.

Nucleotide aptamers may be used, for example, as diagnostic tools or as specific inhibitors to dissect intracellular signaling and transport pathways. *See* James Curr. Opin. Pharmacol. 1:540-546 (2001). The high affinity and specificity of nucleotide aptamers makes them good candidates for drug discovery. For example, aptamer antagonists to the toxin ricin have been isolated and have IC50 values in the nanomolar range. *See* Hesselberth JR et al. J Biol Chem 275:4937-4942 (2000). Nucleotide aptamers may also be used against infectious disease, malignancy and viral surface proteins to reduce cellular infectivity.

Nucleotide aptamers for use in the present invention may be modified (*e.g*., by modifying the backbone or bases or conjugated to peptides) as described herein for other polynucleotides.

Using the protein structure of LINGO-1 or TrkB, screening for aptamers that act on LINGO-1 or TrkB using the SELEX process would allow for the identification of aptamers that inhibit LINGO-1-mediated or promote TrkB-mediated processes (*e.g*. LINGO-1 or TrkB-mediated promotion of cell survival).

Polypeptide aptamers for use in the present invention are random peptides selected for their ability to bind to and block the action of LINGO-1. Polypeptide aptamers may include a short variable peptide domain attached at both ends to a protein scaffold. This double structural constraint greatly increases the binding affinity of the peptide aptamer to levels comparable to an antibody's (nanomolar range). *See e.g.*, Hoppe-Seyler F et al. J. Mol. Med. 78(8):426-430 (2000). The length of the short variable peptide is typically about 10 to 20 amino acids, and the scaffold may be any protein which has good solubility and compacity properties. One non-limiting example of a scaffold protein is the bacterial protein Thioredoxin-A. *See, e.g*., Cohen BA et al. PNAS 95(24): 14272-14277 (1998). An additional, non-limiting example, of a polypeptide aptamer for use in the present invention is a Ligand Regulated Peptide Aptamer (LiRPA). The LiRPA scaffold may be composed of three protein domains: FK506 binding protein (FKBP), FRBP-Rapamycin binding domain (FRB) and glutathione-S-transferase (GST). *See, e.g.*, Binkowski BF et al., Chem & Biol 12(7): 847-855 (2005).

Polypeptide aptamers are peptides or small polypeptides that act as dominant inhibitors of protein function. Peptide aptamers specifically bind to target proteins, blocking their functional ability. Kolonin et al. Proc. Natl. Acad. Sci. 95: 14,266-14,271 (1998). Peptide aptamers that bind with high affinity and specificity to a target protein can be isolated by a variety of techniques known in the art: Peptide aptamers can be isolated from random peptide libraries by yeast two-hybrid screens (Xu, C.W., et al. Proc. Natl. Acad. Sci. 94:12,473-12,478 (1997)) or by ribosome display (Hanes et al. Proc. Natl. Acad. Sci. 94:4937-4942 (1997)). They can also be isolated from phage libraries (Hoogenboom, H.R., et al. Immunotechnology 4:1-20 (1998)) or chemically generated peptide libraries. Although the difficult means by which peptide aptamers are synthesized makes their use more complex than polynucleotide aptamers, they have unlimited chemical diversity.

Peptide aptamers for use in the present invention may be modified (*e.g.*, conjugated to polymers or fused to proteins) as described for other polypeptides elsewhere herein.

### Fusion Proteins and Conjugated Polypeptides, Aptamers, Compounds and Antibodies

LINGO-1 antagonist or TrkB agonist polypeptides, aptamers, compounds and antibodies for use in the methods disclosed herein may further be recombinantly fused to a heterologous polypeptide at the N- or C-terminus or chemically conjugated (including covalent and non-covalent conjugations) to polypeptides or other compositions. For example, LINGO-1 antagonist or TrkB agonist polypeptides, aptamers, compounds and antibodies may be recombinantly fused or conjugated to molecules useful as labels in detection assays and effector molecules such as heterologous polypeptides, drugs, radionuclides, or toxins. *See, e.g*., PCT publications WO 92/08495; WO 91/14438; WO 89/12624; U.S. Patent No. 5,314,995; and EP 396,387.

LINGO-1 antagonist or TrkB agonist polypeptides, aptamers, compounds and antibodies for use in the methods disclosed herein include derivatives that are modified, *i.e.,* by the covalent attachment of any type of molecule such that covalent attachment does not prevent the LINGO-1 antagonist or TrkB agonist polypeptide, aptamer, compound or antibody from inhibiting the biological function of LINGO-1 or TrkB. For example, but not by way of limitation, the LINGO-1 antagonist or TrkB agonist polypeptides, aptamers, compounds and antibodies for use according to the present invention may be modified *e.g*., by glycosylation, acetylation, pegylation, phosphylation, phosphorylation, amidation, derivatization by known protecting/blocking groups, proteolytic cleavage, association with a cellular ligand or other protein, etc. Any of numerous chemical modifications may be carried out by known techniques, including, but not limited to specific-chemical cleavage, acetylation, formulation, metabolic synthesis of tunicamycin, etc. Additionally, the derivative may contain one or more non-classical amino acids.

LINGO-1 antagonist or TrkB agonist polypeptides, aptamers and antibodies for use in the methods disclosed herein can be composed of amino acids joined to each other by peptide bonds or modified peptide bonds, *i.e*., peptide isosteres, and may contain amino acids other than the 20 gene-encoded amino acids. LINGO-1 antagonist or TrkB agonist polypeptides, aptamers and antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the LINGO-1 antagonist or TrkB agonist polypeptide or antibody, including the peptide backbone, the amino acid side-chains and the amino or carboxyl termini, or on moieties such as carbohydrates. It will be appreciated that the same type of modification may be present in the same or varying degrees at several sites in a given LINGO-1 antagonist or TrkB agonist polypeptide, aptamer or antibody. Also, a given LINGO-1 antagonist or TrkB agonist polypeptide, aptamer or antibody may contain many types of modifications. LINGO-1 antagonist or TrkB agonist polypeptides, aptamers or antibodies may be branched, for example, as a result of ubiquitination, and they may be cyclic, with or without branching. Cyclic, branched, and branched cyclic LINGO-1 antagonist or TrkB agonist polypeptides, aptamers and antibodies may result from posttranslational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (*See*, for instance, Proteins - Structure And Molecular Properties, T. E. Creighton, W. H. Freeman and Company, New York 2nd Ed., (1993); Posttranslational Covalent Modification Of Proteins, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992)).

The present invention also provides for fusion proteins comprising, consisting essentially of, or consisting of a LINGO-1 antagonist or TrkB agonist polypeptide, aptamer or antibody fusion that inhibits or decreases LINGO-1 or increases or promotes TrkB function. In some embodiments, the heterologous polypeptide to which the LINGO-1 antagonist or TrkB agonist polypeptide, aptamer or antibody is fused is useful for function or is useful to target the LINGO-1 antagonist or TrkB agonist polypeptide or antibody. In certain embodiments of the invention a soluble LINGO-1 antagonist or TrkB agonist polypeptide, *e.g*., a LINGO-1 polypeptide comprising the LRR domains, Ig domain, or the entire extracellular domain (corresponding to amino acids 34 to 532 of SEQ ID NO: 2), is fused to a heterologous polypeptide moiety to form a LINGO-1 antagonist fusion polypeptide or a TrkB-agonist polypeptide, such as a BDNF polypeptide is fused to a heterologous polypeptide moiety to form a TrkB agonist fusion polypeptide. LINGO-1 antagonist or TrkB agonist fusion proteins, aptamers and antibodies can be used to accomplish various objectives, *e.g*., increased serum half-life, improved bioavailability, *in vivo* targeting to a specific organ or tissue type, improved recombinant expression efficiency, improved host cell secretion, ease of purification, and higher avidity. Depending on the objective(s) to be achieved, the heterologous moiety can be inert or biologically active. Also, it can be chosen to be stably fused to the LINGO-1 antagonist or TrkB agonist polypeptide, aptamer or antibody or to be cleavable, *in vitro* or *in vivo.* Heterologous moieties to accomplish these other objectives are known in the art.

As an alternative to expression of a LINGO-1 antagonist or TrkB agonist fusion polypeptide, aptamer or antibody, a chosen heterologous moiety can be preformed and chemically conjugated to the LINGO-1 antagonist or TrkB agonist polypeptide, aptamer or antibody. In most cases, a chosen heterologous moiety will function similarly, whether fused or conjugated to the LINGO-1 antagonist or TrkB agonist polypeptide, aptamer or antibody. Therefore, in the following discussion of heterologous amino acid sequences, unless otherwise noted, it is to be understood that the heterologous sequence can be joined to the LINGO-1 antagonist or TrkB agonist polypeptide, aptamer or antibody in the form of a fusion protein or as a chemical conjugate.

Pharmacologically active polypeptides such as LINGO-1 antagonist or TrkB agonist polypeptides, aptamers or antibodies often exhibit rapid *in vivo* clearance, necessitating large doses to achieve therapeutically effective concentration in the body. In addition, polypeptides smaller than about 60 kDa potentially undergo glomerular filtration, which sometimes leads to nephrotoxicity. Fusion or conjugation of relatively small polypeptides such as LINGO-1 antagonist or TrkB agonist polypeptides, aptamers or antibodies can be employed to reduce or avoid the risk of such nephrotoxicity. Various heterologous amino acid sequences, *i.e*., polypeptide moieties or "carriers," for increasing the *in vivo* stability, *i.e*., serum half-life, of therapeutic polypeptides are known.

Due to its long half-life, wide *in vivo* distribution, and lack of enzymatic or immunological function, essentially full-length human serum albumin (HSA), or an HSA fragment, is commonly used as a heterologous moiety. Through application of methods and materials such as those taught in Yeh et al., Proc. Natl. Acad. Sci. USA 89:1904-08 (1992) and Syed et al., Blood 89:3243-52 (1997), HSA can be used to form a LINGO-1 antagonist or TrkB agonist fusion polypeptide, aptamer, antibody or polypeptide/antibody conjugate that displays pharmacological activity by virtue of the LINGO-1 or TrkB-agonist moiety while displaying significantly increased *in vivo* stability, *e.g*., 10-fold to 100-fold higher. The C-terminus of the HSA can be fused to the N-terminus of the soluble LINGO-1 or TrkB-agonist moiety. Since HSA is a naturally secreted protein, the HSA signal sequence can be exploited to obtain secretion of the soluble LINGO-1 or TrkB-agonist fusion protein into the cell culture medium when the fusion protein is produced in a eukaryotic, *e.g*., mammalian, expression system.

In certain embodiments, LINGO-1 antagonist or TrkB agonist polypeptides, aptamers, compounds, antibodies and antibody fragments thereof for use in the present invention further comprise a targeting moiety. Targeting moieties include a protein or a peptide which directs localization to a certain part of the body, for example, to the brain or compartments therein. In certain embodiments, LINGO-1 antagonist or TrkB agonist polypeptides, aptamers, compounds, antibodies or antibody fragments thereof for use in the present invention are attached or fused to a brain targeting moiety. The brain targeting moieties are attached covalently (*e.g*., direct, translational fusion, or by chemical linkage either directly or through a spacer molecule, which can be optionally cleavable) or non-covalently attached (*e.g*., through reversible interactions such as avidin, biotin, protein A, IgG, etc.). In other embodiments, the LINGO-1 antagonist or TrkB agonist polypeptides, aptamers, compounds, antibodies or antibody fragments thereof for use in the present invention are attached to one more brain targeting moieties. In additional embodiments, the brain targeting moiety is attached to a plurality of LINGO-1 antagonist or TrkB agonist polypeptides, aptamers, compounds, antibodies or antibody fragments thereof for use in the present invention.

A brain targeting moiety associated with a LINGO-1 antagonist or TrkB agonist polypeptide, aptamer, compound, antibody or antibody fragment thereof enhances brain delivery of such a LINGO-1 antagonist or TrkB agonist polypeptide, aptamer, compound, antibody or antibody fragment thereof. A number of polypeptides have been described which, when fused to a protein or therapeutic agent, delivers the protein or therapeutic agent through the blood brain barrier (BBB). Non-limiting examples include the single domain antibody FC5 (Abulrob et al. J. Neurochem. 95, 1201-1214 (2005)); mAB 83-14, a monoclonal antibody to the human insulin receptor (Pardridge et al. Pharmacol. Res. 12, 807-816 (1995)); the B2, B6 and B8 peptides binding to the human transferrin receptor (hTfR) (Xia et al. J. Virol. 74, 11359-11366 (2000)); the OX26 monoclonal antibody to the transferrin receptor (Pardridge et al. J. Pharmacol. Exp. Ther. 259, 66-70 (1991)); and SEQ ID NOs: 1-18 of U.S. Patent No. 6,306,365.

Enhanced brain delivery of a LINGO-1 or TrkB composition is determined by a number of means well established in the art. For example, administering to an animal a radioactively, enzymatically or fluorescently labeled LINGO-1 antagonist or TrkB agonist polypeptide, aptamer, compound, antibody or antibody fragment thereof linked to a brain targeting moiety; determining brain localization; and comparing localization with an equivalent radioactively labeled LINGO-1 antagonist or TrkB agonist polypeptide, aptamer, compound, antibody or antibody fragment thereof that is not associated with a brain targeting moiety. Other means of determining enhanced targeting are described in the above references.

The signal sequence is a polynucleotide that encodes an amino acid sequence that initiates transport of a protein across the membrane of the endoplasmic reticulum. Signal sequences useful for constructing an immunofusin include antibody light chain signal sequences, *e.g*., antibody 14.18 (Gillies et al., J. Immunol. Meth. 125:191-202 (1989)), antibody heavy chain signal sequences, *e.g*., the MOPC141 antibody heavy chain signal sequence (Sakano et al., Nature 286:5774 (1980)). Alternatively, other signal sequences can be used. *See, e.g*., Watson, Nucl. Acids Res. 12:5145 (1984). The signal peptide is usually cleaved in the lumen of the endoplasmic reticulum by signal peptidases. This results in the secretion of an immunofusin protein containing the Fc region and the soluble LINGO-1 or TrkB-agonist moiety.

In some embodiments, the DNA sequence may encode a proteolytic cleavage site between the secretion cassette and the LINGO-1 or TrkB-agonist moiety. Such a cleavage site may provide, *e.g*., for the proteolytic cleavage of the encoded fusion protein, thus separating the Fc domain from the target protein. Useful proteolytic cleavage sites include amino acid sequences recognized by proteolytic enzymes such as trypsin, plasmin, thrombin, factor Xa, or enterokinase K.

The secretion cassette can be incorporated into a replicable expression vector. Useful vectors include linear nucleic acids, plasmids, phagemids, cosmids and the like. An exemplary expression vector is pdC, in which the transcription of the immunofusin DNA is placed under the control of the enhancer and promoter of the human cytomegalovirus. *See, e.g.,* Lo et al., Biochim. Biophys. Acta 1088:712 (1991); and Lo et al., Protein Engineering 11:495-500 (1998). An appropriate host cell can be transformed or transfected with a DNA that encodes a soluble LINGO-1 or TrkB-agonist polypeptide and used for the expression and secretion of the soluble LINGO-1 or TrkB-agonist polypeptide. Host cells that are typically used include immortal hybridoma cells, myeloma cells, 293 cells, Chinese hamster ovary (CHO) cells, HeLa cells, and COS cells.

In one embodiment, a soluble LINGO-1 antagonist or TrkB agonist polypeptide is fused to a hinge and Fc region, *i.e*., the C-terminal portion of an Ig heavy chain constant region. Potential advantages of a LINGO-1-Fc of TrkB-agoanist-Fc fusion include solubility, *in vivo* stability, and multivalency, *e.g*., dimerization. The Fc region used can be an IgA, IgD, or IgG Fc region (hinge- C_{H}2- C_{H}3). Alternatively, it can be an IgE or IgM Fc region (hinge- C_{H}2- C_{H}3-C_{H}4). An IgG Fc region is generally used, *e.g*., an IgG₁ Fc region or IgG₄ Fc region. In one embodiment, a sequence beginning in the hinge region just upstream of the papain cleavage site which defines IgG Fc chemically (*i.e.* residue 216, taking the first residue of heavy chain constant region to be 114 according to the Kabat system), or analogous sites of other immunoglobulins, is used in the fusion. The precise site at which the fusion is made is not critical; particular sites are well known and may be selected in order to optimize the biological activity, secretion, or binding characteristics of the molecule. Materials and methods for constructing and expressing DNA encoding Fc fusions are known in the art and can be applied to obtain soluble LINGO-1-antagonist or TrkB-agonist fusions without undue experimentation. Some embodiments of the invention employ a LINGO-1-antagonist or TrkB-agonist fusion protein such as those described in Capon et al., U.S. Patent Nos. 5,428,130 and 5,565,335.

Fully intact, wild-type Fc regions display effector functions that normally are unnecessary and undesired in an Fc fusion protein used in the present invention. Therefore, certain binding sites typically are deleted from the Fc region during the construction of the secretion cassette. For example, since coexpression with the light chain is unnecessary, the binding site for the heavy chain binding protein, Bip (Hendershot et al., Immunol. Today 8:111-14 (1987)), is deleted from the C_{H}2 domain of the Fc region of IgE, such that this site does not interfere with the efficient secretion of the immunofusin. Transmembrane domain sequences, such as those present in IgM, also are generally deleted.

The IgG₁ Fc region is most often used. Alternatively, the Fc region of the other subclasses of immunoglobulin gamma (gamma-2, gamma-3 and gamma-4) can be used in the secretion cassette. The IgG₁ Fc region of immunoglobulin gamma-1 is generally used in the secretion cassette and includes at least part of the hinge region, the C_{H}2 region, and the C_{H}3 region. In some embodiments, the Fc region of immunoglobulin gamma-1 is a C_{H}2-deleted-Fc, which includes part of the hinge region and the C_{H}3 region, but not the C_{H}2 region. A C_{H}2-deleted-Fc has been described by Gillies et al. Hum. Antibod. Hybridomas 1:47 (1990). In some embodiments, the Fc region of one of IgA, IgD, IgE, or IgM, is used.

LINGO-1-antagonist-Fc or TrkB-agonist-Fc fusion proteins can be constructed in several different configurations. In one configuration the C-terminus of the soluble LINGO-1 or TrkB-agonist moiety is fused directly to the N-terminus of the Fc hinge moiety. In a slightly different configuration, a short polypeptide, *e.g*., 2-10 amino acids, is incorporated into the fusion between the N-terminus of the soluble LINGO-1 or TrkB-agonist moiety and the C-terminus of the Fc moiety. Such a linker provides conformational flexibility, which may improve biological activity in some circumstances. If a sufficient portion of the hinge region is retained in the Fc moiety, the LINGO-1-Fc or TrkB-agonist-Fc fusion will dimerize, thus forming a divalent molecule. A homogeneous population of monomeric Fc fusions will yield monospecific, bivalent dimers. A mixture of two monomeric Fc fusions each having a different specificity will yield bispecific, bivalent dimers.

Any of a number of cross-linkers that contain a corresponding amino-reactive group and thiol-reactive group can be used to link LINGO-1 antagonist or TrkB agonist polypeptides to serum albumin. Examples of suitable linkers include amine reactive cross-linkers that insert a thiol-reactive maleimide, *e.g*., SMCC, AMAS, BMPS, MBS, EMCS, SMPB, SMPH, KMUS, and GMBS. Other suitable linkers insert a thiol-reactive haloacetate group, *e.g*., SBAP, SIA, SIAB. Linkers that provide a protected or non-protected thiol for reaction with sulfhydryl groups to product a reducible linkage include SPDP, SMPT, SATA, and SATP. Such reagents are commercially available (*e.g*., Pierce Chemicals).

Conjugation does not have to involve the N-terminus of a soluble LINGO-1 or TrkB-agonist polypeptide or the thiol moiety on serum albumin. For example, soluble LINGO-1-albumin or TrkB-agonist-albumin fusions can be obtained using genetic engineering techniques, wherein the soluble LINGO-1 or TrkB-agonist moiety is fused to the serum albumin gene at its N-terminus, C-terminus, or both.

Soluble LINGO-1 or TrkB-agonist polypeptides can be fused at the N- or C-terminus to heterologous peptides in order to facilitate purification or identification of the soluble LINGO-1 or TrkB-agonist moiety. For example, a histidine tag can be fused to a soluble LINGO-1 or TrkB-agonist polypeptide to facilitate purification using commercially available chromatography media. Additionally, an epitope tag enables soluble LINGO-1 or TrkB-agonist fusion polypeptides to be readily purified by affinity purification using an anti-tag antibody or another type of affinity matrix that binds to the epitope tag. Many examples of such purification tags are known in the art and include, but are not limited to, poly-histidine (poly-his) or poly-histidine-glycine (poly-his-gly) tags; the influenza hemagglutinin (HA) tag polypeptide and its antibody 12CA5 (Field et al., Mol. Cell. Biol, 8:2159-2165 (1988)); the c-myc tag and the 8F9, 3C7, 6E10, G4, B7 and 9E10 antibodies thereto (Evan et al., Mol. Cell. Bio. 5:3610-3616 (1985)); and the Herpes Simplex virus glycoprotein D (gD) tag and its antibody (Paborsky et al., Protein Engineering 3(6):547-553 (1990)). Other tag polypeptides include the Flag-peptide (Hopp et al., BioTechnology 6:1204-1210 (1988)); the KT3 epitope peptide (Martin et al., Science 255:192-194 (1992)); an α -tubulin epitope peptide *(*Skinner et al., J. Biol. Chem. 266:15163-15166 (1991)); and the T7 gene 10 protein peptide tag (Lutz-Freyermuth et al., Proc. Natl. Acad. Sci. USA, 87:6393-6397 (1990)). The tag can be any peptide epitope which is recognized by an antibody and does not interfere with the function of the soluble LINGO-1 or TrkB-agonist polypeptide.

In some embodiments of the invention, a soluble LINGO-1 or TrkB-agonist fusion construct is used to enhance the production of a soluble LINGO-1 or TrkB-agonist moiety in bacteria: In such constructs a bacterial protein normally expressed and/or secreted at a high level is employed as the N-terminal fusion partner of a soluble LINGO-1 or TrkB-agonist polypeptide. *See, e.g.*, Smith et al., Gene 67:31 (1988); Hopp et al., Biotechnology 6:1204 (1988); La Vallie et al., Biotechnology 11:187 (1993).

By fusing a soluble LINGO-1 or TrkB-agonist moiety at the amino and carboxy termini of a suitable fusion partner, bivalent or tetravalent forms of a soluble LINGO-1 or TrkB-agonist polypeptide can be obtained. For example, a soluble LINGO-1 or TrkB-agonist moiety can be fused to the amino and carboxy termini of an Ig moiety to produce a bivalent monomeric polypeptide containing two soluble LINGO-1 or TrkB-agonist moieties. Upon dimerization of two of these monomers, by virtue of the Ig moiety, a tetravalent form of a soluble LINGO-1 or TrkB-agonist protein is obtained. Such multivalent forms can be used to achieve increased binding affinity for the target. Multivalent forms of soluble LINGO-1 or TrkB-agonist also can be obtained by placing soluble LINGO-1 or TrkB-agonist moieties in tandem to form concatamers, which can be employed alone or fused to a fusion partner such as Ig or HSA.

### Conjugated Polymers (other than polypeptides)

Some embodiments of the invention involve a soluble LINGO-1 or TrkB-agonist polypeptide, LINGO-1-antagonist or TrkB-agonist aptamer, TrkB agonist compound or antagonistic LINGO-1 or agonistic TrkB antibody wherein one or more polymers are conjugated (covalently linked) to the LINGO-1 or TrkB-agonist polypeptide, compound, aptamer or antibody for use in the present invention. Examples of polymers suitable for such conjugation include polypeptides (discussed above), aptamers, sugar polymers and polyalkylene glycol chains. Typically, but not necessarily, a polymer is conjugated to the soluble LINGO-1 or TrkB-agonist polypeptide, aptamer, TrkB agonist compound or LINGO-1 or TrkB antibody for the purpose of improving one or more of the following: solubility, stability, or bioavailability.

The class of polymer generally used for conjugation to a LINGO-1 antagonist polypeptide, compound, aptamer or antibody or to a TrkB agonist polypeptide, compound, aptamer or antibody is a polyalkylene glycol. Polyethylene glycol (PEG) is most frequently used. PEG moieties, *e.g*., 1, 2, 3, 4 or 5 PEG polymers, can be conjugated to each LINGO-1 antagonist or TrkB agonist polypeptide, aptamer, or antibody, or TrkB agonist compound to increase serum half life, as compared to the LINGO-1 antagonist or TrkB agonist polypeptide, aptamer, compound or antibody alone. PEG moieties are non-antigenic and essentially biologically inert. PEG moieties used in the practice of the invention may be branched or unbranched.

The number of PEG moieties attached to the LINGO-1 antagonist or TrkB agonist polypeptide, aptamer, compound or antibody and the molecular weight of the individual PEG chains can vary. In general, the higher the molecular weight of the polymer, the fewer polymer chains attached to the polypeptide. Usually, the total polymer mass attached to the LINGO-1 antagonist or TrkB agonist polypeptide, compound, aptamer or antibody is from 20 kDa to 40 kDa. Thus, if one polymer chain is attached, the molecular weight of the chain is generally 20-40 kDa. If two chains are attached, the molecular weight of each chain is generally 10-20 kDa. If three chains are attached, the molecular weight is generally 7-14 kDa.

The polymer, *e.g*., PEG, can be linked to the LINGO-1 antagonist or TrkB agonist polypeptide, aptamer or antibody through any suitable, exposed reactive group on the polypeptide. The exposed reactive group(s) can be, *e.g.*, an N-terminal amino group or the epsilon amino group of an internal lysine residue, or both. An activated polymer can react and covalently link at any free amino group on the LINGO-1 antagonist or TrkB agonist polypeptide, aptamer or antibody. Free carboxylic groups, suitably activated carbonyl groups, hydroxyl, guanidyl, imidazole, oxidized carbohydrate moieties and mercapto groups of the LINGO-1 antagonist or TrkB agonist polypeptide, aptamer or antibody (if available) also can be used as reactive groups for polymer attachment.

In a conjugation reaction, from about 1.0 to about 10 moles of activated polymer per mole of polypeptide, depending on polypeptide concentration, is typically employed. Usually, the ratio chosen represents a balance between maximizing the reaction while minimizing side reactions (often non-specific) that can impair the desired pharmacological activity of the LINGO-1 antagonist or TrkB agonist polypeptide or antibody. In some embodiments, at least 50% of the biological activity (as demonstrated, *e.g.*, in any of the assays described herein or known in the art) of the LINGO-1 antagonist or TrkB agonist polypeptide, aptamer or antibody is retained, and in some embodiments nearly 100% is retained.

The polymer can be conjugated to the LINGO-1 antagonist or TrkB agonist polypeptide, aptamer or antibody using conventional chemistry. For example, a polyalkylene glycol moiety can be coupled to a lysine epsilon amino group of the LINGO-1 antagonist or TrkB agonist polypeptide, aptamer or antibody. Linkage to the lysine side chain can be performed with an N-hydroxylsuccinimide (NHS) active ester such as PEG succinimidyl succinate (SS-PEG) and succinimidyl propionate (SPA-PEG). Suitable polyalkylene glycol moieties include, *e.g*., carboxymethyl-NHS and norleucine-NHS, SC. These reagents are commercially available. Additional amine-reactive PEG linkers can be substituted for the succinimidyl moiety. These include, *e.g*., isothiocyanates, nitrophenylcarbonates (PNP), epoxides, benzotriazole carbonates, SC-PEG, tresylate, aldehyde, epoxide, carbonylimidazole and PNP carbonate. Conditions are usually optimized to maximize the selectivity and extent of reaction. Such optimization of reaction conditions is within ordinary skill in the art.

PEGylation can be carried out by any of the PEGylation reactions known in the art. *See, e.g.,* Focus on Growth Factors 3:4-10 (1992), and European patent applications EP 0 154 316 and EP 0 401 384. PEGylation may be carried out using an acylation reaction or an alkylation reaction with a reactive polyethylene glycol molecule (or an analogous reactive water-soluble polymer).

PEGylation by acylation generally involves reacting an active ester derivative of polyethylene glycol. Any reactive PEG molecule can be employed in the PEGylation. PEG esterified to N-hydroxysuccinimide (NHS) is a frequently used activated PEG ester. As used herein, "acylation" includes without limitation the following types of linkages between the therapeutic protein and a water-soluble polymer such as PEG: amide, carbamate, urethane, and the like. *See, e.g.,* Bioconjugate Chem. 5:133-140, 1994. Reaction parameters are generally selected to avoid temperature, solvent, and pH conditions that would damage or inactivate the soluble LINGO-1 or TrkB-agonist polypeptide, aptamer or antibody.

Generally, the connecting linkage is an amide and typically at least 95% of the resulting product is mono-, di- or tri-PEGylated. However, some species with higher degrees of PEGylation may be formed in amounts depending on the specific reaction conditions used. Optionally, purified PEGylated species are separated from the mixture, particularly unreacted species, by conventional purification methods, including, *e.g*., dialysis, salting-out, ultrafiltration, ion-exchange chromatography, gel filtration chromatography, hydrophobic exchange chromatography, and electrophoresis.

PEGylation by alkylation generally involves reacting a terminal aldehyde derivative of PEG with LINGO-1 antagonist or TrkB agonist polypeptide, aptamer or antibody in the presence of a reducing agent. In addition, one can manipulate the reaction conditions to favor PEGylation substantially only at the N-terminal amino group of LINGO-1 antagonist or TrkB agonist polypeptide, aptamer or antibody, *i.e.* a mono-PEGylated protein. In either case of mono-PEGylation or poly-PEGylation, the PEG groups are typically attached to the protein via a - CH₂-NH- group. With particular reference to the - CH₂- group, this type of linkage is known as an "alkyl" linkage.

Derivatization via reductive alkylation to produce an N-terminally targeted mono-PEGylated product exploits differential reactivity of different types of primary amino groups (lysine versus the N-terminal) available for derivatization. The reaction is performed at a pH that allows one to take advantage of the pKa differences between the epsilon-amino groups of the lysine residues and that of the N-terminal amino group of the protein. By such selective derivatization, attachment of a water-soluble polymer that contains a reactive group, such as an aldehyde, to a protein is controlled: the conjugation with the polymer takes place predominantly at the N-terminus of the protein and no significant modification of other reactive groups, such as the lysine side chain amino groups, occurs.

The polymer molecules used in both the acylation and alkylation approaches are selected from among water-soluble polymers. The polymer selected is typically modified to have a single reactive group, such as an active ester for acylation or an aldehyde for alkylation, so that the degree of polymerization may be controlled as provided for in the present methods. An exemplary reactive PEG aldehyde is polyethylene glycol propionaldehyde, which is water stable, or mono C1-C10 alkoxy or aryloxy derivatives thereof (*see, e.g.*, Harris et al., U.S. Pat. No. 5,252,714). The polymer may be branched or unbranched. For the acylation reactions, the polymer(s) selected typically have a single reactive ester group. For reductive alkylation, the polymer(s) selected typically have a single reactive aldehyde group. Generally, the water-soluble polymer will not be selected from naturally occurring glycosyl residues, because these are usually made more conveniently by mammalian recombinant expression systems.

Methods for preparing a PEGylated LINGO-1 antagonist or TrkB-agonist polypeptide, aptamer or antibody generally includes the steps of (a) reacting a LINGO-1 antagonist or TrkB agonist polypeptide, aptamer or antibody with polyethylene glycol (such as a reactive ester or aldehyde derivative of PEG) under conditions whereby the molecule becomes attached to one or more PEG groups, and (b) obtaining the reaction product(s). In general, the optimal reaction conditions for the acylation reactions will be determined case-by-case based on known parameters and the desired result. For example, a larger ratio of PEG to protein generally leads to a greater the percentage of poly-PEGylated product.

, Reductive alkylation to produce a substantially homogeneous population of mono-polymer/soluble LINGO-1 antagonist or TrkB-agonist polypeptide, LINGO-1 antagonist or TrkB agonist aptamer or antagonistic LINGO-1 antibody or agonistic TrkB antibody generally includes the steps of: (a) reacting a LINGO-1-antagonist or TrkB-agonist protein or polypeptide with a reactive PEG molecule under reductive alkylation conditions, at a pH suitable to pen-nit selective modification of the N-terminal amino group of the polypeptide or antibody; and (b) obtaining the reaction product(s).

For a substantially homogeneous population of mono-polymer/soluble LINGO-1 or TrkB-agonist polypeptide, LINGO-1 antagonist or TrkB agonist aptamer or antagonistic LINGO-1 antibody or agonistic TrkB antibody, the reductive alkylation reaction conditions are those that permit the selective attachment of the water-soluble polymer moiety to the N-terminus of the polypeptide or antibody. Such reaction conditions generally provide for pKa differences between the lysine side chain amino groups and the N-terminal amino group. For purposes of the present invention, the pH is generally in the range of 3-9, typically 3-6.

LINGO-1 antagonist or TrkB-agonist polypeptides, aptamers or antibodies can include a tag, *e.g*., a moiety that can be subsequently released by proteolysis. Thus, the lysine moiety can be selectively modified by first reacting a His-tag modified with a low-molecular-weight linker such as Traut's reagent (Pierce) which will react with both the lysine and N-terminus, and then releasing the His tag. The polypeptide will then contain a free SH group that can be selectively modified with a PEG containing a thiol-reactive head group such as a maleimide group, a vinylsulfone group, a haloacetate group, or a free or protected SH.

Traut's reagent can be replaced with any linker that will set up a specific site for PEG attachment. For example, Traut's reagent can be replaced with SPDP, SMPT, SATA, or SATP (Pierce). Similarly, one could react the protein with an amine-reactive linker that inserts a maleimide (for example SMCC, AMAS, BMPS, MBS, EMCS, SMPB, SMPH, KMUS, or GMBS), a haloacetate group (SBAP, SIA, SIAB), or a vinylsulfone group and react the resulting product with a PEG that contains a free SH.

In some embodiments, the polyalkylene glycol moiety is coupled to a cysteine group of the LINGO-1 antagonist or TrkB agonist polypeptide, aptamer or antibody for use in the present invention. Coupling can be effected using, *e.g*., a maleimide group, a vinylsulfone group, a haloacetate group, or a thiol group.

Optionally, the LINGO-1 antagonist or TrkB-agonist polypeptide, aptamer or antibody is conjugated to the polyethylene-glycol moiety through a labile bond. The labile bond can be cleaved in, *e.g*., biochemical hydrolysis, proteolysis, or sulfhydryl cleavage. For example, the bond can be cleaved under *in vivo* (physiological) conditions.

The reactions may take place by any suitable method used for reacting biologically active materials with inert polymers, generally at about pH 5-8, *e.g*., pH 5, 6, 7, or 8, if the reactive groups are on the alpha amino group at the N-terminus. Generally the process involves preparing an activated polymer and thereafter reacting the protein with the activated polymer to produce the soluble protein suitable for formulation.

### LINGO-1 Antagonist and TrkB Agonist Polynucleotides

### LINGO-1 Antagonist Polynucleotides

LINGO-1 antagonists in the present invention include a LINGO-1 antagonist polynucleotide which comprises a nucleic acid molecule which specifically binds to a polynucleotide which encodes LINGO-1. The LINGO-1 polynucleotide antagonist generally prevents expression of LINGO-1 (knockdown). LINGO-1 antagonists include, but are not limited to antisense molecules, ribozymes, siRNA, shRNA, RNAi. Typically, such binding molecules are separately administered to the animal (*see*, for example, O'Connor, J. Neurochem. 56:560 (1991), but such binding molecules may also be expressed *in vivo* from polynucleotides taken up by a host cell and expressed *in vivo. See also* Oligodeoxynucleotides as Antisense Inhibitors of Gene Expression, CRC Press, Boca Raton, FL (1988).

RNAi refers to the expression of an RNA which interferes with the expression of the targeted mRNA. Specifically, the RNAi silences a targeted gene via interacting with the specific mRNA (*e.g*. LINGO-1 or TrkB) through an siRNA (short interfering RNA). The ds RNA complex is then targeted for degradation by the cell. Additional RNAi molecules include short hairpin RNA (shRNA); also short interfering hairpin. The shRNA molecule contains sense and antisense sequences from a target gene connected by a loop. The shRNA is transported from the nucleus into the cytoplasm, it is degraded along with the mRNA. Pol III or U6 promoters can be used to express RNAs for RNAi. In some embodiments of the invention, the shRNA is expressed from a lentiviral vector (*e.g*. pLL3.7).

RNAi is mediated by double stranded RNA (dsRNA) molecules that have sequence-specific homology to their "target" mRNAs (Caplen et al., Proc Natl Acad Sci USA 98:9742-9747 (2001)). Biochemical studies in *Drosophila*. cell-free lysates indicates that the mediators of RNA-dependent gene silencing are 21-25 nucleotide "small interfering" RNA duplexes (siRNAs). Accordingly, siRNA molecules are advantageously used in the present invention. The siRNAs are derived from the processing of dsRNA by an RNase known as DICER (Bernstein et al., Nature 409:363-366 (2001)). It appears that siRNA duplex products are recruited into a multi-protein siRNA complex termed RISC (RNA Induced Silencing Complex). Without wishing to be bound by any particular theory, it is believed that a RISC is guided to a target mRNA, where the siRNA duplex interacts sequence-specifically to mediate cleavage in a catalytic fashion (Bernstein et al., Nature 409:363-366 (2001); Boutla et al., Curr Biol 11:1776-1780 (2001)).

RNAi has been used to analyze gene function and to identify essential genes in mammalian cells (Elbashir et al., Methods 26:199-213 (2002); Harborth et al., J Cell Sci 114:4557-4565 (2001)), including by way of non-limiting example neurons (Krichevsky et al., Proc Natl Acad Sci USA 99:11926-11929 (2002)). RNAi is also being evaluated for therapeutic modalities, such as inhibiting or blocking the infection, replication and/or growth of viruses, including without limitation poliovirus (Gitlin et al., Nature 418:379-380 (2002)) and HIV (Capodici et al., J Immunol 169:5196-5201 (2002)), and reducing expression of oncogenes (*e.g.,* the bcr-abl gene; Scherr et al., Blood 101(4):1566-9 (2002)). RNAi has been used to modulate gene expression in mammalian (mouse) and amphibian (*Xenopus*) embryos (respectively, Calegari et al., Proc Natl Acad Sci USA 99:14236-14240 (2002); and Zhou, et al., Nucleic Acids Res 30:1664-1669 (2002)), and in postnatal mice (Lewis et al., Nat Genet 32:107-108 (2002)), and to reduce transgene expression in adult transgenic mice (McCaffrey et al., Nature 418:38-39 (2002)). Methods have been described for determining the efficacy and specificity of siRNAs in cell culture and *in vivo* (see, *e.g.,* Bertrand et al., Biochem Biophys Res Commun 296:1000-1004 (2002); Lassus et al., Sci STKE 2002(147):PL13 (2002); and Leirdal et al., Biochem Biophys Res Commun 295:744-748 (2002)).

Molecules that mediate RNAi, including without limitation siRNA, can be *produced in vitro* by chemical synthesis (Hohjoh, FEBS Lett 521:195-199 (2002)), hydrolysis of dsRNA (Yang et al., Proc Natl Acad Sci USA 99:9942-9947 (2002)), by *in vitro* transcription with T7 RNA polymerase (Donzeet et al., Nucleic Acids Res 30:e46, (2002); Yu et al., Proc Natl Acad Sci USA 99:6047-6052 (2002)), and by hydrolysis of double-stranded RNA using a nuclease such as E. coli RNase III (Yang et al., Proc Natl Acad Sci USA 99:9942-9947 (2002)).

siRNA molecules may also be formed by annealing two oligonucleotides to each other, typically have the following general structure, which includes both double-stranded and single-stranded portions:

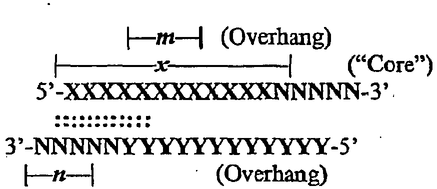

Wherein N, X and Y are nucleotides; X hydrogen bonds to Y; ":" signifies a hydrogen bond between two bases; x is a natural integer having a value between 1 and about 100; and ***m*** and ***n*** are whole integers having, independently, values between 0 and about 100. In some embodiments, N, X and Y are independently A, G, C and T or U. Non-naturally occurring bases and nucleotides can be present, particularly in the case of synthetic siRNA (*i.e.,* the product of annealing two oligonucleotides). The double-stranded central section is called the "core" and has base pairs (bp) as units of measurement; the single-stranded portions are overhangs, having nucleotides (nt) as units of measurement. The overhangs shown are 3' overhangs, but molecules with 5' overhangs are also within the scope of the invention. Also within the scope of the invention are siRNA molecules with no overhangs (*i.e., **m*** = 0 and ***n*** = 0), and those having an overhang on one side of the core but not the other (*e.g., **m*** = 0 and ***n*** ≥ 1, or vice-versa).

Initially, RNAi technology did not appear to be readily applicable to mammalian systems. This is because, in mammals, dsRNA activates dsRNA-activated protein kinase (PKR) resulting in an apoptotic cascade and cell death (Der et al, Proc. NatL Acad. Sci. USA 94:3279-3283 (1997)). In addition, it has long been known that dsRNA activates the interferon cascade in mammalian cells, which can also lead to altered cell physiology (Colby et al, Annu. Rev. Microbiol. 25:333 (1971); Kleinschmidt et al., Annu. Rev. Biochem. 41:517 (1972); Lampson et al., Proc. Natl. Acad. Sci. USA 58L782 (1967); Lomniczi et al., J. Gen. Virol. 8:55 (1970); and Younger et al., J. Bacteriol. 92:862 (1966)). However, dsRNA-mediated activation of the PKR and interferon cascades requires dsRNA longer than about 30 base pairs. In contrast, dsRNA less than 30 base pairs in length has been demonstrated to cause RNAi in mammalian cells (Caplen et al.;. Proc. Natl. Acad. Sci. USA 98:9742-9747 (2001)). Thus, it is expected that undesirable, non-specific effects associated with longer dsRNA molecules can be avoided by preparing short RNA that is substantially free from longer dsRNAs.

References regarding siRNA: Bernstein et al., Nature 409:363-366 (2001); Boutla et al., Curr Biol 11:1776-1780 (2001); Cullen, Nat Immunol. 3:597-599 (2002); Caplen et al., Proc Natl Acad Sci USA 98:9742-9747 (2001); Hamilton et al., Science 286:950-952 (1999); Nagase et al., DNA Res. 6:63-70 (1999); Napoli et al., Plant Cell 2:279-289 (1990); Nicholson et al., Mamm. Genome 13:67-73 (2002); Parrish et al., Mol Cell 6:1077-1087 (2000); Romano et al., Mol Microbiol 6:3343-3353 (1992); Tabara et al., Cell 99:123-132 (1999); and Tuschl, Chembiochem. 2:239-245 (2001).

Paddison et al. (Genes & Dev. 16:948-958 (2002)) have used small RNA molecules folded into hairpins as a means to effect RNAi. Accordingly, such short hairpin RNA (shRNA) molecules are also advantageously used in the invention. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the dsRNA products of the DICER RNase and, in any event, have the same capacity for inhibiting expression of a specific gene.

Antisense technology can be used to control gene expression through antisense DNA or RNA, or through triple-helix formation. Antisense techniques are discussed, for example, in Okano, J. Neurochem. 56:560 (1991); Oligodeoxynucleotides as Antisense Inhibitors of Gene Expression, CRC Press, Boca Raton, FL (1988). Triple helix formation is discussed in, for instance, Lee et al., Nucleic Acids Research 6:3073 (1979); Cooney et al., Science 241:456 (1988); and Dervan et al., Science 251:1300 (1991). The methods are based on binding of a polynucleotide to a complementary DNA or RNA.

For example, the 5' coding portion of a polynucleotide that encodes LINGO-1 may be used to design an antisense RNA oligonucleotide of from about 10 to 40 base pairs in length. A DNA oligonucleotide is designed to be complementary to a region of the gene involved in transcription, thereby preventing transcription and the production of the target protein. The antisense RNA oligonucleotide hybridizes to the mRNA *in vivo* and blocks translation of the mRNA molecule into the target polypeptide.

In one embodiment, antisense nucleic acids specific for the LINGO-1 gene are produced intracellularly by transcription from an exogenous sequence. For example, a vector or a portion thereof, is transcribed, producing an antisense nucleic acid (RNA). Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the antisense molecule, can be by any promoter known in the art to act in vertebrate, especially human cells, such as those described elsewhere herein.

Absolute complementarity of an antisense molecule is not required. A sequence complementary to at least a portion of an RNA encoding LINGO-1 means a sequence having sufficient complementarity to be able to hybridize with the RNA, forming a stable duplex; or triplex formation may be assayed. The ability to hybridize will depend on both the degree of complementarity and the length of the antisense nucleic acid. Generally, the larger the hybridizing nucleic acid, the more base mismatches it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of a messenger RNA, *e.g.,* the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. *See,* generally, Wagner, R., Nature 372:333-335 (1994). Thus, oligonucleotides complementary to either the 5'- or 3'- non-translated, non-coding regions could be used in an antisense approach to inhibit translation of LINGO-1. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Antisense nucleic acids should be at least six nucleotides in length, and in some embodiments are oligonucleotides ranging from 6 to about 50 nucleotides in length. In specific aspects the oligonucleotide is at least 10 nucleotides, at least 17 nucleotides, at least 25 nucleotides or at least 50 nucleotides.

In yet another embodiment, an antisense oligonucleotide for use in the methods disclosed herein is an α-anomeric oligonucleotide. An α-anomeric oligonucleotide forms specific double-stranded hybrids with complementary RNA in which, contrary to the usual situation, the strands run parallel to each other (Gautier et al., Nucl. Acids Res. 15:6625-6641(1987)). The oligonucleotide is a 2'-0-methylribonucleotide (Inoue et al., Nucl. Acids Res. 15:6131-6148(1987)), or a chimeric RNA-DNA analogue (Inoue et al., FEBS Lett. 215:327-330 (1987)).

Polynucleotide compositions for use in the methods disclosed herein further include catalytic RNA, or a ribozyme (*See, e.g.,* PCT International Publication WO 90/11364, published October 4, 1990; Sarver et al., Science 247:1222-1225 (1990)). In some embodiments of the invention, hammerhead ribozymes are used. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, Nature 334:585-591 (1988). The ribozyme can be engineered so that the cleavage recognition site is located near the 5' end of the target mRNA; *i.e.,* to increase efficiency and minimize the intracellular accumulation of non-functional mRNA transcripts.

As in the antisense approach, ribozymes for use in the methods disclosed herein can be composed of modified oligonucleotides (*e.g.* for improved stability, targeting, etc.) and may be delivered to cells which express LINGO-1 *in vivo.* DNA constructs encoding the ribozyme may be introduced into the cell in the same manner as described above for the introduction of antisense encoding DNA. One method of delivery involves using a DNA construct "encoding" the ribozyme under the control of a strong constitutive promoter, such as, for example, pol III or pol II promoter, so that transfected cells will produce sufficient quantities of the ribozyme to destroy endogenous LINGO-1 messages and inhibit translation. Since ribozymes, unlike antisense molecules, are catalytic, a lower intracellular concentration is required for efficiency.

### TrkB Agonist Polynucleotides

TrkB agonists for use in the present invention include a TrkB agonist polynucleotide which comprises a nucleic acid molecule which encodes a TrkB polypeptide, fragment, isoform or variant thereof. The TrkB agonist polynucleotides for use according to the invention also include nucleic acid molecules which encode a TrkB ligand polypeptide, fragment, isoform or variant thereof. In some embodiments, the TrkB agonist polynucleotide encodes BDNF.: TrkB agonist polynucleotides include any polynucelotide which encodes a TrkB agonist polypeptide of the present invention.

### LINGO-1 Antagonists and/or TrkB Agonist Polynucleotides

Polynucleotides for use in the methods disclosed herein, including aptamers described *supra,* can be DNA or RNA or chimeric mixtures or derivatives or modified versions thereof, single-stranded or double-stranded. The polynucleotide can be modified at the base moiety, sugar moiety, or phosphate backbone, for example, to improve stability of the molecule, hybridization, etc. The polynucleotide may include other appended groups such as peptides *(e.g.,* for targeting host cell *receptors in vivo),* or agents facilitating transport across the cell membrane (see, *e.g.,* Letsinger et al., Proc. Natl. Acad. Sci. U.S.A. 86:6553-6556 (1989); Lemaitre et al., Proc. Natl. Acad. Sci. 84:648-652 (1987)); PCT Publication No. WO88/09810, published December 15, 1988) or the blood-brain barrier (see, *e.g.,* PCT Publication No. WO89/10134, published April 25, 1988), hybridization-triggered cleavage agents. (*See, e.g.,* Krol et al., BioTechniques 6:958-976 (1988)) or intercalating agents. (*See, e.g.,* Zon, Pharm. Res. 5:539-549(1988)). To this end, the polynucleotide may be conjugated to another molecule, *e.g*., a peptide, hybridization triggered cross-linking agent, transport agent, hybridization-triggered cleavage agent, etc.

Polynucleotides, including aptamers, for use in the methods disclosed herein may comprise at least one modified base moiety which is selected from the group including, but not limited to, 5fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N-6-isopentenyladenine, 1-methylguanine, 1-methylinosine, 2,2-dimethylguanine, 2-methyladenine, 2-methylguanine, 3-methylcytosine, 5-methylcytosine, N-6-adenine, 7-methylguanine, 5-methylaminomethyluracil, 5-methoxyaminomethyl-2-thiouracil, beta-D-mannosylqueosine, 5'methoxycarboxymethyluracil, 5-methoxyuracil, 2-methylthio-N-6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3(3-amino-3-N2-carboxypropyl) uracil, (acp3)w, and 2,6-diaminopurine.

Polynucleotides, including aptamers, for use.in the methods disclosed herein may also comprise at least one modified sugar moiety selected from the group including, but not limited to, arabinose, 2-fluoroarabinose, xylulose, and hexose.

In yet another embodiment, a polynucleotide, including an aptamer, for use in the methods disclosed herein comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

Polynucleotides, including aptamers, for use in the invention may be synthesized by standard methods known in the art, *e.g.* by use of an automated DNA synthesizer (such as are commercially available from Biosearch, Applied Biosystems, etc.). As examples, phosphorothioate oligonucleotides may be synthesized by the method of Stein et al., Nucl. Acids Res. 16:3209 (1988), methylphosphonate oligonucleotides can be prepared by use of controlled pore glass polymer supports (Sarin et al., Proc. Natl. Acad Sci. U.S.A. 85:7448-7451 (1988)), etc.

### Vectors and Host Cells

Host-expression systems represent vehicles by which the coding sequences of interest may be produced and subsequently purified, but also represent cells which may, when transformed or transfected with the appropriate nucleotide coding sequences, express an antibody molecule for use according to the invention *in situ.* These include but are not limited to microorganisms such as bacteria (*e.g., E. coli, B. subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g.,* baculovirus) containing antibody coding sequences; plant cell systems infected with recombinant virus expression vectors (*e.g.,* cauliflower mosaic virus, CaMV; tobacco mosaic virus, TMV) or transformed with recombinant plasmid expression vectors (*e.g.,* Ti plasmid) containing antibody coding sequences; or mammalian cell systems *(e.g.,* COS, CHO, BLK, 293, 3T3 cells) harboring recombinant expression constructs containing promoters derived from the genome of mammalian cells (*e.g.,* metallothionein promoter) or from mammalian viruses (*e.g.,* the adenovirus late promoter; the vaccinia virus 7.5K promoter). Bacterial cells such as *Escherichia coli,* or eukaryotic cells (especially for the expression of whole recombinant antibody molecule) are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., EMBD J. 2:1791 (1983)), in which the antibody coding sequence may be ligated individually into the vector in frame with the lacZ coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is typically used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g.,* region E1 or E3) will result in a recombinant virus that is viable and capable of expressing the antibody molecule in infected hosts. (*e.g.,* see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*see* Bittner et al., Methods in Enzymol. 153:51-544 (1987)).

In addition, a host cell strain may be chosen which modulates the expression of the inserted sequences, or modifies and processes the gene product in the specific fashion desired. Such modifications (*e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, HeLa, COS, MDCK, 293, 3T3, WI38, and in particular, breast cancer cell lines such as, for example, BT483, Hs578T, HTB2, BT20 and T47D, and normal mammary gland cell line such as, for example, CRL7030 and Hs578Bst.

For long-term, high-yield production of recombinant proteins, stable expression may be used. For example, cell lines which stably express the antibody molecule may be engineered. Rather than using expression vectors which contain viral origins of replication, host cells can be transformed with DNA controlled by appropriate expression control elements (*e.g.,* promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

A. number of selection systems may be used including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 (1980)) genes, can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); TIB TECH 11(5):155-215 (May, 1993); and hygro, which confers resistance to hygromycin (Santerre et al., Gene 30:147 (1984). Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981).

The expression levels of an antibody molecule can be increased by vector amplification (for a review, *see* Bebbington and Hentschel, The use of vectors based on gene amplification for the expression of cloned genes in mammalian cells in DNA cloning, Academic Press, New York, Vol. 3. (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

Vectors comprising nucleic acids encoding LINGO-1 antagonist or TrkB agonists may also be used to produce polynucleotides or polypeptides for use in the invention. The choice of vector and expression control sequences to which such nucleic acids are operably linked depends on the functional properties desired, *e.g*., protein expression, and the host cell to be transformed.

Expression control elements useful for regulating the expression of an operably linked coding sequence are known in the art. Examples include, but are not limited to, inducible promoters, constitutive promoters, secretion signals, and other regulatory elements. When an inducible promoter is used, it can be controlled, *e.g*., by a change in nutrient status, or a change in temperature, in the host cell medium.

The vector can include a prokaryotic replicon, *i.e.,* a DNA sequence having the ability to direct autonomous replication and maintenance of the recombinant DNA molecule extra-chromosomally in a bacterial host cell. Such replicons are well known in the art. In addition, vectors that include a prokaryotic replicon may also include a gene whose expression confers a detectable marker such as a drug resistance. Examples of bacterial drug-resistance genes are those that confer resistance to ampicillin or tetracycline.

Vectors that include a prokaryotic replicon can also include a prokaryotic or bacteriophage promoter for directing expression of the coding gene sequences in a bacterial host cell. Promoter sequences compatible with bacterial hosts are typically provided in plasmid vectors containing convenient restriction sites for insertion of a DNA segment to be expressed. Examples of such plasmid vectors are pUC8, pUC9, pBR322 and pBR329 (BioRad), pPL and pKK223 (Pharmacia). Any suitable prokaryotic host can be used to express a recombinant DNA molecule encoding a protein used in the invention.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (*e.g.,* antibiotics) or resistance to heavy metals such as copper. The selectable marker gene can either be directly linked to the DNA sequences to be expressed, or introduced into the same cell by cotransformation. The neomycin phosphotransferase (neo) gene is an example of a selectable marker gene (Southern et al., J. Mol. Anal Genet. 1:327-341 (1982)). Additional elements may also be needed for optimal synthesis of mRNA. These elements may include signal sequences or splice signals, as well as transcriptional promoters, enhancers, and termination signals.

In one embodiment, an expression vector referred to as NEOSPLA (U.S. patent 6,159,730) may be used. This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. This vector has been found to result in very high-level expression upon transfection in CHO cells, followed by selection in G418-containing medium and methotrexate amplification. Of course, any expression vector which is capable of eliciting expression in eukaryotic cells may be used. Examples of suitable vectors include, but are not limited to, plasmids pcDNA3, pHCMV/Zeo, pCR3.1, pEF1/His, pIND/GS, pRc/HCMV2, pSV40/Zeo2, pTRACER-HCMV, pUB6/V5-His, pVAX1, and pZeoSV2 (available from Invitrogen, San Diego, CA), and plasmid pCI (available from Promega, Madison, WI). Additional eukaryotic cell expression vectors are known in the art and are commercially available. Typically, such vectors contain convenient restriction sites for insertion of the desired DNA segment. Exemplary vectors include pSVL and pKSV-10 (Pharmacia), pBPV-1, pml2d (International Biotechnologies), pTDT1 (ATCC 31255), retroviral expression vector pMIG and pLL3.7, adenovirus shuttle vector pDC315, and AAV vectors. Other exemplary vector systems are disclosed *e.g*., in U.S. Patent 6,413,777.

In general, screening large numbers of transformed cells for those which express suitably high levels of the antagonist is routine experimentation which can be carried out, for example, by robotic systems.

Frequently used regulatory sequences for mammalian host cell expression include viral elements that direct high levels of protein expression in mammalian cells, such as promoters and enhancers derived from retroviral LTRs, cytomegalovirus (CMV) (such as the CMV promoter/enhancer), Simian Virus 40 (SV40) (such as the SV40 promoter/enhancer), adenovirus, (*e.g.,* the adenovirus major late promoter (AdmlP)), polyoma and strong mammalian promoters such as native immunoglobulin and actin promoters. For further description of viral regulatory elements, and sequences thereof, *see, e.g.,* Stinski, U.S. Pat. No. 5,168,062; Bell, U.S. Pat. No. 4,510,245; and Schaffner, U.S. Pat. No. 4,968,615.

The recombinant expression vectors may carry sequences that regulate replication of the vector in host cells (*e.g.,* origins of replication) and selectable marker genes. The selectable marker gene facilitates selection of host cells into which the vector has been introduced (*see, e.g.,* Axel, U.S. Pat. Nos. 4,399,216; 4,634,665 and 5,179,017). For example, typically the selectable marker gene confers resistance to a drug, such as G418, hygromycin or methotrexate, on a host cell into which the vector has been introduced. Frequently used selectable marker genes include the dihydrofolate reductase (DHFR) gene (for use in dhfr- host cells with methotrexate selection/amplification) and the neo gene (for G418 selection).

Vectors encoding LINGO-1 antagonist or TrkB agonists can be used for transformation of a suitable host cell. Transformation can be by any suitable method. Methods for introduction of exogenous DNA into mammalian cells are well known in the art and include dextran-mediated transfection, calcium phosphate precipitation, polybrene-mediated transfection, protoplast fusion, electroporation, transfection via encapsulation of the polynucleotide(s) in liposomes, and direct microinjection of the DNA into nuclei. In addition, nucleic acid molecules may be introduced into mammalian cells by viral vectors. Mammalian cells may also be transduced by recombinant viruses containing the exogenous DNA which is to be introduced into the mammalian cells.

Host cells for expression of a LINGO-1 antagonist or TrkB agonist for use in the invention may be prokaryotic or eukaryotic. Exemplary eukaryotic host cells include, but are not limited to, yeast and mammalian cells, *e.g*., Chinese hamster ovary (CHO) cells (ATCC Accession No. CCL61), NIH Swiss mouse embryo cells NIH-3T3 (ATCC Accession No. CRL1658), and baby hamster kidney cells (BHK). Other useful eukaryotic host cells include insect cells and plant cells. Exemplary prokaryotic host cells are E. coli and Streptomyces.

Transformation of host cells can be accomplished by conventional methods suited to the vector and host cell employed. For transformation of prokaryotic host cells, electroporation and salt treatment methods can be employed (Cohen et al., Proc. Natl. Acad. Sci. USA 69:2110-14 (1972)). For transformation of vertebrate cells, electroporation, cationic lipid or salt treatment methods can be employed. *See, e.g.,* Graham et al., Virology 52:456-467 (1973); Wigler et al., Proc. Natl. Acad. Sci. USA 76:1373-76 (1979).

In certain embodiments, the host cell line used for protein expression can be of mammalian origin; those skilled in the art are credited with ability to determine particular host cell lines which are best suited for the desired gene product to be expressed therein. Exemplary mammalian host cell lines include, but are not limited to, NSO, SP2 cells, baby hamster kidney (BHK) cells, monkey kidney cells (COS), human hepatocellular carcinoma cells (*e.g.,* Hep G2), A549 cells DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3x63-Ag3.653 (mouse myeloma), BFA-1c1BPT (bovine endothelial cells), RAJI (human lymphocyte) and 293 (human kidney). Host cells for expression of a LINGO-1 antagonist or TrkB agonist for use in the invention may also be prokaryotic. Exemplary prokaryotic host cells are *E. coli* and *Streptomyces.* Host cell lines are typically available from commercial services, the American Tissue Culture Collection or from published literature.

Expression of polypeptides from production cell lines can be enhanced using known techniques. For example, the glutamine synthetase (GS) system is commonly used for enhancing expression under certain conditions. *See, e.g.,* European Patent Nos. 0 216 846, 0 256 055, and 0 323 997 and European Patent Application No. 89303964.4.

### Gene Therapy

A LINGO-1 and certain TrkB agonist (*e.g.* polynucleotide, polypeptide, antibodies and aptamers) can be produced *in vivo* in a mammal, *e.g.,* a human patient, using a gene-therapy approach to treatment of a disease, disorder or injury associated with neuronal degeneration, death or lack of regeneration. This involves administration of a suitable LINGO-1 antagonist and/or TrkB agonist-encoding nucleic acid operably linked to suitable expression control sequences. Generally, these sequences are incorporated into a viral vector. Suitable viral vectors for such gene therapy include an an adenoviral vector, an alphavirus vector, an enterovirus vector, a pestivirus vector, a lentiviral vector, a baculoviral vector, a herpesvirus vector (*e.g*. an Epstein Barr viral vector, or a herpes simplex viral vector) a papovaviral vector, a poxvirus vector (*e.g*. a vaccinia viral vector) and a parvovirus. The viral vector can be a replication-defective viral vector. Adenoviral vectors that have a deletion in their E1 gene or E3 genes are typically used. When an adenoviral vector is used, the vector usually does not have a selectable marker gene.

### Pharmaceutical Compositions and Administrative Methods.

The LINGO-1 antagonist or TrkB agonists used in the invention may be formulated into pharmaceutical compositions for administration to mammals, including humans. The pharmaceutical compositions used in the invention comprise pharmaceutically acceptable carriers, including, *e.g.,* ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions used in the present invention may be administered by any suitable method, *e.g*., parenterally, intraventricularly, orally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. As described previously, LINGO-1 antagonist or TrkB agonists used in the invention act in the nervous system to promote survival of neurons. Accordingly, in the invention, the LINGO-1 antagonist or TrkB agonists are administered in such a way that they cross the blood-brain barrier. This crossing can result from the physico-chemical properties inherent in the LINGO-1 antagonist or TrkB agonist molecule itself, from other components in a pharmaceutical formulation, or from the use of a mechanical device such as a needle, cannula or surgical instruments to breach the blood-brain barrier. Where the LINGO-1 antagonist or TrkB agonist is a molecule that does not inherently cross the blood-brain barrier, *e.g*., a fusion to a moiety that facilitates the crossing, suitable routes of administration are, *e.g.,* intrathecal or intracranial, *e.g.,* directly into a chronic lesion of MS. Where the LINGO-1 antagonist or TrkB agonist is a molecule that inherently crosses the blood-brain barrier, the route of administration may be by one or more of the various routes described below.

Sterile injectable forms of the compositions used in the invention may be aqueous or oleaginous suspension. These suspensions may be formulated according to techniques known in the art using suitable dispersing or wetting agents and suspending agents. The sterile, injectable preparation may also be a sterile, injectable solution or suspension in a non-toxic parenterally acceptable diluent or solvent, for example as a suspension in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Parenteral formulations may be a single bolus dose, an infusion or a loading bolus dose followed with a maintenance dose. These compositions may be administered at specific fixed or variable intervals, *e.g*., once a day, or on an "as needed" basis.

Certain pharmaceutical compositions used in this invention may be orally administered in an acceptable dosage form including, *e.g*., capsules, tablets, aqueous suspensions or solutions. Certain pharmaceutical compositions also may be administered by nasal aerosol or inhalation. Such compositions may be prepared as solutions in saline, employing benzyl alcohol or other suitable preservatives, absorption promoters to enhance bioavailability, and/or other conventional solubilizing or dispersing agents.

The amount of a LINGO-1 antagonist and/or TrkB agonist that may be combined with the carrier materials to produce a single dosage form will vary depending upon the host treated, the type of antagonist used and the particular mode of administration. The composition may be administered as a single dose, multiple doses or over an established period of time in an infusion. Dosage regimens also may be adjusted to provide the optimum desired response (*e.g*., a therapeutic or prophylactic response).

The invention uses a "therapeutically effective amount" or a "prophylactically effective amount" of a LINGO-1 antagonist or TrkB agonist. Such a therapeutically or prophylactically effective amount may vary according to factors such as the disease state, age, sex, and weight of the individual. A therapeutically or prophylactically effective amount is also one in which any toxic or detrimental effects are outweighed by the therapeutically beneficial effects.

A specific dosage and treatment regimen for any particular patient will depend upon a variety of factors, including the particular LINGO-1 antagonist or TrkB agonist used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

The LINGO-1 antagonist or TrkB agonists are generally administered directly to the nervous system, intracerebroventricularly, or intrathecally, *e.g.* into a chronic lesion of MS. Compositions for administration according to the invention can be formulated so that a dosage of 0.001- 10 mg/kg body weight per day of the LINGO-1 antagonist polypeptide is administered. In some embodiments of the invention, the dosage is 0.01 - 1.0 mg/kg body weight per day. In some embodiments, the dosage is 0.001 - 0.5 mg/kg body weight per day.

For treatment with a LINGO-1 antagonist or TrkB agonist antibody, the dosage can range, *e.g*., from about 0.0001 to 100 mg/kg, and more usually 0.01 to 5 mg/kg (*e.g.,* 0.02 mg/kg, 0.25 mg/kg, 0.5 mg/kg, 0.75 mg/kg, 1mg/kg, 2 mg/kg, etc.), of the host body weight. For example, dosages can be 1 mg/kg body weight or 10 mg/kg body weight or within the range of 1-10 mg/kg, optionally at least 1 mg/kg. Doses intermediate in the above ranges are also intended to be within the scope of the invention. Subjects can be administered such doses daily, on alternative days, weekly or according to any other schedule determined by empirical analysis. An exemplary treatment entails administration in multiple dosages over a prolonged period, for example, of at least six months. Additional exemplary treatment regimes entail administration once every two weeks or once a month or once every 3 to 6 months. Exemplary dosage schedules include 1-10 mg/kg or 15 mg/kg on consecutive days, 30 mg/kg on alternate days or 60 mg/kg weekly. In some methods, two or more monoclonal antibodies with different binding specificities are administered simultaneously, in which case the dosage of each antibody administered falls within the ranges indicated.

In certain embodiments, a subject can be treated with a nucleic acid molecule encoding a LINGO-1 antagonist or TrkB agonist polynucleotide. Doses for nucleic acids range from about 10 ng to 1 g, 100 ng to 100 mg, 1 µg to 10 mg, or 30-300 µg DNA per patient. Doses for infectious viral vectors vary from 10-100, or more, virions per dose.

In certain embodiments, LINGO-1 antagonists may be administered in an amount effective to block interaction of LINGO-1 and TrkB by at least 5%, 10%, 20%, 25%, 30%, 40%, 50%, 60%, 70%, 75%, 80%, 85%, 90%, 95% or 100% as measured by a competition assay or immunoprecipitation assay as compared to the interaction of LINGO-1 and TrkB in the absence of LINGO-1 antagonists.

In certain embodiments, LINGO-1 antagonists may be administered in an amount effective to promote phosphorylation of TrkB by at least 5%, 10%, 20%, 25%, 30%, 40%, 50%, 60%, 70%, 75%, 80%, 85%, 90%, 95% or 100% as compared to the amount of phosphorylated TrkB in the absence of LINGO-1 antagonists.

In certain embodiments, LINGO-1 antagonists may be administered in an amount effective to decrease JNK phosphorylation by 5%, 10%, 20%, 25%, 30%, 40%, 50%, 60%, 70%, 75%, 80%, 85%, 90%, 95% or 100% as compared to the amount of phosphorylated JNK in the absence of LINGO-1 antagonists.

In certain embodiments, TrkB agonists and LINGO-1 antagonists may be administered in an amount effective to promote survival of a CNS neuron by an increase in the number of surviving neurons of at least 5%, 10%, 20%, 25%, 30%, 40%, 50%, 60%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, or 1000% as compared to the number of surviving neurons in an untreated CNS neuron or mammal.

Supplementary active compounds also can be incorporated into the compositions used in the invention. For example, a soluble LINGO-1 or TrkB-agonist polypeptide or a fusion protein may be coformulated with and/or coadministered with one or more additional therapeutic agents.

The invention encompasses any suitable delivery method for a LINGO-1 antagonist or TrkB agonist to a selected target tissue, including bolus injection of an aqueous solution or implantation of a controlled-release system. Use of a controlled-release implant reduces the need for repeat injections.

The LINGO-1 antagonist or TrkB agonist used in the invention may be directly infused into the brain. Various implants for direct brain infusion of compounds are known and are effective in the delivery of therapeutic compounds to human patients suffering from neurological disorders. These include chronic infusion into the brain using a pump, stereotactically implanted, temporary interstitial catheters, permanent intracranial catheter implants, and surgically implanted biodegradable implants. *See, e.g.,* Gill *et al., supra*; Scharfen et al., "High Activity Iodine-125 Interstitial Implant For Gliomas," Int. J. Radiation Oncology Biol. Phys. 24(4):583-591 (1992); Gaspar et al., "Permanent 125I Implants for Recurrent Malignant Gliomas," Int. J. Radiation Oncology Biol. Phys. 43(5):977-982 (1999); chapter 66, pages 577-580, Bellezza et al., "Stereotactic Interstitial Brachytherapy," in Gildenberg et al., Textbook of Stereotactic and Functional Neurosurgery, McGraw-Hill (1998); and Brem et al., "The Safety of Interstitial Chemotherapy with BCNU-Loaded Polymer Followed by Radiation Therapy in the Treatment of Newly Diagnosed Malignant Gliomas: Phase I Trial," J. Neuro-Oncology 26:111-23 (1995).

The compositions may also comprise a LINGO-1 antagonist or TrkB agonist dispersed in a biocompatible carrier material that functions as a suitable delivery or support system for the compounds. Suitable examples of sustained release carriers include semipermeable polymer matrices in the form of shaped articles such as suppositories or capsules. Implantable or microcapsular sustained release matrices include polylactides (U.S. Patent No. 3,773,319; EP 58,481), copolymers of L-glutamic acid and gamma-ethyl-L-glutamate (Sidman et al., Biopolymers 22:547-56 (1985)); poly(2-hydroxyethyl-methacrylate), ethylene vinyl acetate (Langer et al., J. Biomed. Mater. Res. 15:167-277 (1981); Langer, Chem. Tech. 12:98-105 (1982)) or poly-D-(-)-3hydroxybutyric acid (EP 133,988).

In some embodiments of the invention, a LINGO-1 antagonist or TrkB agonist is administered to a patient by direct infusion into an appropriate region of the brain. *See, e.g.,* Gill et al., "Direct brain infusion of glial cell line-derived neurotrophic factor in Parkinson disease," Nature Med. 9:589-95 (2003). Alternative techniques are available and may be applied to administer a LINGO-1 antagonist according to the invention. For example, stereotactic placement of a catheter or implant can be accomplished using the Riechert-Mundinger unit and the ZD (Zamorano-Dujovny) multipurpose localizing unit. A contrast-enhanced computerized tomography (CT) scan, injecting 120 ml of omnipaque, 350 mg iodine/ml, with 2 mm slice thickness can allow three-dimensional multiplanar treatment planning (STP, Fischer, Freiburg, Germany). This equipment permits planning on the basis of magnetic resonance imaging studies, merging the CT and MRI target information for clear target confirmation.

The Leksell stereotactic system (Downs Surgical, Inc., Decatur, GA) modified for use with a GE CT scanner (General Electric Company, Milwaukee, WI) as well as the Brown-Roberts-Wells (BRW) stereotactic system (Radionics, Burlington, MA) can be used for this purpose. Thus, on the morning of the implant, the annular base ring of the BRW stereotactic frame can be attached to the patient's skull. Serial CT sections can be obtained at 3 mm intervals though the (target tissue) region with a graphite rod localizer frame clamped to the base plate. A computerized treatment planning program can be run on a VAX 11/780 computer (Digital Equipment Corporation, Maynard, Mass.) using CT coordinates of the graphite rod images to map between CT space and BRW space.

The methods of treatment of disorders as described herein are typically tested *in vitro,* and then *in vivo* in an acceptable animal model, for the desired therapeutic or prophylactic activity, prior to use in humans. Suitable animal models, including transgenic animals, are will known to those of ordinary skill in the art. For example, *in vitro* assays to demonstrate the survival effect of the LINGO-1 antagonist or TrkB agonists are described herein. Finally, *in vivo* tests can be performed by creating transgenic mice which express the LINGO-1 antagonist or TrkB agonist or by administering the LINGO-1 antagonist or TrkB agonist to mice or rats in models as described herein.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning: A Laboratory Manual (3-Volume Set), J. Sambrook, D. W. Russell, Cold Spring Harbor Laboratory Press (2001); Genes VIII, B. Lewin, Prentice Hall (2003); PCR Primer, C.W. Dieffenbach and G.S. Dveksler, CSHL Press (2003); DNA Cloning, D. N. Glover ed., Volumes I and II (1985); Oligonucleotide Synthesis: Methods and Applications (Methods in Molecular Biology), P. Herdewijn (Ed.), Humana Press (2004); Culture of Animal Cells: A Manual of Basic Technique, 4th edition, R. I. Freshney, Wiley-Liss (2000); Oligonucleotide Synthesis, M. J. Gait (Ed.), (1984); Mullis et al. U.S. Pat. No: 4,683,195; Nucleic Acid Hybridization, B. D. Hames & S. J. Higgins eds. (1984); Nucleic Acid Hybridization, M. L. M. Anderson, Springer (1999); Animal Cell Culture and Technology, 2nd edition, M. Butler, BIOS Scientific Publishers (2004); Immobilized Cells and Enzymes: A Practical Approach (Practical Approach Series), J. Woodward, Irl Pr (1992); Transcription And Translation, B. D. Hames & S. J. Higgins (Eds.) (1984); Culture Of Animal Cells, R. I. Freshney, Alan R. Liss, Inc., (1987); Immobilized Cells And Enzymes, IRL Press, (1986); A Practical Guide To Molecular Cloning, 3rd edition, B. Perbal, John Wiley & Sons Inc. (1988); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155, Wu et al. (Eds.); Immunochemical Methods In Cell And Molecular Biology, Mayer and Walker, (Eds.), Academic Press, London (1987); Handbook Of Experimental Immunology, Volumes I-IV, D. M. Weir and C. C. Blackwell (Eds.), (1986); Immunology Methods Manual: The Comprehensive Sourcebook of Techniques (4 Volume Set), 1st edition, I. Lefkovits, Academic Press (1997); Manipulating the Mouse Embryo: A Laboratory Manual, 3rd edition, Cold Spring Harbor Laboratory Press (2002); and in Ausubel et al., Current Protocols in Molecular Biology, John Wiley and Sons, Baltimore, Maryland (1989).

General principles of antibody engineering are set forth in Antibody Engineering: Methods and Protocols (Methods in Molecular Biology), B.L. Lo (Ed.), Humana Press (2003); Antibody engineering, R. Kontermann and S. Dubel (Eds.), Springer Verlag (2001); Antibody Engineering, 2nd edition, C.A.K. Borrebaeck (Ed.), Oxford Univ. Press (1995). General principles of protein engineering are set forth in Protein Engineering, A Practical Approach, Rickwood, D., et al. (Eds.), IRL Press at Oxford Univ. Press, Oxford, Eng. (1995). General principles of antibodies and antibody-hapten binding are set forth in: Antibodies: A Laboratory Manual, E. Harlow and D. Lane, Cold Spring Harbor Laboratory Press (1988); Nisonoff, A., Molecular Immunology, 2nd edition, Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (Eds.), Immunochemical Protocols (Methods in Molecular Biology), 2nd edition, J. D. Pound (Ed.), Humana Press (1998), Weir's Handbook of Experimental Immunology, 5th edition, D. M. Weir (Ed.), Blackwell Publishers (1996), Methods in Cellular Immunology, 2nd edition, R., Fernandez-Botran, CRC Press (2001); Basic and Clinical Immunology, 8th edition, Appleton & Lange, Norwalk, CT (1994) and Mishell and Shiigi (Eds.), Selected Methods in Cellular Immunology, W.H. Freeman and Co., New York (1980).

Standard reference works setting forth general principles of immunology include Current Protocols in Immunology, John Wiley & Sons, New York; Klein, J.; Kuby Immunology, 4th edition, R. A. Goldsby, et al., H. Freeman & Co. (2000); Basic and Clinical Immunology, M. Peakman, et al., Churchill Livingstone (1997); Immunology, 6th edition, I. Roitt, et al., Mosby, London (2001); Cellular and Molecular Immunology, 5th edition; A.K. Abbas, A.H. Lichtman, Elsevier - Health Sciences Division (2005); Immunology Methods Manual: The Comprehensive Sourcebook of Techniques (4 Volume Set), 1st edition, 1. Lefkovits, Academic Press (1997) Immunology, 5th edition, R.A. Goldsby, et al., W. H. Freeman (2002); Monoclonal Antibodies : Principles and Practice, 3rd Edition , J.W. Goding, Academic Press (1996); Immunology: The Science of Self-Nonself Discrimination, John Wiley & Sons, New York (1982); Kennett, R., et al. (Eds.), Monoclonal Antibodies, Hybridoma: A New Dimension in Biological Analyses, Plenum Press, New York (1980); Campbell, A., "Monoclonal Antibody Technology" in Burden, R, et al. (Eds.), Laboratory Techniques in Biochemistry and Molecular Biology, Vol. 13, Elsevere, Amsterdam (1984).

### EXAMPLES

### MATERIALS AND METHODS

### Generation of Recombinant LINGO-1-Fc and anti-LINGO-1 Monoclonal Antibody

LINGO-1-Fc (LINGO-1-Fc protein) was prepared as described previously (Mi et al., Nat. Neurosci. 7:221-228 (2004)). Residues 1-532 of human LINGO-1 were fused to the hinge and Fc region of human IgG1 and expressed in CHO cells. Human IgG1 (control protein) was purchased from Protos Immunoresearch (San Francisco, CA). The anti-LINGO-1 mab 1A7 was generated in mice immunized with LINGO-1-Fc. The hybridoma cell line was grown in DMEM and the antibody was purified by Protein A Sepharose. MOPC21 Mouse IgG (control protein) was purchased from Protos Immunoresearch (San Francisco, CA).

### Ocular Hypertension Model

Experiments were carried out according to the National Institutes of Health Guide for the Care and Use of Laboratory Animals (NIH Publications No. 80-23) revised in 1996 and approved by the University of Hong Kong Animal Ethics Committee. Adult female Sprague-Dawley (SD) rats weighing approximately 250 g were used. They were housed 3 per standard laboratory cage and maintained on food and water ad libitum with a 12-h dark/light cycle (7:00 a.m./7:00 p.m.). All operations were carried out in animals anesthetized with intraperitoneal injection of ketamine (80 mg/kg) and xylazine (8 mg/kg). 0.5% alcaine (Alcon-Couvreur, Belgium) was applied to the eyes before all operations and antiseptic eye drops (Tobres [Tobramycin 0.3%], Alcon-Couvreur, Belgium) were used to prevent infection after the treatment. Rimadyl (0.025mg/ml) in drinking water was used to relieve the pain for 7 days after the surgeries.

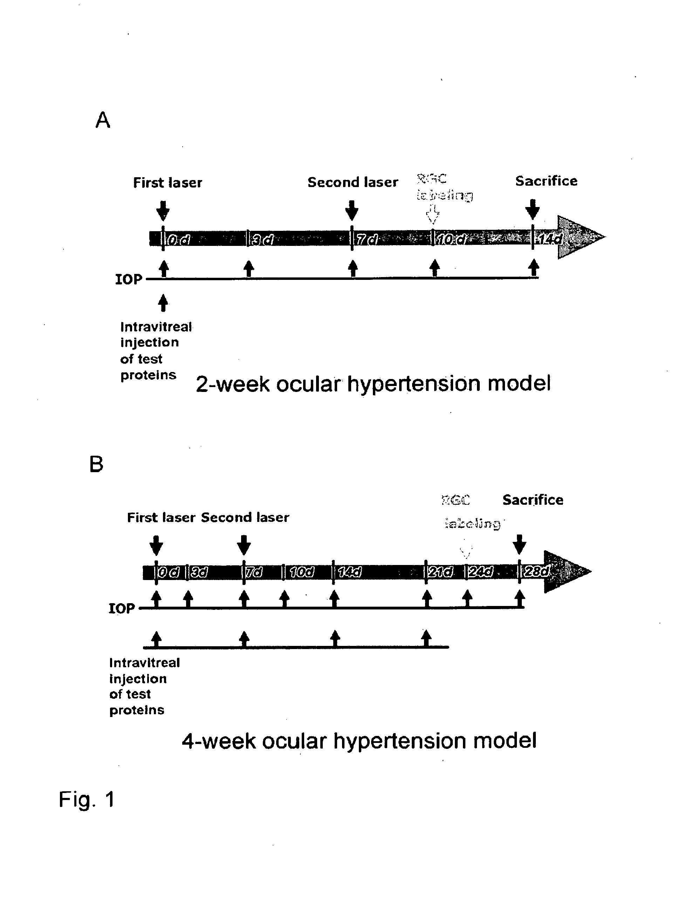

Experimental glaucoma was induced using a chronic hypertension model. SD rats received argon laser photocoagulation of the episcleral and limbal veins in the right eye at a power of 1000 mW, a spot size of 500-100 µm and a duration of 0.1 (Ji et al., Eur. J. Neurosci. 19:265-272 (2004); WoldeMussie et al., Invest. Ophthalmol. 42:2849-2855 (2001)). About 90 spots were applied on the three episcleral veins and 70 spots around the limbal vein. A secondary laser surgery was delivered to block the reconnected vascular flow seven days later. The animals' left eyes were used as a contralateral control and were not operated on. Animals were allowed to survive for 2 or 4 weeks post first laser exposure before they were sacrificed. The IOP of right and left eyes were measured using a Tonopen XL Tonometer at different time points after laser operation. An average of ten measurements was obtained for each eye. FG labeling of RGCs was performed four days before sacrifice. Both superior colliculi (SC) were exposed after removing a small piece of skull and cortex, and a piece of Gelfoam (Pharmacia & Upjohn) soaked with FG (6% v/v, Fluorochrome, Denver, CO) was placed on the surface of the SC. FG retrogradely labeled intact RGCs. Twelve animals were treated with PBS and allowed to survive 4 weeks post first laser exposure. In all other experimental groups, ten animals were used. The procedure of glaucoma model is summarized in Figure 1.

Following the first laser treatment, animals immediately received an intravitreal injection of 2 µg LINGO-1-Fc, 2 µg 1A7 or 2 µg control protein in PBS. In the 4-week glaucoma model, the proteins were re-injected once a week. Treatments were masked to avoid bias of investigators during counting of RGCs. All animals were euthanized with an overdose of anesthesia.

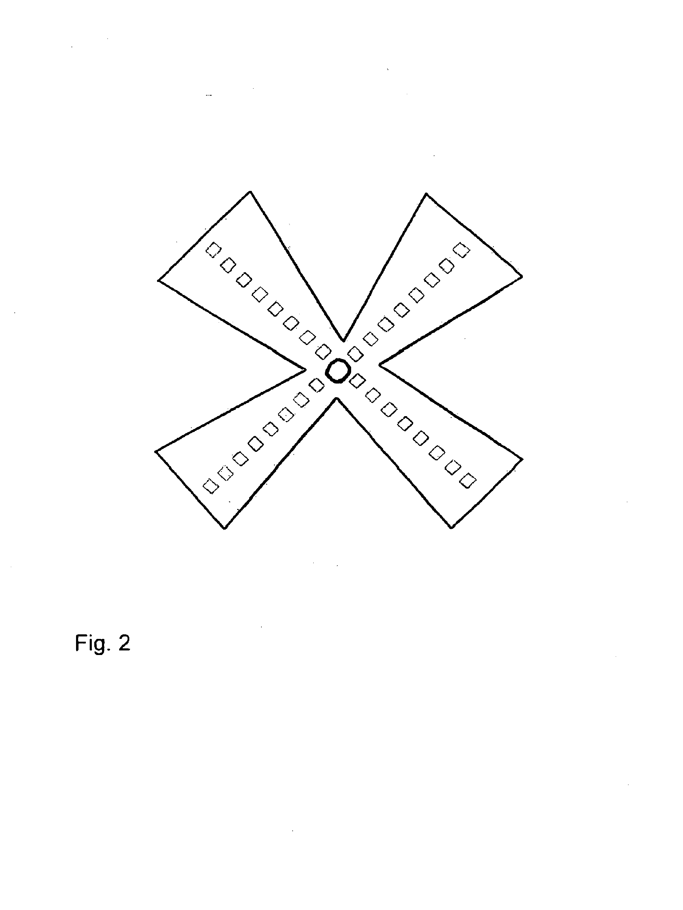

At predefined times, rats were sacrificed with an overdose of anesthesia. Both eyes of each animal were enucleated and fixed in 4% paraformaldehyde for 60 minutes. Retinas were prepared as flat-mounts and the FG labeled RGCs were counted under fluorescence microscopy using an ultra-violet filter (excitation wave length = 330-380 nm) as described (Cheung et al., Mol. Cell. Neurosci. 25:383-393 (2004); Ji et al., Eur. J. Neurosci. 19:265-272 (2004)). The RGCs were quantified under an eyepiece grid of 200 × 200 µm² along the median line of each quadrant, starting from the optic disc to the border at 500 µm intervals (Figure 2). Eight microscopic fields for each quadrant and a total of 32 per retina for four quadrants were counted, corresponding to approximately 3-3.2% of each retinal area. Percent loss of RGCs was measured to examine the survival effects of different treatments. The data was expressed in terms of relative percentage of RGC loss in the injured eye compared to the contralateral intact eye (% contralateral, mean + sem).

### Tissue Processing for Transmission Electron Microscopy

Two mm segments of the retinas were obtained from normal and 2 week-glaucoma groups treated with PBS, soluble LINGO-1 or 1A7. They were placed in Karnovsky electron microscope (EM) fixative for 2-4 hours at 4°C. After washing in 0.1M phosphate buffer (PB), tissue samples were post-fixed in 1% osmium tetroxide in 0.1M PB, then dehydrated in ethanol and embedded in Epon. Semithin (1µm) sections were obtained from each of the blocks using a Reichert-Jung ultramicrotome with glass knives made on a LKB knife-maker. The sections were stained with toluidine blue. Ultrathin sections with silver interference color were obtained, stained with Reynolds lead citrate and uranyl acetate and examined with electron microscope.

### Primary RGC Culture

The eyes of P7 Long-Evans rats were removed and placed in dissociation medium (DM; 90 mM Na₂SO₄, 30 mM K₂SO₄, 5.8 mM MgCl₂, 0.25 mM CaCl₂, 1 mM HEPES, 0.001% Phenol red) (Furshpan and Potter, 1989). Retinas were removed from the eyes and incubated in 2 ml DM containing 15 U/ml Papain (Worthington, NJ), 1 mM L-cysteine, 0.5 mM EDTA, 0.005% DNase I for 30 min at 37°C. Retinas were rinsed twice in DM, resuspended in 2 ml DM containing 10 mg/ml ovomucoid protease inhibitor, 10 mg/ml Bovine Serum Albumin (both from Worthington) and gently triturated 10-15 times using a 2 ml serological pipette. Dissociated cells were pelleted at 1000 rpm for 5 min, resuspended in 2 ml of growth medium modified from Meyer-Franke *et al*. (Meyer-Franke et al. Neuron 15:805-819 (1995)) (Neurobasal, 1x B27 from Invitrogen, 5µM Forskolin, 60 nM T3, 1mM Pyruvate, 2 mM Glutamine) and counted with a hemocytometer. Cells were plated at a density of 50,000 cells/cm² in BD Biocoat 96-well tissue culture plates coated with poly-D-Lysine and Laminin (BD Biosciences, Bedford, MA) and grown for 3 days in a CO₂ incubator. At the time of plating, cells were treated in quadruplicate wells with human IgG1 (10 µg/ml), LINGO-1-Fc (10µg/ml) alone, human IgG1 and 25 ng/ml BDNF, or LINGO-1 Fc and BDNF.

Cells were fixed 15 min in methanol at -20°C, rinsed twice in PBS and blocked for 1 h in 5% normal goat serum in PBS for 1 h. Cells were incubated in Thy1.1 antibody (Serotec, clone OX-7, 1:40 in PBS) overnight at 4°C, rinsed 3 times for 5 min in PBS, incubated in goat anti-mouse IgG-conjugated with Alexa 594 (Molecular Probes, Eugene, OR; 1:1000) for 1 h and rinsed 3 times for 5 min in PBS.

RGCs were identified under epifluorescence microscopy using a Zeiss axiovert inverted microscope. The entire surface of each well was visually scanned under epifluorescence to count surviving RGCs. Only Thy 1.1 positive cells with neuronal morphology and bearing at least one process with a minimum length 3 times the cell body diameter were counted.

### Immunohistochemistry for LINGO-1 and p-TrkB

RGCs were retrogradely labeled with FG at 4 days before sacrifice. The eyes were enucleated at 2 weeks after injury following transcardial perfusion with 0.9% saline and subsequently post-fixed in 4% PFA for 4 h. Ten-micron-thick frozen sections were incubated with mouse anti-LINGO-1 (Biogen), rabbit anti-phosphor-TrkB (Tyr785) (a gift from Dr. B. Sun, Shanghai Institutes of Biological Sciences, Shanghai, China) antibodies (Ji et al., Nat. Neurosci. 8:164-172 (2005)), following by treatment with Alexa-labeled secondary antibody. After washing, the sections were mounted with fluorescent mounting medium (DakoCytomation) and analyzed under Carl Zeiss LSM 510 META Confocal microscopy.

### Western Blotting

To measure LINGO-1, BDNF or p-TrkB in the retina, the animals were euthanized at 2 weeks after laser coagulation. For assessing the temporal profile of Akt phosphorylation, animals treated with LINGO-1-Fc or PBS were euthanized at 6 hr, 1 day and 5 days after laser coagulation. To measure the effects of injury, BDNF, and LINGO-Fc or 1A7 on TrkB phosphorylation, we injected BDNF intravitreally (5 µg/eye, recombinant human BDNF; Regeneron Pharmaceutical, Tarrytown, NY) or BDNF combined with LINGO-1-Fc or 1A7 (2 µg/eye) at the same time of laser coagulation and then euthanized the animals 5 days later. Retinas were dissected and homogenized in lysis buffer (10 mM Tris pH7.4, 150 mM NaCl, 1 mM EDTA, 1 mM EGTA) supplemented with 10% protease inhibitor cocktail and 1% phosphatase inhibitor cocktails from Sigma. Following centrifugation at 13,000 rpm for 30 minutes to remove cell debris, the protein concentration of the supernatant was measured using a Bio-Rad DC protein Assay Kit (Bio-Rad Laboratories, CA, USA). A 40-80 µg aliquot of proteins from individual animals was subjected to 6-12.5% SDS-polyacrylamide gel electrophoresis and transferred onto PVDF membrane. The membranes were blocked with 5% non fat dry milk and 2% bovine serum albumin (BSA) in Tris-buffered saline containing 0.1% Tween 20 (TBST) for 1 h at room temperature. Incubations with mouse anti-LINGO-1 (Biogen), rabbit anti-BDNF (Chemicon), mouse anti-phosphor-Akt (1;1000), rabbit total Akt (1;1000), anti-phospho-JNK (1:1000), total-JNK (Cell Signaling Technology), rabbit anti-phosphor-TrkB (Tyr785) (1:1000) (a gift from Dr. B. Sun, Shanghai Institutes of Biological Sciences, Shanghai, China) (Ji et al., Nat. Neurosci. 8:164-172 (2005)) and chicken IgY total TrkB (Promega) (1:100) antibodies were performed overnight at 4°C. After washing, the membranes were incubated with Horseradish Peroxidase-conjugated secondary antibody in 5% non fat dry milk and 2% BSA in TBST for 1 h at room temperature. Immunoreactive proteins were detected using the enhanced chemiluminescence method (ECL, Amersham). Protein loading was controlled using a monoclonal goat antibody against actin (1:1000, C-11, Santa Cruz Biotechnology). The intensity of each band was quantified by densitometric scanning using Labworks gel documentation (UVP, Inc, Upland, CA). All experiments for Western blotting were performed with 3-5 animals in each group and the samples were run on the gels as individual animals. Protein levels were finally expressed as relative values compared to total proteins of normal retinas.

### Immunoprecipitations and Western Blotting for LINGO-1 and TrkB

293T cells (100 mm dishes) were transfected with HA-tagged full length human LINGO-1, myc-tagged full length human TrkB, or a combination of LINGO-1/TrkB. The cells were harvested after 48 h and lysed in 1 ml RIPA buffer (50 mM Tris, pH 7.2, 1% Triton X-100, 0.5% sodium deoxycholate, 0.1% SDS, 150 mM NaCl, 10 mM MgCl₂, 5% glycerol) for 30 min at 4°C. After centrifugation at 14,000 x g for 15 min, the supernatants were incubated with ProteinA/G plus-Sepharose beads (Santa Cruz Biotechnology, CA) at 4°C for 1 hr. The pre-cleared lysates were then incubated with an anti-LINGO-1 antibody (Biogen Idec) at 4°C for 1 hr followed by addition of Protein A/G-Sepharose beads for 1 hr. The beads were washed 3 times with 1% Triton buffer (50 mM HEPES, pH 7.5, 150 mM NaCl, 1.5 mM MgCl₂, 1mM EGTA, 1% Triton X-100 and 10% glycerol), boiled in Laemmli sample buffer, subjected to 4-20% SDS-PAGE and analyzed by Western blotting with anti-TrkB antibody (Myc, Roche) or anti-LINGO-1 antibody (HA, Roche). Retinal lysates from normal or 2-week ocular hypertensive rats were also immunoprecipitated with anti-TrkB antibody (Chemicon), an anti-LINGO-1 antibody (Upstate), or a non-specific antibody at 4°C overnight and analyzed by Western blotting with an anti-LINGO-1 antibody (Upstate).

Neuroscreen-1 cells (a subclonal line of PC12 cells, Cellomics) or Neuroscreen cells with over-expressed stable HA-LINGO1 were infected with TrkB lentiviruses for 2 days. The cells were serum-starved overnight before they were subjected to BDNF for 0 or 30 min in serum-free media. The cell lysates were immunoprecipitated by a pan-Trk antibody (Santa Cruz, sc-139), and then assessed by Western blotting using either an anti-Phospho-Tyr antibodies for Phospho-TrkB or an anti-TrkB antibody for total TrkB (Santa Cruz). The cell lysates were also assessed by Western blotting for LINGO-1 expression (HA, Roche).

### Statistics

Statistical analysis was performed using Student's t test for comparisons between two groups, or by one-way analysis of variance (ANOVA) followed by post-hoc tests (Student-Neuman-Keuls) for comparisons of more than two groups.

### EXAMPLE 1

### Increased Expression of LINGO-1 in a Rat Glaucoma Model

The expression of LINGO-1 was examined in a rat glaucoma model. In this model, an argon laser was used to block the outflow of aqueous humor by photocoagulation of the limbal and episcleral drainage vessels, resulting in reliable increase of intraocular pressure (IOP) (*See* Figure 1).

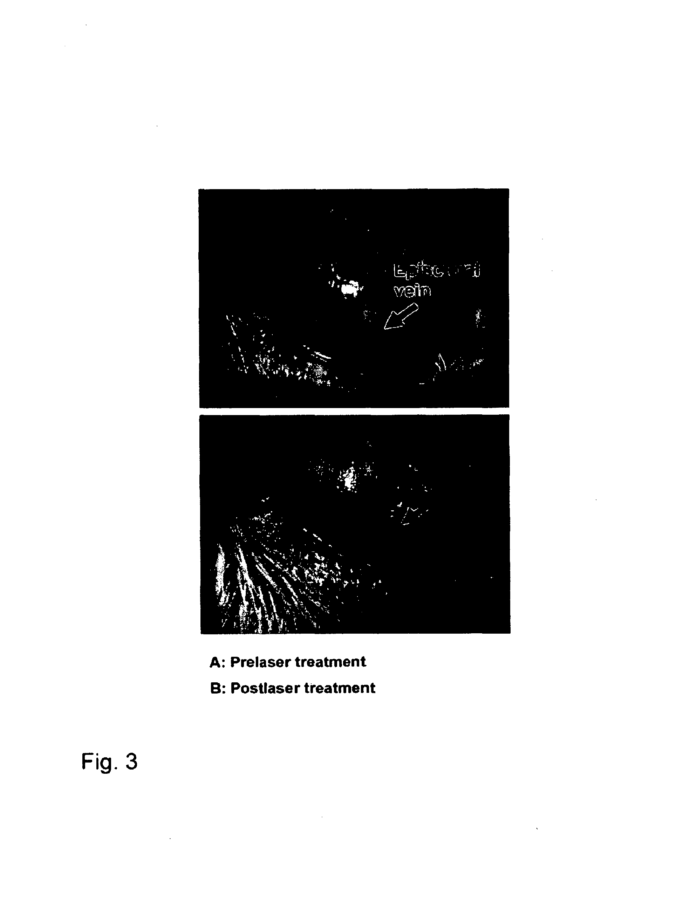

To generate a ocular hypertensive state, the limba1 and three episcleral veins were photocoagulated twice at a 7-day interval using an Argon laser. Examination of the aqueous veins prior to and immediately after the laser photocoagulation revealed a marked decrease in venous blood flow, as shown in Figure 3.

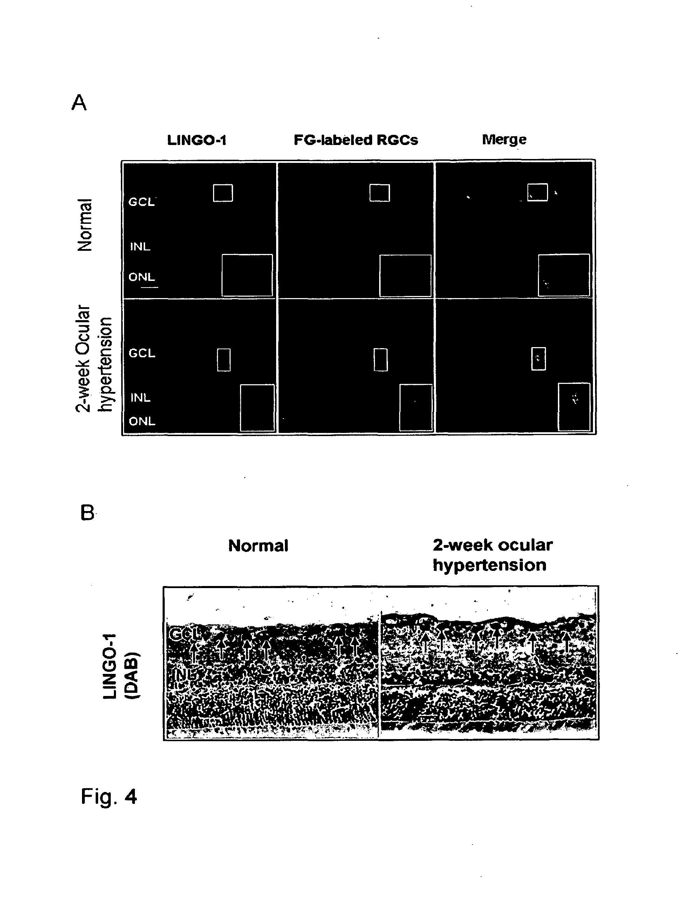

LINGO-1 expression was examined in normal and injured rat retina sections. Normal retinal ganglion cells (RGCs) expressed low level staining for LINGO-1, but much stronger immunoreactivity for LINGO-1 occurred in RGCs at 2 weeks after laser coagulation. The number of labeled RGCs and the intensity of labeling both increased (See Figure 4). (The term "after laser coagulation" means "after first laser coagulation".)

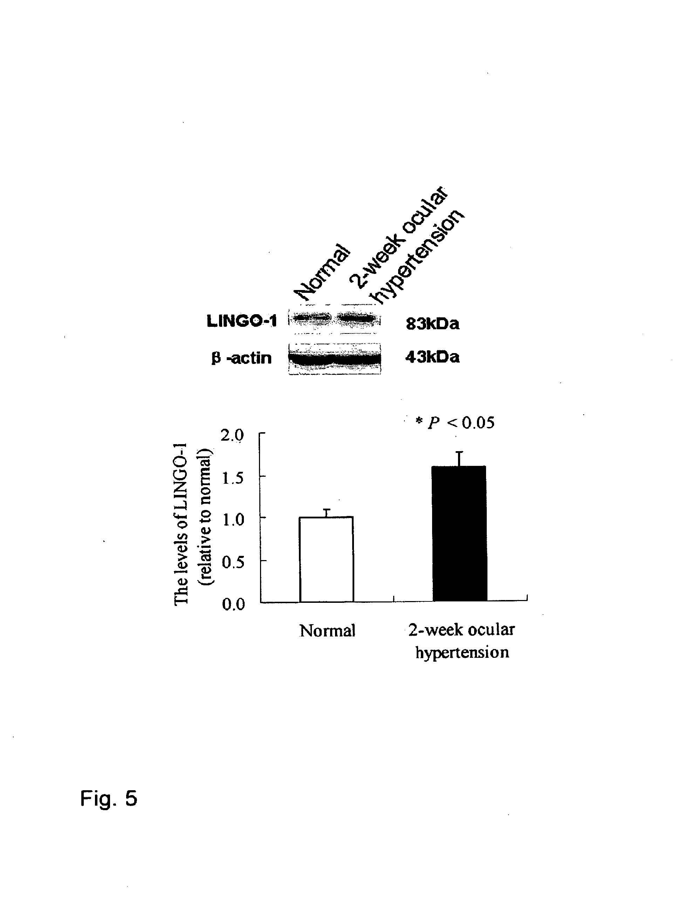

These finding were confirmed by western blotting, which showed that LINGO-1 expression was low in normal retina and increased to 1.6 fold at 2 weeks after the injury (P < 0.05) (See Figure 5).

### EXAMPLE 2

### LINGO-1 Antagonists Act as Neuroprotectants Without Affecting Intraocular Pressure

Experimental ocular hypertension can be monitored by measuring changes in pressure or neuronal survival. Therefore, the effect of LINGO-1 antagonists on both intraocular pressure and RGC survival was examined. Animals were subjected to laser treatment, as described *supra* and immediately afterwards received an intravitreal injection of LINGO-1-Fc, 1A7 or control protein.

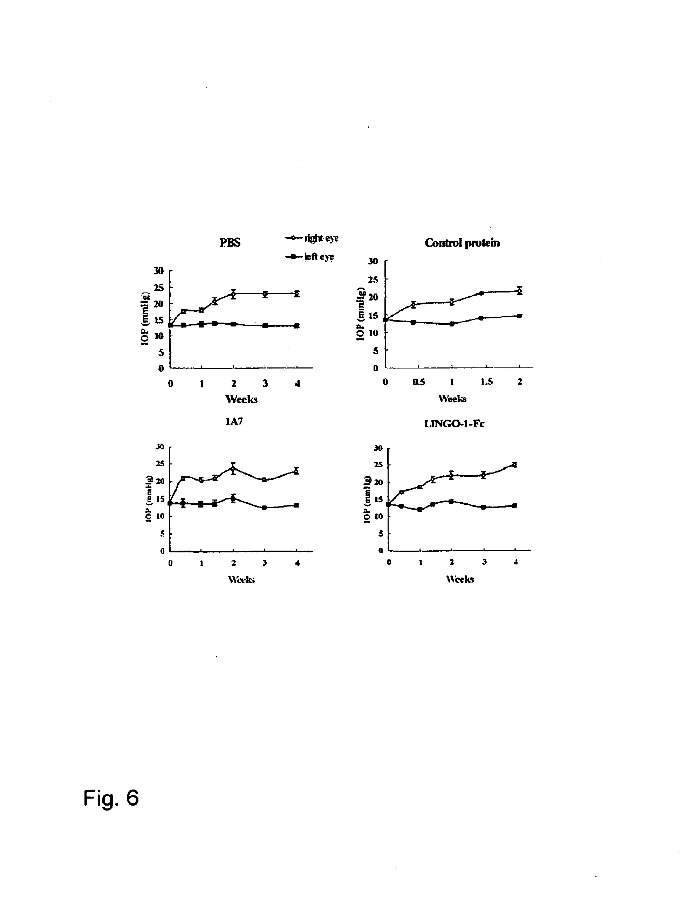

The intraocular pressure (IOP) in both treated and untreated eyes was monitored. IOP in the contralateral left eye of treated animals was about 13 mmHg (See Figure 6) and remained at the same level through the experiment. The IOP of the laser-treated right eye in all four groups increased after the first laser surgery, reached approximately 22 mmHg and remained at this level until sacrifice. Under these experimental conditions, treatment with LINGO-1-Fc and the neutralizing anti-LINGO-1 antibody, mAb 1A7, did not lower IOP (See Figure 6).

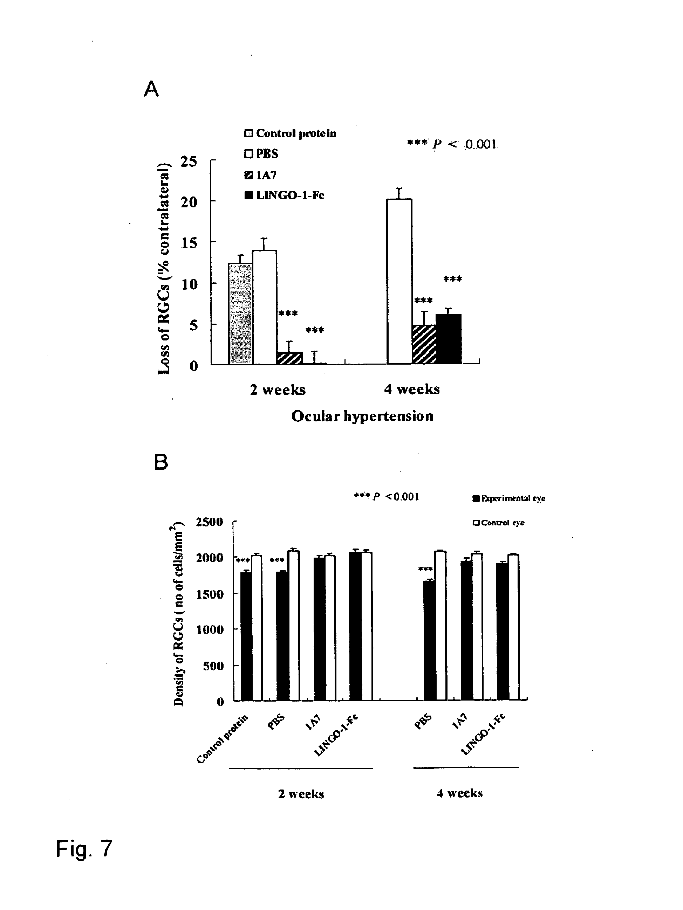

LINGO-1 antagonists did have an affect on neuronal survival. First, surviving RGCs were quantitated 2 weeks after laser injury. Retinas from both eyes were prepared and suviving RGCs were counted. RGC loss at 2 weeks after laser coagulation in PBS and control protein treatment groups was 13.93 ± 1.44% and 12.37 ± 1.84 % respectively (See Figure 7A). Injection of LINGO-1-Fc prevented RGC loss. LINGO-1-Fc treated retinas has only 0.09 ± 1.47 % RGC loss (P < 0.001, compared to PBS and human IgG control groups). Similarly, mAb 1A7 treatment limited RGC loss to 1.46 ± 1.32 % (P < 0.001 compared to the control groups).

To investigate the effect of LINGO-1 antagonists on the long-term survival of RGCs, LINGO-1-Fc and the neutralizing LINGO-1 antibody 1A7 were injected intravitreally once a week and the animals were allowed to survive for 4 weeks. The results (see Figure 7A) showed that treatment with LINGO-1-Fc antagonists significantly reduced loss of injured RGCs from 20.09 ± 1.36 % (PBS control) to 5.98 ± 0.83 % (P < 0.001) for LINGO-1-Fc and to 4.73 ± 1.72 % (P < 0.001) for 1A7 4 weeks after laser coagulation.

The data on RGC survival are also presented as the mean density (No. of cells/mm²) for each group (See Figure 7B). Previous investigations of the death of RGCs in our rat glaucoma model at 2, 4, 8 and 12 weeks after laser coagulation showed that the loss of RGCs reaches a maximal level after 4 weeks (Li et al., Invest. Ophthalmol. Vis. Sci. 47:2951-2958 (2006)). In contrast significant neuroprotection of LINGO-1-Fc and 1A7 was observed 4 weeks after laser coagulation. Unlike ciliary neurotrophic factor (CNTF) treatment (data not shown), neither LINGO-1-Fc or anti-LINGO-1 antibody caused cataracts in the long-term survival experiment.

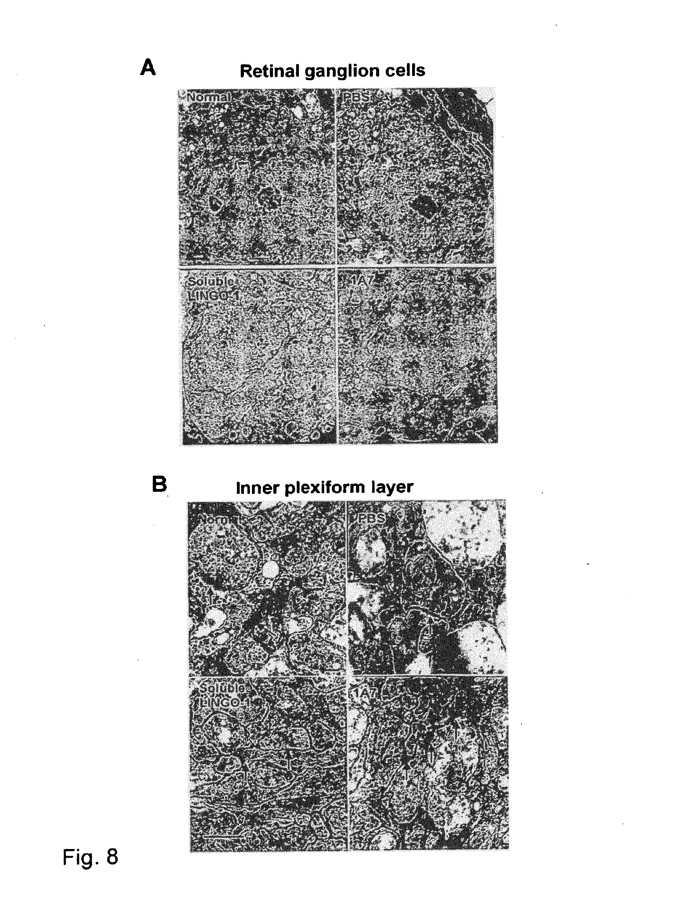

Electron microscopy was also used to evaluate the effects of LINGO-1 antagonists. As shown in Figure 8A, at two weeks after ocular hypertension, some of the PBS treated RGCs have an irregular or even cracked nucleus with swollen and melting mitochondrion. Some also show a loss of rough endoplasmic reticulum (ER) and Golgi organelles. In contrast, eyes treated with soluble LINGO-1 and 1A7 keep characteristics of typical retinal ganglion cells with normal ER and Golgi organelles. The organells of cells in the inner plexiform layer (IPL), where the RGCs, amarcine cells and bipolar cells make connections were also examined. Compared to the LINGO-1 or 1A7 treated animals, which maintained an almost normal axons and dentrites, the cells in the PBS treated animals had less organelles inside of more swollen axons and dendrites (See Figure 8B). In addition, the synapses in PBS-treated animals become narrow but still remain un-separated, while the retinas that had received treatment of LINGO-1-Fc or 1A7 maintained normal characteristics of synapses.

### EXAMPLE 3

### LINGO-1 Antagonists Alone Do Not Promote Survival of Retinal Ganglion Cells In Vitro

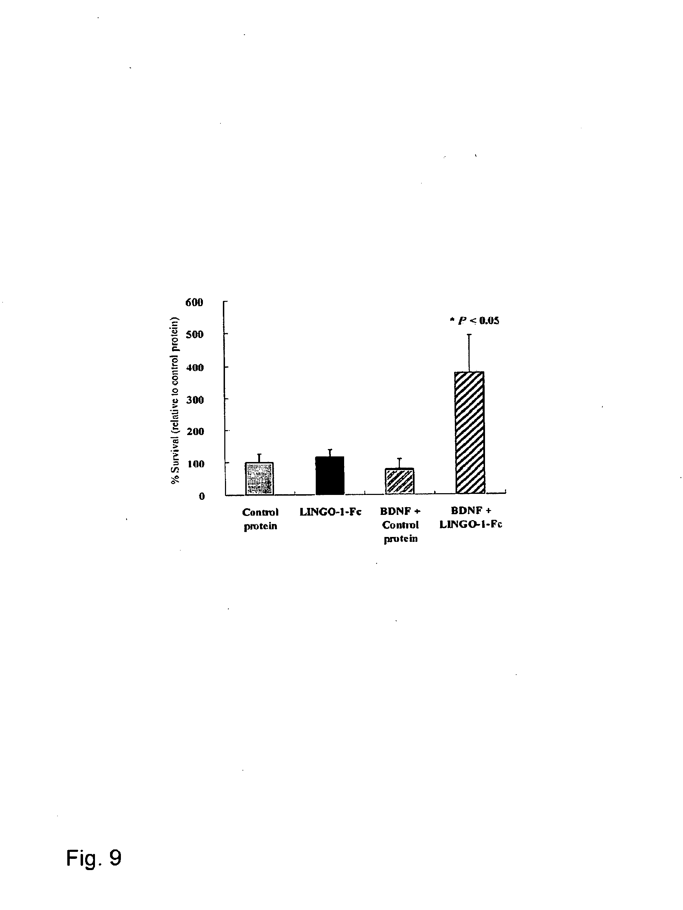

To study the effects of LINGO-1-Fc on RGC survival further, an RGC primary culture system was used (Meyer-Franke et al., Neuron, 15:805-819 (1995)). Dissociated retinal cultures were grown for 3 days in the presence of control protein or LINGO-1-Fc. Primary RGCs were identified by Thy 1.1 immunostaining, a marker for RGCs, and counted. Unlike the evident neuroprotective activity *in vivo*, under these experimental conditions, LINGO-1-Fc treatment alone did not promote survival of cultured RGCs (See Figure 9).

### EXAMPLE 4

### LINGO-1 Acts on the BDNF Pathway

The effect of LINGO-1 antagonists on the suvival of RGCs *in vitro* in the presence of brain-derived neurotrophic factor (BDNF) was also examined. In the presence of BDNF, LINGO-1 antagonists were able to promote survival of RCGs. RGCs were grown as in Example 3 and treated with either BDNF and control protein or BDNF and LINGO-1-Fc. In the presence of BDNF, a significantly greater percentage of cell survival in cells treated with LINGO-1-Fc than in cells treated with control protein. This result, in addition to the fact that *in vivo*, animal retinas are exposed to endogenous BDNF, while the RGC cultures did not have any BDNF, demonstrates that LINGO-1-Fc rescues RGCs by modulating their response to BDNF, and/or that LINGO-1-Fc indirectly increases survival by enhancing neurotrophin receptors.

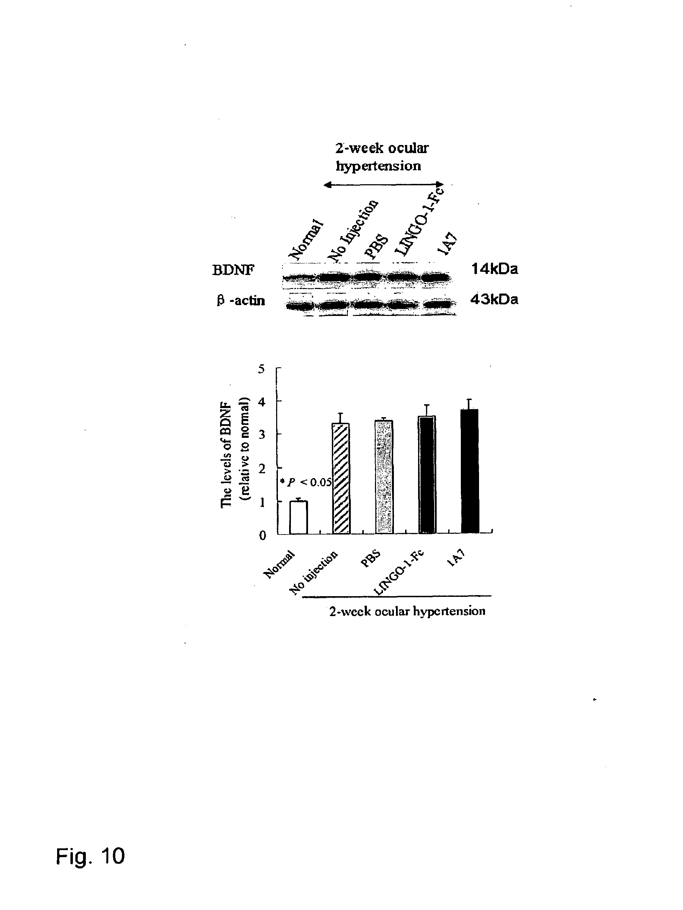

Therefore, in order to investigate the relationship of LINGO-1-Fc and BDNF in the retinas, BDNF was measured in the retina of ocular hypertensive rats using western blotting. Previous studies showed that normal rat retina express BDNF (Rudzinski et al., J. Neurobiol. 58:341-351 (2004)). The results showed a low level of BDNF in the normal retina (P < 0.05, Figure 10). However, BDNF levels increased more than 3-fold 2 weeks after laser coagulation (P < 0.05), consistent with the previously reported results (Rudzinski et al., J. Neurobiol. 58:341-351 (2004)). Thus, laser coagulation activated an endogenous neurotrophin response. However, this response was inadequate to protect against neuronal injury completely, as shown in Figure 7. Treatment with LINGO-1-Fc and 1A7 after ocular hypertension did not change the high levels of BDNF compared to the PBS control group (P < 0.05 compared to normal, Figure 10). These results and the *in vitro* data indicate that LINGO-1-Fc or 1A7 modulates the response to BDNF and reinforces the neuroprotective activity of endogenous BDNF in the glaucomatous retinas.

A neutralizing anti-BDNF antibody was also injected in the presence of LINGO-1-Fc and 1A7. In these experiments, 3µg of the anti-BDNF antibody (Chemicon) was intravitreally injected to the experimental eye on days 0, 3, 7 and 10 after laser coagulation and/or 2 µg LINGO-1-Fc or 1A7 was administrated once on day 0. The rats were euthanized at 2 weeks and FG-labeled RGCs were counted.

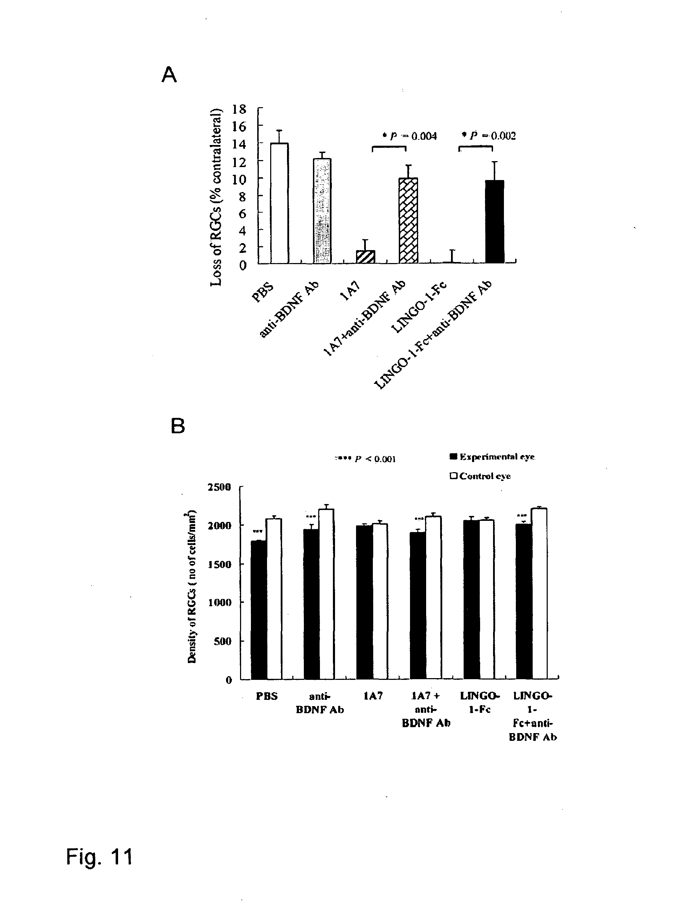

Anti-BDNF antibody significantly reversed the protective function of LINGO-1-Fc (P = 0.002) or 1A7 (P = 0.004) at 2 weeks after laser coagulation. There was no difference in the RGC loss between the PBS and the anti-BDNF antibody group or the anti-BDNF antibody combined with LINGO-1-Fc or 1A7 groups (Figure 11A). The data are also presented as the mean density of RGCs (No. of cells/mm²) (Figure 11B). The proteins in each group have no effect on intraocular pressure. These results further confirmed that LINGO-1-Fc or 1A7 rescued the injured RGCs by reinforcing the increased endogenous BDNF in the retina.

### EXAMPLE 5

### LINGO-1 Binds to and Negatively Regulates TrkB

As shown in Example 4, LINGO-1-Fc exerts its neuroprotective activity by modulating response to BDNF. The relationship of LINGO-1 and the BDNF receptor TrkB was examined using immunoprecipitation and immunoblotting methods. Neurotrophins activate two different classes of receptors, the Trk family of receptor tyrosine kinases and p75 ^{NTR} that in turn activate many downstream signaling pathways. Trk receptors include TrkA, B and C. BDNF activates TrkB (Huang and Reichardt, 2003, Annu. Rev. Biochem. 72:609-642; Huang and Reichardt, 2001, Annu. Rev. Neurosci. 24:677-736). It has previouisly been demonstrated that the dominating Trk receptor in the retina is TrkB (Cui et al., Invest. Ophthalmol. Vis. Sci. 43:1954-1964 (2002)).

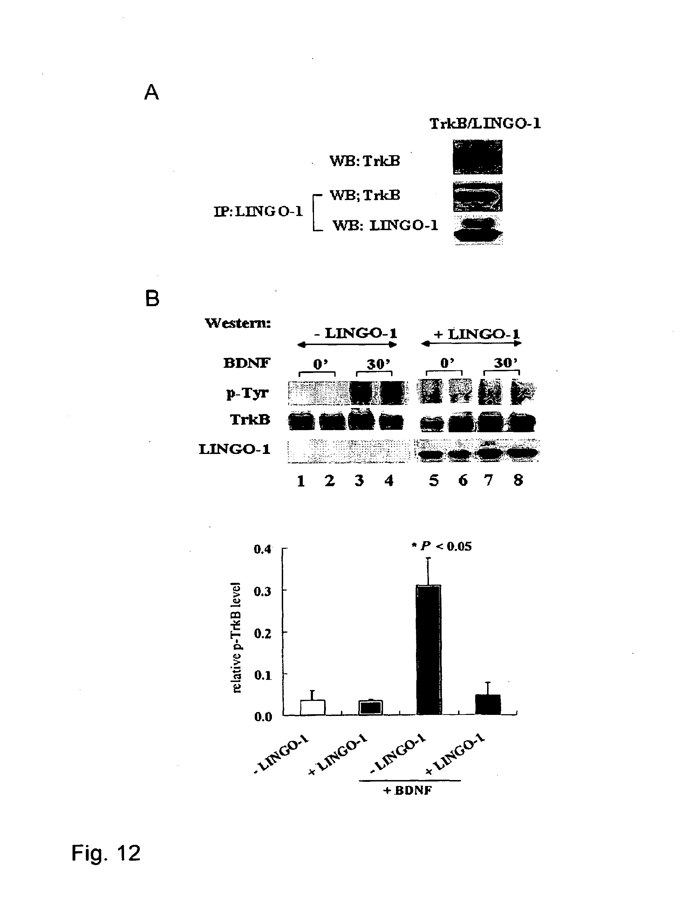

To determine whether LINGO-1 interacts directly with TrkB, cell lysates from transfected 293T cells co-expressing TrkB and LINGO-1 were examined. The lysates were immunoprecipiated with anti-LINGO-1 antibody, and an anti-TrkB antibody or anti-LINGO-1 antibody were used for western blotting. Our results indicated that TrkB was immunoprecipitated with anti-LINGO-1 antibody from 293T cells coexpressing both TrkB and LINGO-1 (Figure 12A). This indicates that LINGO-1 binds TrkB. In addition, a LINGO-1 expressing cell line was transfected with TrkB lentiviruses for 2 days and the cells were treated with BDNF. Lysates were immunoprecipitated using a pan-Trk antibody and then levlels of phosphor-TrkB (p-TrkB) and total TrkB were assessed by western blotting. Total TrkB remained at about the same level in all of the groups (Figure 12B). BDNF stimulation resulted in an incease in phosphorylated TrkB in the absence of LINGO-1 (lanes 3 and 4 of Figure 12B). However, levels of p-TrkB were lower in the presence of LINGO-1 after BDNF stimulation (lanes 7 and 8 of Figure 12B, P < 0.05). These results indicate that LINGO-1 binds TrkB and limits TrkB activation after TrkB is bound by neurotrophins. LINGO-1 thus modulates the function of TrkB receptors, acting as a negative regulator of the neuroprotective activity of the BDNF-TrkB system.

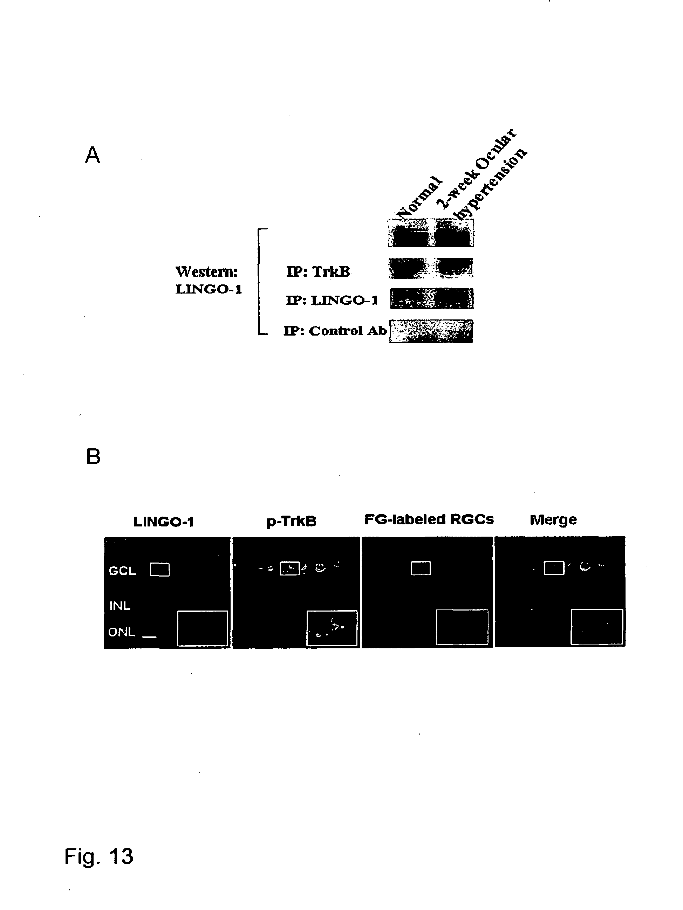

In order to determine whether LINGO-1 and TrkB co-immunoprecipitate from RGCs *in vivo*, the retinal lysates from normal or 2-week ocular hypertension rats were immunoprecipitated with an anti-TrkB antibody, an anti-LINGO-1 antibody or a non-specific antibody, and an anti-LINGO-1 antibody was used for western blotting. LINGO-1 was co-immunoprecipitated from the retinal lysates with anti-TrkB antibody indicating that LINGO-1 interacts with TrkB in normal retina. Furthermore, LINGO-1 constitutively bound to TrkB at 2 weeks after the induction of ocular hypertension (Figure 13). The co-localization of LINGO-1 and TrkB in RGCs was also examined. LINGO-1 and p-TrkB were co-expressed in RGCs retrogradely labeled with FG. Figure 13B shows representative photomicrographs of the co-localization of LINGO-1 and p-TrkB in the retinas 2 weeks after the induction of ocular hypertension and treatment with 1A7.

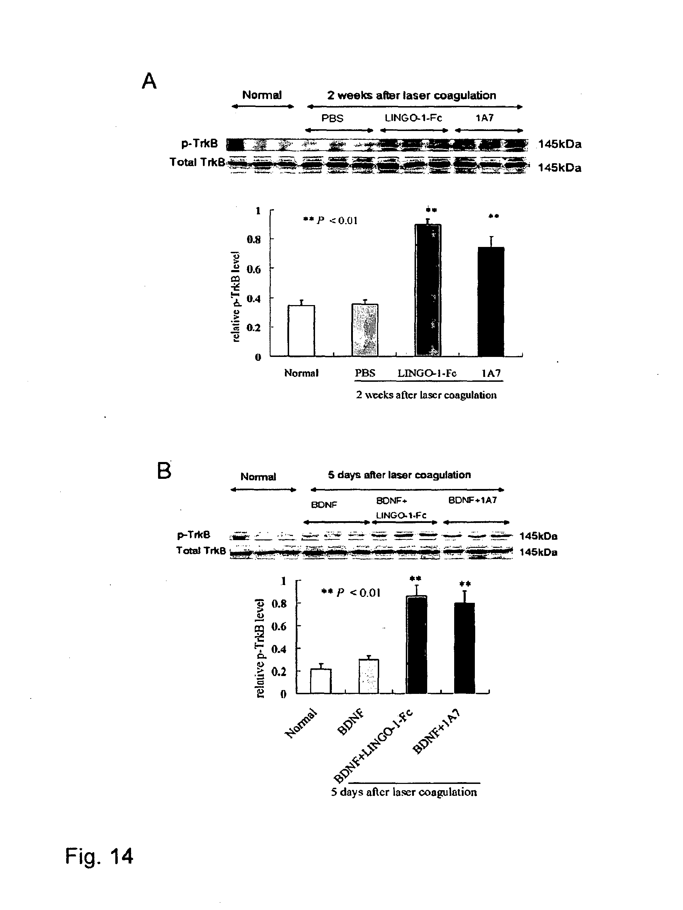

To further assess the mechanisms of LINGO-1-Fc and 1A7 in the ocular hypertension model, the effects of LINGO-1-Fc and 1A7 on TrkB activation were assesed with western blotting. Normal rat retinas expressed high levels of total TrkB (Figure 14A). The total TrkB level was no different between the control group and LINGO-1-Fc group or the 1A7 group 2 weeks after the induction of ocular hypertension (Figure 14A). However, LINGO-1-Fc or 1A7 administration up-regulated p-TrkB level significantly more than the control protein 2 weeks after laser treatment (Figure 14A) demonstrating that LINGO-1-Fc and 1A7 treatment permit activation of TrkB in the presence of endogenous levels of BDNF. BDNF was after injected after laser coagulation. Total TrkB levels remained at the same level up to 5 days after laser coagulation of the retina, whether the eyes were treated with BDNF alone or with a combination of BDNF and LINGO-1-Fc or 1A7 (Figure 14B). BDNF treatment alone increased p-TrkB levels, but the change was not statistically significant after 5 days of treatment. However, BDNF in combination with LINGO-1-Fc or with 1A7 significantly increased p-TrkB levels in the retina (Figure 14B).

These findings indicate that BDNF stimulation alone produced only limited activation of TrkB receptors. This was true even when exogenous BDNF was added. However treatment with LINGO-1-Fc or 1A7 relieved this limitation. These results are consistent with the *in vitro* results showing that LINGO-1 binds with TrkB and negatively regulates TrkB activation after TrkB is bound by BDNF. LINGO-1-Fc and 1A7 treatment promotes neuroprotective activity by increasing BDNF activation of TrkB receptors.

### EXAMPLE 6

### LINGO-1 Antagonists Increase Akt Activation After Ocular Hypertension

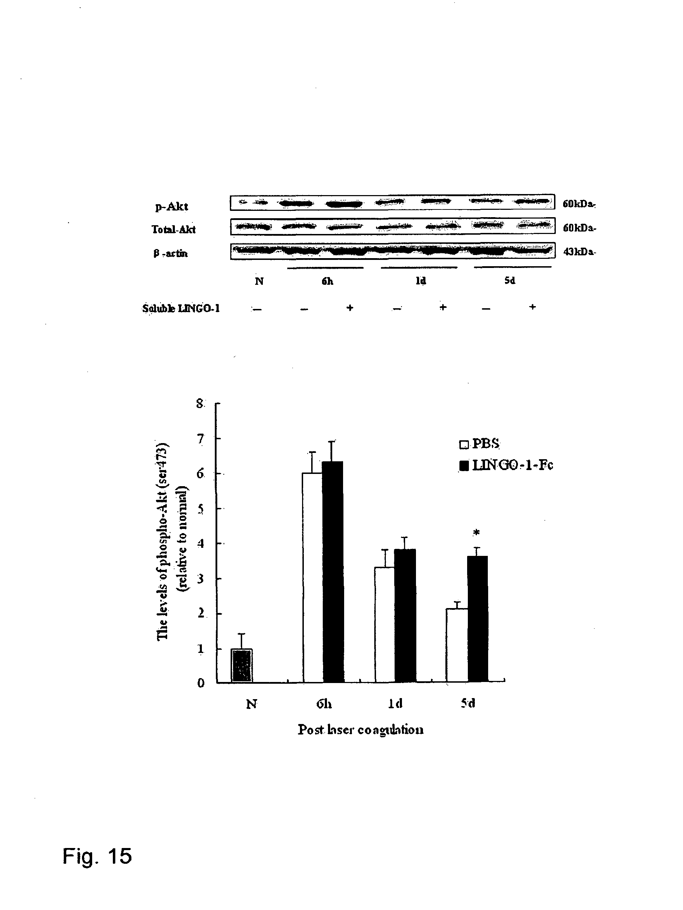

BDNF activation of TrkB receptors initiates several downstream signaling pathways including PI-3 kinase (PI3K). Phosphorylation of serine 473 and threonine 308 by PI3K are important survival signals for neurons (Brazil et al., Cell 111:293-303 (2002)). As shown in Examples 4 and 5, LINGO-1-Fc and 1A7 regulate the function of BDNF and its cognate receptor TrkB. In addition, the effects of LINGO-1-Fc on the PI3K/Akt signaling pathway were examined by measuring the total Akt and pAkt (the active form of Akt) at various times after laser coagulation surgery. Western blot analysis revealed that total Akt levels remained unchanged up to 5 days post laser coagulation (Figure 15). pAkt levels were low in normal control retinas but increased 5-fold by 6 h after laser treatment and then declined from day 1 to day 5 after laser coagulation. A similar bell shaped-response in pAkt levels has been previously reported after optic nerve transection (Cheung et al., Mol. Cell Neurosci. 25:383-393 (2004)). In LINGO-1-Fc treated retinas, the levels of pAkt followed a similar pattern, peaking at 6 h and then declining over 5 days after laser coagulation. However, the levels of pAkt on day 5 were significantly higher in LINGO-1-Fc treated retinas than in control retinas treated with PBS (P < 0.05, Figure 15). Thus, treatment with LINGO-1-Fc affects Akt signaling.

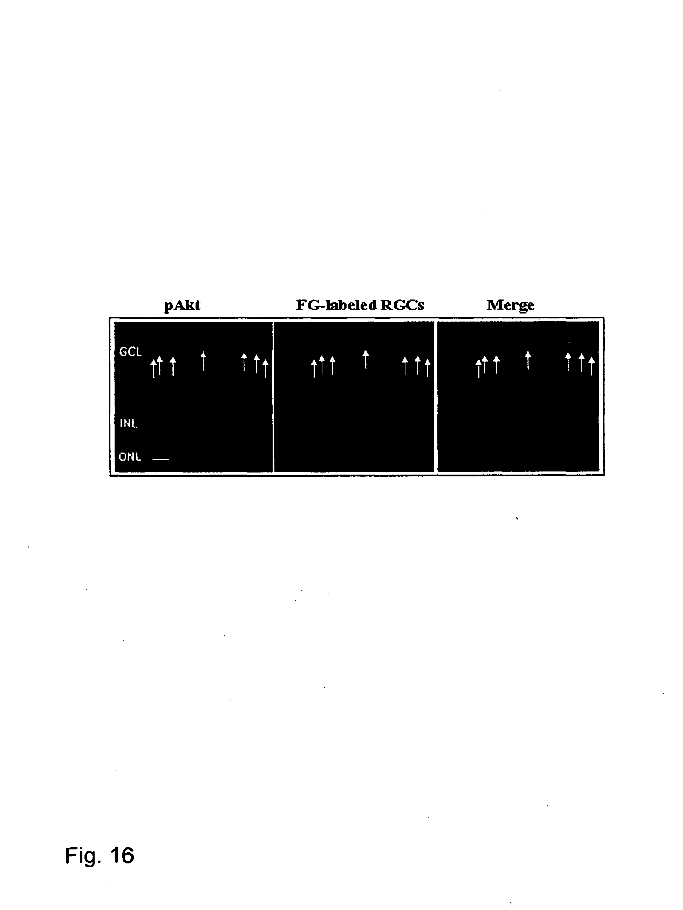

To verify that the changes in pAkt levels occurred in RGCs and not in other cells of the retina, pAkt localization was examined by immunohistochemical study of the retinas. pAkt immunoreactivity only in RGCs retrogradely labeled with FG, and the pAkt localization did not change after laser injury and treatment with LINGO-1-Fc. Figure 16 shows representative photomicrographs of pAkt immunolabelling the retinas at 6 h after laser coagulation and treatment with LINGO-1-Fc.

To further examine the role of pAkt in the neuroprotective activity of LINGO-1-Fc after injury, the effects of an inhibitor of the PI3K/Akt pathway, LY294002 (LY, Calbiochem) were observed. In these experiments, 10 mM LY294002 was dissolved in 100% dimethylsulfoxide (DMSO; Sigma) and subsequently diluted to 2 mM using sterile PBS. 2 mM LY294002 or 2 µl vehicle (20% DMSO in PBS) were injected intravitreally on days 0, 3, 7 and 10 days after the first laser photocoagulation was performed on the right eye. Animals were euthanized on day 14 and FG-labeled RGCs were counted.

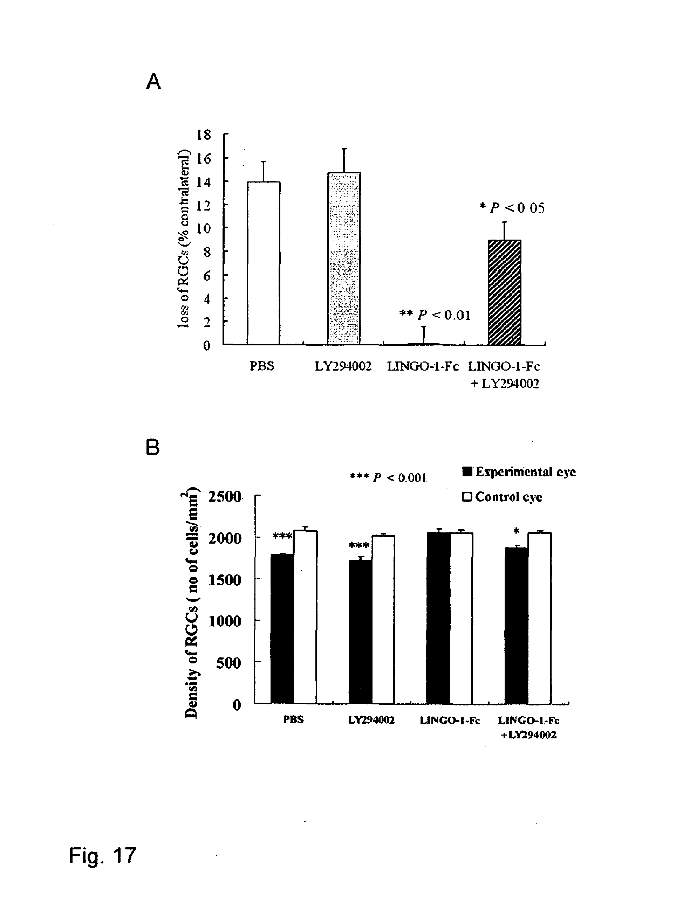

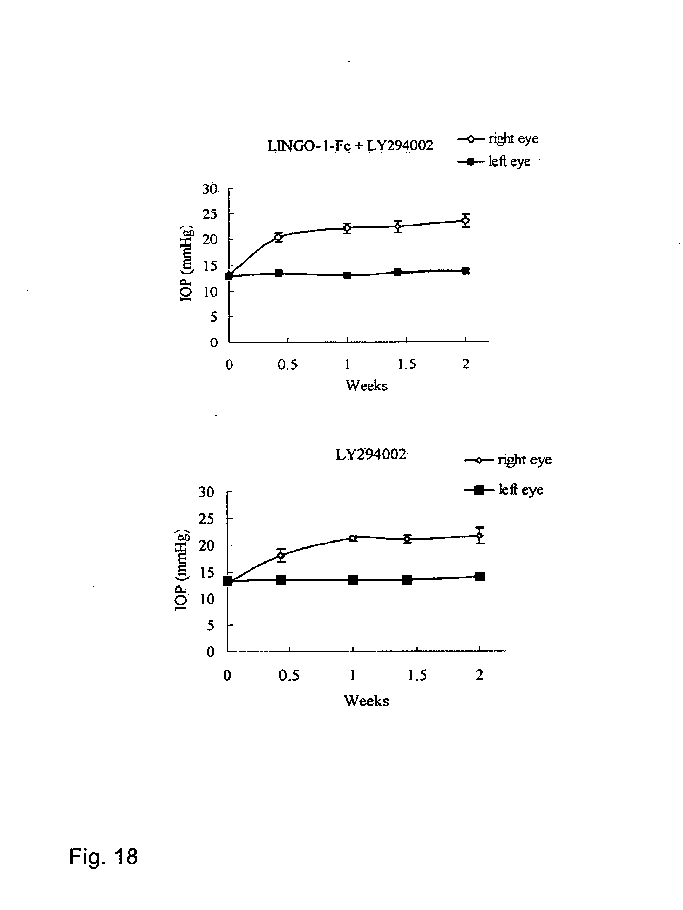

While LINGO-1-Fc promoted RGC survival at 2 weeks after ocular hypertension, combining LINGO-1-Fc with LY294002 abolished the neuroprotective effect (Figure 17A). Loss of RGCs increased from 0.09 ± 1.47 % in eyes treated with LINGO-1-Fc to 9.0 ± 1.6% in eyes treated with LINGO-1-Fc and LY294002 (P = 0.004). The data are also presented by the density of RGCs (Figure 17B). Neither LY294002 alone or in combination with LINGO-1-Fc lowered intraocular pressure (Figure 18).

### EXAMPLE 7

### LINGO-1 Antagonists Decrease c-Jun N-terminal Kinase Activation After Ocular Hypertension

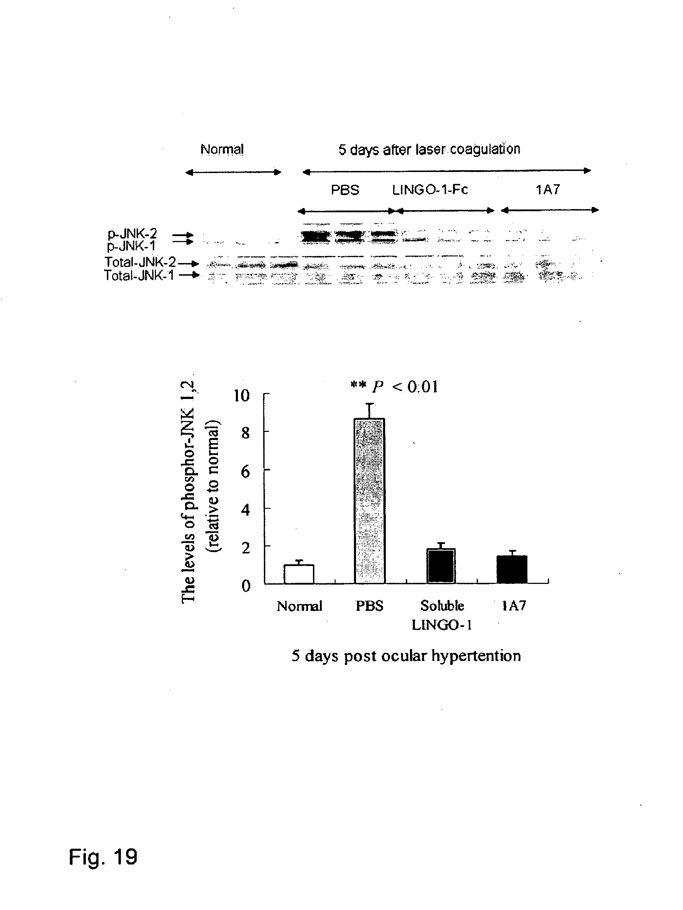

The JNK pathway can be activated by a variety of cellular stresses. More recently, phosphorylated JNK was detected in human and experimental rat glaucomatous retinas, and the activation of JNK was temporally associated with the death of RGCs (Tezel et al. Invest. Opthamol.Vis. Sci. 44:3025-3033 (2003)). Recent findings also confirmed that phosphorylated JNK is located in the RGCs after IOP elevation. Total JNK-1 and JNK-2 levels remain unchanged up to 5 days after ocular hypertension (Figure 19). Phosphorylated JNK-1,2 were low in the normal retina, then increased to more than 8-fold after 5 days. A similar response in the p-JNK levels following IOP elevation in a rat glaucoma model has been previously reported by immunohistochemistry. After treatment with LINGO-1-Fc or 1A7, the activation of JNK was almost reduced to the normal level (P < 0.01) (Figure 19). These results indicate that LINGO-1 antagonists may rescue injured RGCs by inhibiting the activation of JNK signaling pathway.

### EXAMPLE 8

### LINGO-1 Antagonists Decrease RhoA Activation After Ocular Hypertension

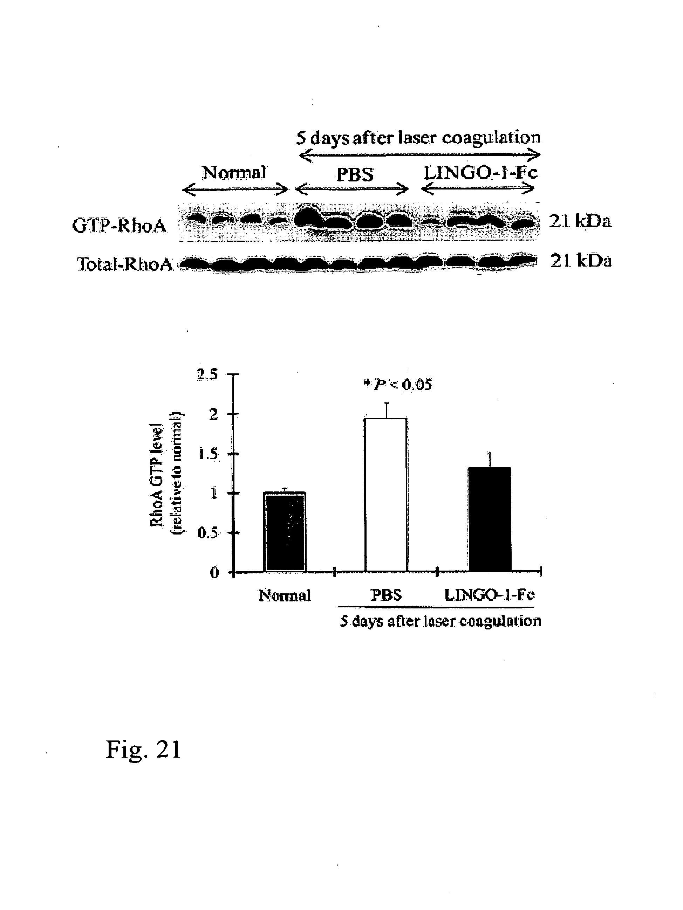

Since Rho acts downstream of the NgR1-LINGO-1-p75/TROY complex, and JNK is phosphorylated as a downstream consequence of Rho activation, experiments were also performed to determined if LINGO-1 antagonists have an effect on the activity of Rho after ocular hypertension. Rho activity can be assessed by evaluating GTP-RhoA levels, where increased levels of GTP-RhoA are associated with increased Rho activity. Low levels of GTP-RhoA were observed in normal retina, and the GTP-RhoA levels increased almost twofold five days after laser coagulation (p < 0.05 compared with normal group) (Figure 21). In contrast, LINGO-1-Fc treated retinas significantly reduced the high level of GTP-RhoA to the basal level (p < 0.05 compared with the PBS group) (Figure 21). These results suggest that LINGO-1-Fc exerts neuroprotective activity by inhibiting RhoA activation.

The present invention is not to be limited in scope by the specific embodiments described which are intended as single illustrations of individual aspects of the invention. Various modifications of the invention in addition to those shown and described herein will become apparent to those skilled in the art from the foregoing description and accompanying drawings.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in anyway.

## Claims

1. A LINGO-1 antagonist for use in the treatment of glaucoma,
wherein the treatment comprises administration of the LINGO-1 antagonist in combination with a TrkB agonist;
wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or antigen-binding fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody, or antigen-binding fragment thereof;
(iv) a TrkB agonist polynucleotide; and
(v) a TrkB agonist aptamer.

2. A TrkB agonist for use in the treatment of glaucoma,
wherein the treatment comprises administration of the TrkB agonist in combination with a LINGO-1 antagonist;
wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or antigen-binding fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody, or antigen-binding fragment thereof;
(iv) a TrkB agonist polynucleotide; and
(v) a TrkB agonist aptamer.

3. A LINGO-1 antagonist and TrkB agonist for use in the treatment of glaucoma,
wherein the treatment comprises administration of the TrkB agonist and the LINGO-1 antagonist simultaneously, separately or sequentially;
wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or antigen-binding fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody, or antigen-binding fragment thereof;
(iv) a TrkB agonist polynucleotide; and
(v) a TrkB agonist aptamer.

4. Use of a LINGO-1 antagonist for the preparation of a medicament for the treatment of glaucoma,
wherein the treatment comprises administration of the medicament in combination with a TrkB agonist;
wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or antigen-binding fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody, or antigen-binding fragment thereof;
(iv) a TrkB agonist polynucleotide; and
(v) a TrkB agonist aptamer.

5. Use of a TrkB agonist for the preparation of a medicament for the treatment of glaucoma,
wherein the treatment comprises administration of the medicament in combination with a LINGO-1 antagonist;
wherein the LINGO-1 antagonist is selected from the group consisting of:
(i) a soluble LINGO-1 polypeptide, optionally fused to a non-LINGO-1-antagonist heterologous polypeptide;
(ii) a LINGO-1 antagonist antibody, or antigen-binding fragment thereof; and
(iii) a LINGO-1 antagonist polynucleotide; and
wherein the TrkB agonist is selected from the group consisting of:
(i) a TrkB agonist compound selected from the group consisting of L-783,281, adenosine and CGS 21680;
(ii) a polypeptide selected from the group consisting of a TrkB polypeptide, a neurotrophin, BDNF, NT-3, NT-4/5, a chimeric neurotrophin, a pan-neurotrophin, a mini-neurotrophin and B_{AG}, optionally fused to a non-TrkB-agonist heterologous polypeptide;
(iii) a TrkB agonist antibody, or antigen-binding fragment thereof;
(iv) a TrkB agonist polynucleotide; and
(v) a TrkB agonist aptamer.

6. The LINGO-1 antagonist for use according to Claim 1, the TrkB agonist for use according to Claim 2, the LINGO-1 antagonist and TrkB agonist for use according to Claim 3, or the use of Claim 4 or 5, wherein the soluble LINGO-1 polypeptide is selected from the group consisting of:
(i) a polypeptide comprising a LINGO-1 LRR domain, a LINGO-1 basic region C-terminal to the LRR domain, and a LINGO-1 immunoglobulin (Ig) domain;
(ii) a polypeptide comprising amino acids 34-532 of SEQ ID NO:2 or amino acids 36-532 of SEQ ID NO:2;
(iii) a polypeptide comprising a LINGO-1 Ig domain; and
(iv) a polypeptide comprising amino acids 417-493 of SEQ ID NO:2.

7. The LINGO-1 antagonist for use according to Claim 1 or Claim 6, the TrkB agonist for use according to Claim 2 or Claim 6, the LINGO-1 antagonist and TrkB agonist for use according to Claim 3 or Claim 6, or the use according to any of Claims 4 to 6, wherein the non-LINGO-1 antagonist heterologous polypeptide is selected from the group consisting of an immunoglobulin fragment, serum albumin, a targeting protein, a reporter protein, and a purification-facilitating protein.

8. The LINGO-1 antagonist for use according to Claim 1, the TrkB agonist for use according to Claim 2, the LINGO-1 antagonist and TrkB agonist for use according to Claim 3, or the use of Claim 4 or 5, wherein the LINGO-1 antibody is an isolated LINGO-1 antibody that specifically binds to the same LINGO-1 epitope as a reference monoclonal antibody selected from the group consisting of: 1A7, 2F3, 3P1D10.2C3, 3P1 E11.3B7, 3B5.230-C12 (Li01), 38-D01 (Li02), 35-E04 (Li03), 36-C09 (Li04), 30-A11 (Li05), 34-F02 (Li06), 29-E07 (Li07), 34-G04 (Li08), 36-A12 (Li09), 28-D02 (Li10), 30-B01 (Li11), 34-B03 (Li12), Li13, Li32, Li33, Li34, 3383 (L1a.1), 3495(L1a.2), 3563 (L1a.3), 3564 (L1a.4), 3565 (L1a.5), 3566 (L1a.6), 3567 (L1a.7), 3568 (L1a.8), 3569 (L1a.9), 3570 (L1a.10), 3571 (L1a.11), 3582 (L1a.12), and 1968 (L1a.13).

9. The LINGO-1 antagonist for use according to Claim 1, the TrkB agonist for use according to Claim 2, the LINGO-1 antagonist and TrkB agonist for use according to Claim 3, or the use of Claim 4 or 5, wherein the LINGO-1 antagonist comprises a LINGO-1 antagonist polynucleotide is selected from the group consisting of:
(i) an antisense polynucleotide;
(ii) a ribozyme;
(iii) a small interfering RNA (siRNA); and
(iv) a small-hairpin RNA (shRNA).

10. The LINGO-1 antagonist, the TrkB agonist or the LINGO-1 antagonist and TrkB agonist for use according to Claim 9, or the use of Claim 9, wherein said shRNA comprises the nucleotide sequence: UGAUCGUCAU CCUGCUAGAC UUCAAGAGAG UCUAGCAGGA UGACGAUCUU UUUUC (SEQ ID NO:34).

11. The LINGO-1 antagonist for use according to Claim 1, the TrkB agonist for use according to Claim 2, the LINGO-1 antagonist and TrkB agonist for use according to Claim 3, or the use of Claim 4 or 5, wherein the TrkB polypeptide comprises:
(a) amino acids 1 to 828 of SEQ ID NO:4 fused to an IgG domain; or
(b) amino acids 32 to 828 of SEQ ID NO: 4 fused to an IgG domain.

12. The LINGO-1 antagonist for use according to Claim 1, the TrkB agonist for use according to Claim 2, the LINGO-1 antagonist and TrkB agonist for use according to Claim 3, or the use of Claim 4 or 5, wherein the TrkB agonist polypeptide is a BDNF polypeptide, or the TrkB agonist polynucleotide encodes a BDNF polypeptide.

13. The LINGO-1 antagonist for use according to Claim 1, the TrkB agonist for use according to Claim 2, the LINGO-1 antagonist and TrkB agonist for use according to Claim 3, or the use of Claim 4 or 5, wherein the TrkB agonist antibody or antigen-binding fragment thereof is selected from the group consisting of: 6E2, 7F5, 11 E1, 16E11, 17D11, 19E12, and 29D7.

14. The LINGO-1 antagonist for use according to Claim 1, the TrkB agonist for use according to Claim 2, the LINGO-1 antagonist and TrkB agonist for use according to Claim 3, or the use of Claim 4 or 5, wherein
the LINGO-1 antibody is an isolated LINGO-1 antibody that specifically binds to the same LINGO-1 epitope as a reference monoclonal antibody selected from Li13 or Li33; and wherein
the TrkB agonist antibody or antigen-binding fragment thereof is selected from the group consisting of: 6E2, 7F5, 11E1, 16E11, 17D11, 19E12, and 29D7, or the TrkB agonist polypeptide is a BDNF polypeptide.

## Patentansprüche

1. LINGO-1-Antagonist zur Verwendung bei der Behandlung eines Glaukoms,
wobei die Behandlung das Verabreichen des LINGO-1-Antagonisten in Kombination mit einem TrkB-Agonisten beinhaltet;
wobei der LINGO-1-Antagonist ausgewählt ist aus der Gruppe bestehend aus:
(i) einem löslichen LINGO-1-Polypeptid, optional fusioniert mit einem heterologen Nicht-LINGO-1-Antagonisten-Polypeptid;
(ii) einem LINGO-1-Antagonisten-Antikörper oder Antigenbindungsfragment davon; und
(iii) einem LINGO-1-Antagonisten-Polynucleotid; und
wobei der TrkB-Agonist ausgewählt ist aus der Gruppe bestehend aus:
(i) einer TrkB-Agonistenverbindung, ausgewählt aus der Gruppe bestehend aus L-783,281, Adenosin und CGS 21680;
(ii) einem Polypeptid, ausgewählt aus der Gruppe bestehend aus einem TrkB-Polypeptid, einem Neurothropin, BDNF, NT-3, NT-4/5, einem chimären Neurotrophin, einem Pan-Neurotrophin, einem Mini-Neurotrophin und B_{AG}, optional fusioniert mit einem heterologen Nicht-TrkB-Agonisten-Polypeptid;
(iii) einem TrkB-Agonisten-Antikörper oder Antigenbindungsfragment davon;
(iv) einem TrkB-Agonisten-Polynucleotid; und
(v) einem TrkB-Agonisten-Aptamer.

2. TrkB-Agonist zur Verwendung bei der Behandlung eines Glaukoms,
wobei die Behandlung das Verabreichen des TrkB-Angonisten in Kombination mit einem LINGO-1-Antagonisten beinhaltet;
wobei der LINGO-1-Antagonist ausgewählt ist aus der Gruppe bestehend aus:
(i) einem löslichen LINGO-1-Polypeptid, optional fusioniert mit einem heterologen Nicht-LINGO-1-Antagonisten-Polypeptid;
(ii) einem LINGO-1-Antagonisten-Antikörper oder Antigenbindungsfragment davon; und
(iii) einem LINGO-1-Antagonisten-Polynucleotid; und
wobei der TrkB-Agonist ausgewählt ist aus der Gruppe bestehend aus:
(i) einer TrkB-Agonistenverbindung, ausgewählt aus der Gruppe bestehend aus L-783,281, Adenosin und CGS 21680;
(ii) einem Polypeptid, ausgewählt aus der Gruppe bestehend aus einem TrkB-Polypeptid, einem Neurotrophin, BDNF, NT-3, NT-4/5, einem chimären Neurotrophin, einem Pan-Neurotrophin, einem Mini-Neurotrophin und B_{AG}, optional fusioniert mit einem heterologen Nicht-TrkB-Agonisten-Polypeptid;
(iii) einem TrkB-Agonisten-Antikörper oder Antigenbindungsfragment davon;
(iv) einem TrkB-Agonisten-Polynucleotid; und
(v) einem TrkB-Agonisten-Aptamer.

3. LINGO-1-Antagonist und TrkB-Agonist zur Verwendung bei der Behandlung eines Glaukoms,
wobei die Behandlung das gleichzeitige, separate oder sequenzielle Verabreichen des TrkB-Agonisten und LINGO-1-Antagonisten beinhaltet;
wobei der LINGO-1-Antagonist ausgewählt ist aus der Gruppe bestehend aus:
(i) einem löslichen LINGO-1-Polypeptid, optional fusioniert mit einem heterologen Nicht-LINGO-1-Antagonisten-Polypeptid;
(ii) einem LINGO-1-Antagonisten-Antikörper oder Antigenbindungsfragment davon; und
(iii) einem LINGO-1-Antagonisten-Polynucleotid; und
wobei der TrkB-Agonist ausgewählt ist aus der Gruppe bestehend aus:
(i) einer TrkB-Agonistenverbindung, ausgewählt aus der Gruppe bestehend aus L-783,281, Adenosin und CGS 21680;
(ii) einem Polypeptid, ausgewählt aus der Gruppe bestehend aus einem TrkB-Polypeptid, einem Neurotrophin, BDNF, NT-3, NT-4/5, einem chimären Neurotrophin, einem Pan-Neurotrophin, einem Mini-Neurotrophin und B_{AG}, optional fusioniert mit einem heterologen Nicht-TrkB-Agonisten-Polypeptid;
(iii) einem TrkB-Agonisten-Antikörper oder Antigenbindungsfragment davon;
(iv) einem TrkB-Agonisten-Polynucleotid; und
(v) einem TrkB-Agonisten-Aptamer.

4. Verwendung eines LINGO-1-Antagonisten zur Herstellung eines Medikaments zur Behandlung eines Glaukoms,
wobei die Behandlung das Verabreichen des Medikaments in Kombination mit einem TrkB-Agonisten beinhaltet;
wobei der LINGO-1-Antagonist ausgewählt ist aus der Gruppe bestehend aus:
(i) einem löslichen LINGO-1-Polypeptid, optional fusioniert mit einem heterologen Nicht-LINGO-1-Antagonisten-Polypeptid;
(ii) einem LINGO-1-Antagonisten-Antikörper oder Antigenbindungsfragment davon; und
(iii) einem LINGO-1-Antagonisten-Polynucleotid; und
wobei der TrkB-Agonist ausgewählt ist aus der Gruppe bestehend aus:
(i) einer TrkB-Agonistenverbindung, ausgewählt aus der Gruppe bestehend aus L-783,281, Adenosin und CGS 21680;
(ii) einem Polypeptid, ausgewählt aus der Gruppe bestehend aus einem TrkB-Polypeptid, einem Neurotrophin, BDNF, NT-3, NT-4/5, einem chimären Neurotrophin, einem Pan-Neurotrophin, einem Mini-Neurotrophin und B_{AG}, optional fusioniert mit einem heterologen Nicht-TrkB-Agonisten-Polypeptid;
(iii) einem TrkB-Agonisten-Antikörper oder Antigenbindungsfragment davon;
(iv) einem TrkB-Agonisten-Polynucleotid; und
(v) einem TrkB-Agonisten-Aptamer.

5. Verwendung eines TrkB-Agonisten zur Herstellung eines Medikaments zur Behandlung eines Glaukoms,
wobei die Behandlung das Verabreichen des Medikaments in Kombination mit einem LINGO-1-Antagonisten beinhaltet;
wobei der LINGO-1-Antagonist ausgewählt ist aus der Gruppe bestehend aus:
(i) einem löslichen LINGO-1-Polypeptid, optional fusioniert mit einem heterologen Nicht-LINGO-1-Antagonisten-Polypeptid;
(ii) einem LINGO-1-Antagonisten-Antikörper oder Antigenbindungsfragment davon; und
(iii) einem LINGO-1-Antagonisten-Polynucleotid; und
wobei der TrkB-Agonist ausgewählt ist aus der Gruppe bestehend aus:
(i) einer TrkB-Agonistenverbindung, ausgewählt aus der Gruppe bestehend aus L-783,281, Adenosin und CGS 21680;
(ii) einem Polypeptid, ausgewählt aus der Gruppe bestehend aus einem TrkB-Polypeptid, einem Neurotrophin, BDNF, NT-3, NT-4/5, einem chimären Neurotrophin, einem Pan-Neurotrophin, einem Mini-Neurotrophin und B_{AG}, optional fusioniert mit einem heterologen Nicht-TrkB-Agonisten-Polypeptid;
(iii) einem TrkB-Agonisten-Antikörper oder Antigenbindungsfragment davon;
(iv) einem TrkB-Agonisten-Polynucleotid; und
(v) einem TrkB-Agonisten-Aptamer.

6. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei das lösliche LINGO-1-Polypeptid ausgewählt ist aus der Gruppe bestehend aus:
(i) einem Polypeptid, das eine LINGO-1-LRR-Domäne, eine LINGO-1 Basenregion C-terminal zur LRR-Domäne und eine LINGO-1-Immunglobulin-(Ig)-Domäne beinhaltet;
(ii) einem Polypeptid, das die Aminosäuren 34-532 der SEQ ID Nr. 2 oder die Aminosäuren 36-532 der SEQ ID Nr. 2 beinhaltet;
(iii) Polypeptid, das eine LINGO-1-Ig-Domäne beinhaltet; und
(iv) Polypeptid, das die Aminosäuren 417-493 der SEQ ID Nr. 2 beinhaltet.

7. LINGO-1-Antagonist zur Verwendung nach Anspruch 1 oder Anspruch 6, TrkB-Agonist zur Verwendung nach Anspruch 2 oder Anspruch 6, LINGO-1-Antagonist und TrkB-Agonist zur Verwendung nach Anspruch 3 oder Anspruch 6 oder Verwendung nach einem der Ansprüche 4 bis 6, wobei das heterologe Nicht-LINGO-1-Antagonisten-Polypeptid aus der Gruppe bestehend aus einem Immunglobulinfragment, Serumalbumin, einem Targetierungsprotein, einem Reporterprotein und einem reinigungserleichternden Protein ausgewählt ist.

8. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei der LINGO-1-Antikörper ein isolierter LINGO-1-Antikörper ist, der sich spezifisch an das gleiche LINGO-1-Epitop wie ein monoklonaler Referenzantikörper bindet, ausgewählt aus der Gruppe bestehend aus: 1A7, 2F3, 3P1D10.2C3, 3P1E11.3B7, 3B5.230-C12 (Li01), 38-D01 (Li02), 35-E04 (Li03), 36-C09 (Li04), 30-A11 (Li05), 34-F02 (Li06), 29-E07 (Li07), 34-G04 (Li08), 36-A12 (Li09), 28-D02 (Li10), 30-B01 (Li11), 34-B03 (Li12), Li13, Li32, Li33, Li34, 3383 (L1a.1), 3495 (L1a.2), 3563 (L1a.3), 3564 (L1a.4), 3565 (L1a.5), 3566 (L1a.6), 3567 (L1a.7), 3568 (L1a.8), 3569 (L1a.9), 3570 (L1a.10), 3571 (L1a.11), 3582 (L1a.12) und 1968 (L1a.13).

9. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei der LINGO-1-Antagonist ein LINGO-1-Antagonisten-Polynucleotid beinhaltet, ausgewählt aus der Gruppe bestehend aus:
(i) einem Antisense-Polynucleotid;
(ii) einem Ribozym;
(iii) einer kurzen interferierenden RNA (siRNA); und
(iv) einer kleinen Haarnadel-RNA (shRNA).

10. LINGO-1-Antagonist, TrkB-Agonist oder LINGO-1-Antagonist und TrkB-Agonist zur Verwendung nach Anspruch 9 oder Verwendung nach Anspruch 9, wobei die genannte shRNA die folgende Nucleotidsequenz beinhaltet: UGAUCGUCAU CCUGCUAGAC UUCAAGAGAG UCUAGCAGGA UGACGAUCUU UUUUC (SEQ ID Nr. 34).

11. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei das TrkB-Polypeptid Folgendes beinhaltet:
(a) Aminosäuren 1 bis 828 der SEQ ID Nr. 4, fusioniert mit einer IgG-Domäne; oder
(b) Aminosäuren 32 bis 828 der SEQ ID Nr. 4, fusioniert mit einer IgG-Domäne.

12. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei das TrkB-Agonisten-Polypeptid ein BDNF-Polypeptid ist oder das TrkB-Agonisten-Polynucleotid ein BDNF-Polypeptid kodiert.

13. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei der TrkB-Agonisten-Antikörper oder das Antigenbindungsfragment davon aus der Gruppe bestehend aus 6E2, 7F5, 11E1, 16E11, 17D11, 19E12 und 29D7 ausgewählt ist.

14. LINGO-1-Antagonist zur Verwendung nach Anspruch 1, TrkB-Agonist zur Verwendung nach Anspruch 2, LINGO-1-Antagonist und TrkB-Agonist zur Verwendung nach Anspruch 3 oder Verwendung nach Anspruch 4 oder 5, wobei
der LINGO-1-Antikörper ein isolierter LINGO-1-Antikörper ist, der sich spezifisch an das gleiche LINGO-1-Epitop wie ein monoklonaler Referenzantikörper bindet, ausgewählt aus Li13 oder Li33; und wobei
der TrkB-Agonisten-Antikörper oder das Antigenbindungsfragment davon aus der Gruppe bestehend aus 6E2, 7F5, 11E1, 16E11, 17D11, 19E12 und 29D7 ausgewählt ist oder das TrkB-Agonisten-Polypeptid ein BDNF-Polypeptid ist.

## Revendications

1. Antagoniste de LINGO-1 destiné à une utilisation dans le traitement du glaucome,
le traitement comprenant l'administration de l'antagoniste de LINGO-1 en association avec un agoniste de TrkB ;
l'antagoniste de LINGO-1 étant sélectionné dans le groupe constitué de :
(i) un polypeptide soluble de LINGO-1, optionnellement fusionné à un polypeptide hétérologue non antagoniste de LINGO-1 ;
(ii) un anticorps antagoniste de LINGO-1, ou un fragment de celui-ci se liant à un antigène ; et
(iii) un polynucléotide antagoniste de LINGO-1 ; et
l'agoniste de TrkB étant sélectionné dans le groupe constitué de :
(i) un composé agoniste de TrkB sélectionné dans le groupe constitué de L-783,281, de l'adénosine et de CGS 21680 ;
(ii) un polypeptide sélectionné dans le groupe constitué d'un polypeptide de TrkB, d'une neurotrophine, du BDNF, de NT-3, de NT-4/5, d'une neurotrophine chimérique, d'une pan-neurotrophine, d'une mini-neurotrophine et de BAG, optionnellement fusionné à un polypeptide hétérologue non agoniste de TrkB ;
(iii) un anticorps agoniste de TrkB, ou un fragment de celui-ci se liant à un antigène ;
(iv) un polynucléotide agoniste de TrkB ; et
(v) un aptamère agoniste de TrkB.

2. Agoniste de TrkB destiné à une utilisation dans le traitement du glaucome,
le traitement comprenant l'administration de l'agoniste de TrkB en association avec un antagoniste de LINGO-1 ;
l'antagoniste de LINGO-1 étant sélectionné dans le groupe constitué de :
(i) un polypeptide soluble de LINGO-1, optionnellement fusionné à un polypeptide hétérologue non antagoniste de LINGO-1 ;
(ii) un anticorps antagoniste de LINGO-1, ou un fragment de celui-ci se liant à un antigène ; et
(iii) un polynucléotide antagoniste de LINGO-1 ; et
l'agoniste de TrkB étant sélectionné dans le groupe constitué de :
(i) un composé agoniste de TrkB sélectionné dans le groupe constitué de L-783,281, de l'adénosine et de CGS 21680 ;
(ii) un polypeptide sélectionné dans le groupe constitué d'un polypeptide de TrkB, d'une neurotrophine, du BDNF, de NT-3, de NT-4/5, d'une neurotrophine chimérique, d'une pan-neurotrophine, d'une mini-neurotrophine et de B_{AG}, optionnellement fusionné à un polypeptide hétérologue non agoniste de TrkB ;
(iii) un anticorps agoniste de TrkB, ou un fragment de celui-ci se liant à un antigène ;
(iv) un polynucléotide agoniste de TrkB ; et
(v) un aptamère agoniste de TrkB.

3. Antagoniste de LINGO-1 et agoniste de TrkB destinés à une utilisation dans le traitement du glaucome,
le traitement comprenant l'administration de l'agoniste de TrkB et de l'antagoniste de LINGO-1 simultanément, séparément ou séquentiellement ;
l'antagoniste de LINGO-1 étant sélectionné dans le groupe constitué de :
(i) un polypeptide soluble de LINGO-1, optionnellement fusionné à un polypeptide hétérologue non antagoniste de LINGO-1 ;
(ii) un anticorps antagoniste de LINGO-1, ou un fragment de celui-ci se liant à un antigène ; et
(iii) un polynucléotide antagoniste de LINGO-1 ; et l'agoniste de TrkB étant sélectionné dans le groupe constitué de :
(i) un composé agoniste de TrkB sélectionné dans le groupe constitué de L-783,281, de l'adénosine et de CGS 21680 ;
(ii) un polypeptide sélectionné dans le groupe constitué d'un polypeptide de TrkB, d'une neurotrophine, du BDNF, de NT-3, de NT-4/5, d'une neurotrophine chimérique, d'une pan-neurotrophine, d'une mini-neurotrophine et de BAG, optionnellement fusionné à un polypeptide hétérologue non agoniste de TrkB ;
(iii) un anticorps agoniste de TrkB, ou un fragment de celui-ci se liant à un antigène ;
(iv) un polynucléotide agoniste de TrkB ; et
(v) un aptamère agoniste de TrkB.

4. Utilisation d'un antagoniste de LINGO-1 pour la préparation d'un médicament destiné au traitement du glaucome,
le traitement comprenant l'administration du médicament en association avec un agoniste de TrkB ;
l'antagoniste de LINGO-1 étant sélectionné dans le groupe constitué de :
(i) un polypeptide soluble de LINGO-1, optionnellement fusionné à un polypeptide hétérologue non antagoniste de LINGO-1 ;
(ii) un anticorps antagoniste de LINGO-1, ou un fragment de celui-ci se liant à un antigène ; et
(iii) un polynucléotide antagoniste de LINGO-1 ; et l'agoniste de TrkB étant sélectionné dans le groupe constitué de :
(i) un composé agoniste de TrkB sélectionné dans le groupe constitué de L-783,281, de l'adénosine et de CGS 21680 ;
(ii) un polypeptide sélectionné dans le groupe constitué d'un polypeptide de TrkB, d'une neurotrophine, du BDNF, de NT-3, de NT-4/5, d'une neurotrophine chimérique, d'une pan-neurotrophine, d'une mini-neurotrophine et de BAG, optionnellement fusionné à un polypeptide hétérologue non agoniste de TrkB ;
(iii) un anticorps agoniste de TrkB, ou un fragment de celui-ci se liant à un antigène ;
(iv) un polynucléotide agoniste de TrkB ; et
(v) un aptamère agoniste de TrkB.

5. Utilisation d'un agoniste de TrkB pour la préparation d'un médicament destiné au traitement du glaucome,
le traitement comprenant l'administration du médicament en association avec un antagoniste de LINGO-1 ;
l'antagoniste de LINGO-1 étant sélectionné dans le groupe constitué de :
(i) un polypeptide soluble de LINGO-1, optionnellement fusionné à un polypeptide hétérologue non antagoniste de LINGO-1 ;
(ii) un anticorps antagoniste de LINGO-1, ou un fragment de celui-ci se liant à un antigène ; et
(iii) un polynucléotide antagoniste de LINGO-1 ; et l'agoniste de TrkB étant sélectionné dans le groupe constitué de :
(i) un composé agoniste de TrkB sélectionné dans le groupe constitué de L-783,281, de l'adénosine et de CGS 21680 ;
(ii) un polypeptide sélectionné dans le groupe constitué d'un polypeptide de TrkB, d'une neurotrophine, du BDNF, de NT-3, de NT-4/5, d'une neurotrophine chimérique, d'une pan-neurotrophine, d'une mini-neurotrophine et de BAG, optionnellement fusionné à un polypeptide hétérologue non agoniste de TrkB ;
(iii) un anticorps agoniste de TrkB, ou un fragment de celui-ci se liant à un antigène ;
(iv) un polynucléotide agoniste de TrkB ; et
(v) un aptamère agoniste de TrkB.

6. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, le polypeptide soluble de LINGO-1 étant sélectionné dans le groupe constitué de :
(i) un polypeptide comprenant un domaine LRR de LINGO-1, une région basique de LINGO-1 C-terminale relativement au domaine LRR, et un domaine d'immunoglobuline (Ig) de LINGO-1 ;
(ii) un polypeptide comprenant les acides aminés 34 à 532 de la SÉQ. ID n° 2 ou les acides aminés 36 à 532 de la SÉQ. ID n° 2 ;
(iii) un polypeptide comprenant un domaine d'Ig de LINGO-1 ; et
(iv) un polypeptide comprenant les acides aminés 417 à 493 de la SÉQ. ID n° 2.

7. Antagoniste de LINGO-1 destiné à une utilisation selon la revendication 1 ou la revendication 6, agoniste de TrkB destiné à une utilisation selon la revendication 2 ou la revendication 6, antagoniste de LINGO-1 et agoniste de TrkB destinés à une utilisation selon la revendication 3 ou la revendication 6, ou utilisation selon l'une quelconque des revendications 4 à 6, le polypeptide hétérologue non antagoniste de LINGO-1 étant sélectionné dans le groupe constitué d'un fragment d'immunoglobuline, d'une sérumalbumine, d'une protéine de ciblage, d'une protéine reporter, et d'une protéine facilitant la purification.

8. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, l'anticorps anti-LINGO-1 étant un anticorps anti-LINGO-1 isolé qui se fixe spécifiquement au même épitope de LINGO-1 qu'un anticorps monoclonal de référence sélectionné dans le groupe constitué de : 1A7, 2F3, 3P1D10.2C3, 3P1E11.3B7, 3B5.230-C12 (Li01), 38-D01 (Li02), 35-E04 (Li03), 36-C09 (Li04), 30-A11 (Li05), 34-F02 (Li06), 29-E07 (Li07), 34-G04 (Li08), 36-A12 (Li09), 28-D02 (Li10), 30-B01 (Li11), 34-B03 (Li12), Li13, Li32, Li33, Li34, 3383 (L1a.1), 3495 (L1a.2), 3563 (L1a.3), 3564 (L1a.4), 3565 (L1a.5), 3566 (L1a.6), 3567 (L1a.7), 3568 (L1a.8), 3569 (L1a.9), 3570 (L1a.10), 3571 (L1a.11), 3582 (L1a.12), et 1968 (L1a.13).

9. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, l'antagoniste de LINGO-1 comprenant un polynucléotide antagoniste de LINGO-1 sélectionné dans le groupe constitué de :
(i) un polynucléotide antisens ;
(ii) un ribozyme ;
(iii) un petit ARN interférent (ARNsi) ; et
(iv) un petit ARN en épingle à cheveux (ARNsh).

10. Antagoniste de LINGO-1, agoniste de TrkB ou antagoniste de LINGO-1 et agoniste de TrkB destinés à une utilisation selon la revendication 9, ou utilisation selon la revendication 9, ledit ARNsh comprenant la séquence nucléotidique : UGAUCGUCAU CCUGCUAGAC UUCAAGAGAG UCUAGCAGGA UGACGAUCUU UUUUC (SÉQ. ID n° 34).

11. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, le polypeptide de TrkB comprenant :
(a) les acides aminés 1 à 828 de la SÉQ. ID n° 4 fusionnés à un domaine d'IgG ; ou
(b) les acides aminés 32 à 828 de la SÉQ. ID n° 4 fusionnés à un domaine d'IgG.

12. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, le polypeptide agoniste de TrkB étant un polypeptide BDNF, ou le polynucléotide agoniste de TrkB codant pour un polypeptide BDNF.

13. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5, l'anticorps agoniste de TrkB ou le fragment de celui-ci se liant à un antigène étant sélectionné dans le groupe constitué de : 6E2, 7F5, 11E1, 16E11, 17D11, 19E12, et 29D7.

14. Antagoniste de LINGO-1 destiné à une utilisation selon la revendication 1, agoniste de TrkB destiné à une utilisation selon la revendication 2, antagoniste de LINGO-1 et agoniste de TrkB destinés à une utilisation selon la revendication 3, ou utilisation selon la revendication 4 ou 5,
l'anticorps anti-LINGO-1 étant un anticorps anti-LINGO-1 isolé qui se fixe spécifiquement au même épitope de LINGO-1 qu'un anticorps monoclonal de référence sélectionné parmi Li13 ou Li33 ; et
l'anticorps agoniste de TrkB ou le fragment de celui-ci se liant à un antigène étant sélectionné dans le groupe constitué de : 6E2, 7F5, 11E1, 16E11, 17D11, 19E12, et 29D7, ou le polypeptide agoniste de TrkB étant un polypeptide BDNF.
